(19) Europäisches Patentamt / European Patent Office / Office européen des brevets

(11) EP 1 613 583 B1

(12) **EUROPÄISCHE PATENTSCHRIFT**

(45) Veröffentlichungstag und Bekanntmachung des Hinweises auf die Patenterteilung:
**17.08.2011 Patentblatt 2011/33**

(21) Anmeldenummer: **04724254.0**

(22) Anmeldetag: **30.03.2004**

(51) Int Cl.:
*C07C 69/00* (2006.01) *C08F 20/00* (2006.01)
*C08G 65/32* (2006.01) *C08G 65/28* (2006.01)

(86) Internationale Anmeldenummer:
**PCT/EP2004/003348**

(87) Internationale Veröffentlichungsnummer:
**WO 2004/087635 (14.10.2004 Gazette 2004/42)**

(54) **GEMISCHE VON VERBINDUNGEN MIT MINDESTENS ZWEI DOPPELBINDUNGEN UND DEREN VERWENDUNG**

MIXTURES OF COMPOUNDS COMPRISING AT LEAST TWO DOUBLE BONDS AND USE THEREOF

MELANGES DE COMPOSES AYANT AU MOINS DEUX LIAISONS DOUBLES ET LEUR UTILISATION

(84) Benannte Vertragsstaaten:
**AT BE BG CH CY CZ DE DK EE ES FI FR GB GR HU IE IT LI LU MC NL PL PT RO SE SI SK TR**

(30) Priorität:
**03.04.2003 DE 10315345**
**03.04.2003 DE 10315336**
**04.04.2003 DE 10315669**
**29.04.2003 DE 10319462**
**06.06.2003 PCT/EP03/05953**
**10.06.2003 PCT/EP03/06028**
**10.06.2003 PCT/EP03/06054**
**11.12.2003 DE 10358372**

(43) Veröffentlichungstag der Anmeldung:
**11.01.2006 Patentblatt 2006/02**

(73) Patentinhaber: **BASF SE**
**67056 Ludwigshafen (DE)**

(72) Erfinder:
- **RIEGEL, Ulrich**
  **66849 Landstuhl (DE)**
- **DANIEL, Thomas**
  **67165 Waldsee (DE)**
- **HERMELING, Dieter**
  **67459 Böhl-Iggelheim (DE)**
- **ELLIOTT, Mark**
  **67063 Ludwigshafen (DE)**
- **SCHWALM, Reinhold**
  **67157 Wachenheim (DE)**

(56) Entgegenhaltungen:
**US-A- 5 340 669**
**US-A- 5 792 827**
**US-A- 5 837 789**
**US-A1- 2002 032 304**
**US-B2- 6 579 958**

- **DATABASE WPI Section Ch, Week 200216 Derwent Publications Ltd., London, GB; Class A28, AN 2002-117236 XP002301779 & JP 2001 243671 A (VICTOR CO OF JAPAN), 7. September 2001 (2001-09-07)**
- **MILLER H C ED - INSTITUTE OF ELECTRICAL AND ELECTRONICS ENGINEERS: "RADTECH 91" PROCEEDINGS 1999 INTERNATIONAL CONFERENCE ON IMAGE PROCESSING. ICIP'99. KOBE, JAPAN, OCT. 24 - 28, 1999, INTERNATIONAL CONFERENCE ON IMAGE PROCESSING, LOS ALAMITOS, CA : IEEE, US, Bd. VOL.1 OF 4, 1999, XP002255813 ISBN: 0-7803-5468-0**

EP 1 613 583 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft neue Gemische von mindestens zwei Verbindungen mit jeweils mindestens zwei Doppelbindungen, wobei das Gemisch ein GFV von 240 bis 600 g/mol Doppelbindung aufweist, und zumindest zwei der Verbindungen jeweils mindestens zwei (Meth)acrylsäureester als Doppelbindungskomponente enthalten, und GFV bedeutet:

$$\sum_{i=1}^{n} \alpha_i \times MW_i / Z_i = GFV$$

mit

$$\sum_{i=1}^{n} \alpha_i = 1$$

$\alpha_i$ entspricht dem molaren Anteil der Verbindung (i) im Gemisch, $n \geq 2$ (n ist die Anzahl der Verbindungen (Vernetzerkomponenten) im Gemisch), $Z_i$ entspricht der Anzahl der Doppelbindungen in der Verbindung (i), $MW_i$ entspricht dem Molekulargewicht der Verbindung (i), ein vereinfachtes Verfahren zur Herstellung dieser Ester und Verwendung der so erhältlichen Reaktionsgemische.

Quellbare Hydrogel-bildende Polymerisate, sogenannte Superabsorber (Super-Absorbing Polymers, SAP), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber werden außerdem in anderen Gebieten der Technik eingesetzt, bei denen Flüssigkeiten, insbesondere Wasser oder wässrige Lösungen absorbiert werden. Diese Gebiete sind z.B. Lagerung, Verpackung, Transport (Verpackungsmaterial wassersensitiver Artikel, wie z.B. Blumentransport, Schock Protection); Lebensmittelsektor (Transport von Fisch, Frischfleisch; Absorption von Wasser, Blut in Frischfisch/-Fleisch-Verpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textil (Handschuhe, Sportbekleidung, Feuchtigkeitsregulation in Textilien, Schuheinlagen); chem.-techn. Anwendungen (Katalysator für org. Reaktionen, Immobilisierung großer funktioneller Moleküle (Enzyme), Adhesiv für Agglomerationen, Wärmespeicher, Filtrationshilfsmittel, hydrophile Komponente in Polymerlaminaten, Dispergiermittel, Verflüssiger); Bau- und Konstruktionswesen, Installation (Pulverspritzguss, lehmbasierende Putze, Vibrationshemmendes Medium, Hilfsmittel bei Tunnelgrabungen in wasserreichem Untergrund, Kabelummantelung); Wasserbehandlung, Abfallbehandlung (insbesondere die Verfestigung wässriger Abfälle), Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); im Feuerschutz (Funkenflug) (Abdecken von Häusern bzw. Bedecken von Hauswänden mit SAP Gel, da das Wasser eine sehr hohe Wärmekapazität hat, kann ein Entflammen verhindert werden; Versprühen von SAP Gel bei Bränden wie z.B. Waldbränden); Coextrusionsmittel in thermoplastischen Polymeren (Hydrophilierung von Mehrschichtfolien); Herstellung von Folien und thermoplastischen Formkörpern, die Wasser absorbieren können (z.B. Regen- und Tauwasser speichernde Folien für die Landwirtschaft); SAP haltige Folien zum Frischhalten von Obst und Gemüse, die in feuchte Folien verpackt werden können; der SAP speichert vom Obst und Gemüse abgegebenes Wasser ohne Bildung von Kondensationstropfen und gibt das Wasser teilweise an das Obst und Gemüse wieder ab, so dass weder Faulen noch Verwelken eintritt; SAP-Polystyrol Coextrudate z.B. für Lebensmittelverpackungen wie Fleisch, Fisch, Geflügel, Obst und Gemüse); Trägersubstanz in Wirkstoffformulierungen (Pharma, Pflanzenschutz). In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass herkömmliche Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) darf das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden. Das Phänomen des Gelblockings wird somit unterbunden, das im Extremfall zum Austritt der Flüssigkeit, der sog. Leakage des Hygieneartikels führt. Die Flüssigkeitsweiterleitung und -verteilung ist also bei der anfänglichen Absorption von Körperflüssigkeiten von entscheidender Bedeutung.

Gute Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Eine elegante Methode zur Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wässriger Gelphase oder als Oberflächennachvernetzung der gemahlenen und abgesiebten Polymerpartikel.

In WO 90/15830 werden Vernetzergemische aus Methylenbisacrylamid mit Diallylweinsäurediamid beschrieben.

In WO 02/32964 werden Vernetzergemische zwischen Alcrylesterglycolen und Allylverbindungen beschrieben.

In DE 19646484 werden Vernetzergemische zwischen Allyletheracrylesterglycolen und Acrylestern bzw. Allylaminen beschrieben.

Allyle sind weniger reaktiv und führen bei der Polymerisation häufig zu zähen Gelen.

Aus WO 93/21237 sind (Meth)acrylate von alkoxylierten mehrwertigen $C_2$ - $C_{10}$-Kohlenwasserstoffen als Vernetzer bekannt. Verwendet wurden Trimethylpropanvernetzer, die SR 351, SR 454, SR 502, SR 9035 und SR 415 entsprechen. Diese Vernetzer weisen 0, 3, 9, 15 oder 20 EO Einheiten pro TMP auf. Vorteilhaft sind laut WO 93/21237 3-mal 2 bis 7 EO Einheiten pro TMP, insbesondere 3-mal 4 bis 6 EO Einheiten pro TMP.

Nachteilig an diesen Verbindungen ist, dass zur zumindest teilweisen Abtrennung von Einsatzstoffen und Nebenprodukten - die in der genannten Schrift eingesetzten Vernetzer weisen einen Gehalt an Acrylsäure von weniger als 0,1 Gew.-% auf - aufwendige Reinigungsoperationen erforderlich sind.

Die Herstellung solcher höheren (Meth)acrylsäureester durch säurekatalysierte Veresterung von (Meth)acrylsäure mit den entsprechenden Alkoholen in Gegenwart eines Inhibitors/Inhibitorsystems sowie gegebenenfalls eines Lösungsmittels, wie z.B. Benzol, Toluol, Cyclohexan, ist allgemein bekannt.

Da bekanntlich der Bildung des Esters aus (Meth)acrylsäure und Alkohol eine Gleichgewichtsreaktion zugrunde liegt, wird, um wirtschaftliche Umsätze zu erzielen, in der Regel ein Einsatzstoff im Überschuss eingesetzt und/oder das gebildete Veresterungswasser und/oder der Zielester aus dem Gleichgewicht entfernt.

Daher wird bei der Herstellung der höheren (Meth)acrylsäureester in der Regel das Reaktionswasser entfernt

und meist ein Überschuss an (Meth)acrylsäure eingesetzt.

US 4187 383 beschreibt ein Veresterungsverfahren von (Meth)acrylsäure mit organischen Polyolen bei einer Reaktionstemperatur von 20 bis 80 °C mit einem Äquivalent-Überschuss von 2 bis 3 : 1.

Nachteilig an diesem Verfahren ist, dass durch die niedrige Reaktionstemperatur die Reaktionszeiten bis zu 35 Stunden betragen und der Überschuss Säure im Reaktionsgemisch durch eine Neutralisation mit anschließender Phasentrennung entfernt wird.

WO 2001/14438 (Derwent-Abstract Nr. 2001-191644/19) und WO 2001/10920 (Chemical Abstracts 134: 163502) beschreiben Verfahren zur Veresterung von (Meth)acrylsäure mit Polyalkylenglykolmonoalkylethern im Verhältnis 3:1 - 50:1 in Gegenwart von Säuren und Polymerisationsinhibitoren und, nach Desaktivierung des sauren Katalysators, Copolymerisation des Rückstandes aus (Meth)acrylsäureester und (Meth)acrylsäure bei pH 1,5 - 3,5, sowie dessen Verwendung als Zementadditiv.

Nachteilig an diesen Verfahren ist, dass es auf Polyalkylenglykolmonoalkylether beschränkt ist, dass der Katalysator deaktiviert werden muss und dass derartige Copolymerisate nicht als Vernetzer für Hydrogele eingesetzt werden können, da sie lediglich eine Funktionalität aufweisen.

Es bestand die Aufgabe, weitere Verbindungen zur Verfügung zu stellen, die als Radikalvernetzer für Polymere, insbesondere für Superabsorber verwendet werden können und das Herstellungsverfahren für Substanzen, die als Radikalvernetzer für Superabsorber verwendbar sind, zu vereinfachen. Des weiteren wurde nach Vernetzern gesucht deren Hydrolysestabilität hoch ist und/oder die gleichzeitig ein sehr gut zerteilbares Gel im Herstellungsverfahren für Superabsorber ergeben. Außerdem wurde nach Vernetzern gesucht, deren Eigenschaften leicht modifizierbar sind, dabei sollte sowohl bei Änderungen im Herstellungsprozess, wie bei der Zulassung der Produkte möglichst wenig zusätzliche Kosten und möglichst wenig technischer Aufwand entstehen.

Die Aufgabe wird gelöst durch das zur Verfügung stellen von einem Gemisch von mindestens zwei Verbindungen mit jeweils mindestens zwei Doppelbindungen, wobei das Gemisch ein GFV von 240 bis 600 g/mol Doppelbindung aufweist, zumindest zwei der Verbindungen jeweils mindestens zwei (Meth)acrylsäureester als Doppelbindungskomponente enthalten, und GFV bedeutet:

$$\sum_{i=1}^{n} \alpha_i \times MW_i / Z_i = GFV$$

mit

$$\sum_{i=1}^{n} \alpha_i = 1$$

$\alpha_i$ entspricht dem molaren Anteil der Verbindung (i) im Gemisch,
$n \geq 2$.
$Z_i$ entspricht der Anzahl der Doppelbindungen in der Verbindung (i),
$MW_i$ entspricht dem Molekulargewicht der Verbindung (i).

GFV, das Gewichtsfunktionalitätsverhältnis, entspricht der Doppelbindungsfunktionalität des gewünschten Verbindungs- bzw. Vernetzergemisches. Das sich daraus ergebende lineare Gleichungssystem kann mit Hilfe des Gauss-Algorithmus für den allgemeinen Fall (n-Komponenten) gelöst werden, wenn es eine oder mehrere Lösungen besitzt. Ein solches Gemisch kann leicht durch der Verschiebung der relativen Anteile der Mischungskomponenten optimiert werden. Damit lassen sich die spezifischen Vernetzereigenschaften des Gemischs auch durch einfache Anteilsvariation seiner bekannten und zugelassenen Komponenten bei beispielsweise gleichbleibender Einsatzmenge des Gemischs in der Herstellung von vernetzten Polyacrylaten (z.B. Superabsorbern) beliebig modifizieren. Bisher wurden zu diesem Zweck sowohl Einsatzmengen variiert als auch jeweils spezielle neue Vernetzer neu synthetisiert und mit erheblichem Aufwand zugelassen.

Für den speziellen Fall eines 2-Komponenten Gemisches ergibt sich folgendes einfache System:

$$\alpha_1 \times MW_1/Z_1 + \alpha_2 \times MW_2/Z_2 = GFV \quad \text{und} \quad \alpha_2 = 1 - \alpha_1$$

Daraus ergibt sich letztlich

$$\alpha_1 = (GFV - MW_2/Z_2) / (MW_1/Z_1 - MW_2/Z_2).$$

Dieses System ist eindeutig lösbar wenn $MW_1/Z_1 \neq MW_2/Z_2$ und wenn $MW_1/Z_1 < GFV < MW_2/Z_2$ oder $MW_2/Z_2 < GFV < MW_1/Z_1$.

Bevorzugt sind obige Gemische, wobei die Untergrenze bei 240 liegt, die Obergrenze bevorzugt bei 550, 500, 450 oder 400 liegt, das Gemisch ein GFV bevorzugt zwischen 240 und 400 g/mol Doppelbindung aufweist. Besonders bevorzugt liegt die Untergrenze bei 250, 260, 270, 280 oder 290, die Obergrenze bei 390, 380, 370, 360 oder 350, besonders bevorzugt ist ein GFV, das zwischen 250 und 350 g/mol Doppelbindung aufweist. Insbesondere bevorzugt ist eine Bereich zwischen 300 und 330 bzw. 340.

Bevorzugt sind obige Gemische, wobei n 2, 3 oder 4, besonders bevorzugt 2 bedeutet.

Bevorzugt sind obige Gemische, wobei zwischen den Verhältnissen MW/Z von zwei Verbindungen ein Unterschied von mindestens 50, 60, 70, 80 oder 90 g/mol Doppelbindung, bevorzugt von mindestens 100, 110, 120, 130, 140, 150, 160, 170, 180, 190, 200, 210, 220, 230, 240 g/mol Doppelbindung, besonders bevorzugt mindestens 250, 260, 270, 280, 290, 300, 310, 320, 330, 340 g/mol Doppelbindung , insbesondere 350, 360, 270, 380, 390 oder 400 g/mol Doppelbindung liegt.

Bevorzugt sind obige Gemische, wobei eine Verbindung ein Verhältnis MW/Z von unter 400 g/mol Doppelbindung, bevorzugt von unter 300 g/mol Doppelbindung, besonders bevorzugt unter 200 g/mol Doppelbindung, insbesondere unter 150 g/mol Doppelbindung aufweist.

Bevorzugt sind obige Gemische, wobei eine Verbindung ein Verhältnis MW/Z von über 400 g/mol Doppelbindung und unter 10000 g/mol Doppelbindung, bevorzugt von über 600 g/mol Doppelbindung und unter 1000 g/mol Doppelbindung aufweist.

Die Anzahl n der einzelnen Verbindungen im erfindungsgemäßen Gemisch, die zu mehr wie 1 Gew.% des Gemisches vorliegen, liegt bevorzugt höchstens bei 10, z.B. bei 9, 8, 7 oder 6, besonders bevorzugt bei 5 oder weniger, oder 4 oder weniger, ganz besonders bevorzugt bei 3, insbesondere bei 2.

Liegen die einzelnen Komponenten eines Gemisches als Gauß-förmige Verteilung von Einzelverbindungen vor, gilt das oben angeführte bevorzugt für die mittleren Werte der einzelnen Komponenten.

Bevorzugt sind obige Gemische, wobei Z mindestens einer Verbindung zwischen 2 und 6, bevorzugt bei 2, 3 oder 4 liegt und insbesondere bei beiden in diesem Bereich liegt.

Bevorzugt sind obige Gemische, wobei die Verbindungen Ester $F_i$ sind, die durch die Veresterung von Polyalkoholen $A_i$ mit (Meth)acrylsäure zugänglich sind und jeder Polyalkohol $A_i$ $Z_i$ Hydroxyfunktionen und insbesondere 2 bis 50 Kohlenstoffatome aufweist.

Das Molgewicht der einsetzbaren Polyalkohole beträgt in der Regel, wenn nicht anders angegeben, unter 5000 g/mol, bevorzugt unter 2500 g/mol, besonders bevorzugt unter 1500 g/mol, ganz besonders bevorzugt unter 1000 g/mol und insbesondere unter 800 g/mol.

Bevorzugte Polyalkohole A sind Polyole, funktionalisierte Polyole, alkoxylierte Polyole, Zuckeralkohole, teilweise alkoxylierte Zuckeralkohole, Polyetherole, Polyesterole, zumindest teilweise alkoxylierte Polyesterole und zumindest teilweise verseifte, alkoxylierte Polyesterole.

Beispiele für Polyole sind Trimethylolbutan, Trimethylolpropan, Trimethylolethan, Neopentylglykol, Hydroxypivalinsäureneopentylglykolester, Pentaerythrit, Glycerin, 1,2-Ethylenglykol, 1,2-Propylenglykol, 1,3-Propylenglykol; 1,2-Butandiol), 1,3-Butandiol; 1,4-Butandiol, 2-Ethyl-1,3-Propandiol, 2-Methyl-1,3-Propandiol, Hydrochinon, Bisphenol A, Bisphenol F, Bisphenol B, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, But-2-en-1,4-diol und But-2-in-1,4-diol, Zuckeralkohole mit C4 bis C6-Kette wie z.B. Sorbitol.

Die Polyole können auch noch zusätzliche Funktionalitäten tragen wie z.B. Etherfunktionen (-O-), Carboxylfunktionen (-COOH) oder $C_1$-$C_4$-Alkyloxycarbonylfunktionen (Estergruppen), wobei $C_1$-$C_4$-Alkyl in dieser Schrift Methyl, Ethyl, *iso*-Propyl, n-Propyl, n-Butyl, *iso*-Butyl, *sek*-Butyl oder *tert*-Butyl bedeutet.
Beispiele für solche funktionalisierten Polyole sind Ditrimethylolpropan, Dipentaerythrit, Dimethylolpropionsäure, Dimethylolbuttersäure, Trimethylolessigsäure, Hydroxypivalinsäure und die 2-Hydroxyethyl- oder $C_1$-$C_4$-Alkylester dieser genannten Säuren.

Bevorzugte Polyole sind solche der Formel (I):

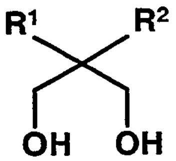

(I)

Darin bedeuten

$R^1$, $R^2$ unabhängig voneinander Wasserstoff, $C_1$- $C_{10}$-Alkyl, bevorzugt $C_1$-$C_4$- Alkyl, $C_1$-$C_{10}$-Hydroxyalkyl, bevorzugt Hydroxy-$C_1$-$C_4$-Alkyl, Carboxyl oder $C_1$-$C_4$-Alkyloxycarbonyl, bevorzugt Wasserstoff, Hydroxymethyl und $C_1$-$C_4$-Alkyl und besonders bevorzugt Hydroxymethyl und $C_1$-$C_4$- Alkyl.

Dabei können die Alkylreste jeweils geradkettig oder verzweigt sein.

Beispiele für $R^1$ und $R^2$ sind Wasserstoff, Methyl, Ethyl, *iso*-Propyl, n-Propyl, n-Butyl, *iso*-Butyl, *sek*-Butyl, *tert*-Butyl, n-Pentyl, n-Hexyl, n-Heptyl, n-Octyl, n-Decyl, Hydroxymethyl, Carboxyl, Methoxycarbonyl, Ethoxycarbonyl oder n-Butoxycarbonyl, bevorzugt Wasserstoff, Hydroxymethyl, Methyl und Ethyl, besonders bevorzugt Hydroxymethyl, Methyl und Ethyl.

Besonders bevorzugte mehrwertige Alkohole der Formel (I) sind Trimethylolbutan, Trimethylolpropan, Trimethylolethan, Neopentylglykol, Pentaerythrit, 2-Ethyl-1,3-Propandiol, 2-Methyl-1,3-Propandiol, 1,3-Propandiol, 1,4-Butandiol, Dimethylolpropionsäure, Dimethylolpropionsäuremethylester, Dimethylolpropionsäureethylester, Dimethylolbuttersäure, Dimethylolbuttersäuremethylester oder Dimethylolbuttersäureethylester, bevorzugt sind Neopentylglykol, Trimethylolpropan, Pentaerythrit und Dimethylolpropionsäure, ganz besonders bevorzugt sind Neopentylglykol, Trimethylolpropan und Pentaerythrit und insbesondere Trimethylolpropan und Pentaerythrit.

Beispiele für Zuckeralkohole sind Sorbit, Mannit, Maltit, Isomalt, Diglycerol, Threit, Erythrit, Adonit (Ribit), Arabit (Lyxit), Xylit und Dulcit (Galactit).

Beispiele für Polyetherole sind Poly-THF mit einer Molmasse zwischen 162 und 2000, bevorzugt zwischen 162 und 1458, besonders bevorzugt zwischen 162 und 1098, ganz besonders bevorzugt zwischen 162 und 738 und insbesondere zwischen 162 und 378, Poly-1,3-propandiol und Poly-1,2-propandiol mit einer Molmasse zwischen 134 und 1178, bevorzugt zwischen 134 und 888, besonders bevorzugt zwischen 134 und 598 und ganz besonders bevorzugt zwischen 134 und 308, Polyethylenglykol mit einer Molmasse zwischen 106 und 898, bevorzugt zwischen 106 und 458, besonders bevorzugt von 106 bis 400, ganz besonders bevorzugt zwischen 106 und 235 und insbesondere Diethylenglykol, Triethylenglykol und Tetraethylenglykol.

Als Polyesterole kommen z.B. solche in Betracht, wie sie durch Veresterung von Polycarbonsäuren, vorzugsweise Dicarbonsäuren, mit den oben genannten Polyolen hergestellt werden können.

Die Ausgangsstoffe für solche Polyesterole sind dem Fachmann bekannt. Bevorzugt können als Polycarbonsäuren Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, deren Isomere und Hydrierungsprodukte sowie veresterbare Derivate, wie Anhydride oder Dialkylester, beispielsweise $C_1$-$C_4$-Alkylester, bevorzugt Methyl-, Ethyl- oder n-Butylester, der genannten Säuren eingesetzt werden.

Als Hydroxygruppen tragende Carbonsäuren oder Lactone kommen 4-Hydroxybenzoesäure, 6-Hydroxy-2-naphthalinsäure, Pivalolacton oder □-Caprolacton in Betracht. Als Polyole kommen die oben genannten mehrfunktionellen Alkohole, vorzugsweise Neopentylglykol, Trimethylolpropan, Trimethylolethan, Pentaerythrit, Dimethylolpropionsäure oder Dimethylolbuttersäure in Betracht.

Bevorzugte Beispiele für derartige Polyesterole sind solche der Formel (IIIa-c),

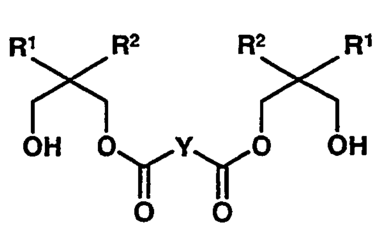

(IIIa)

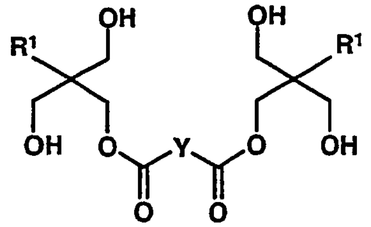

(IIIb)

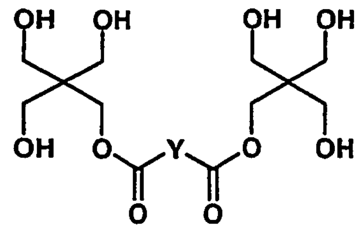

(IIIc)

worin

| | |
|---|---|
| $R^1$, $R^2$ | die oben genannten Bedeutungen haben und |
| Y | eine geradkettige oder verzweigte, gegebenenfalls substituierte Alkylengruppe mit 2 bis 20 Kohlenstoffatomen oder eine gegebenenfalls substituierte Cyclo- alkylen- oder Arylengruppe mit 6 bis 12 Kohlenstoffatomen oder eine Einfach- bindung |

bedeuten.
Beispiele für Y sind eine Einfachbindung, Methylen, 1,2-Ethylen, 1,3-Propylen, 1,4-Butylen, 1,6-Hexylen, 1,7-Heptylen, 1,8-Octylen, cis-1,2-Ethenylen, trans-1,2-Ethenylen, 1,2-, 1,3- oder 1,4-Phenylen, 1,2-Cyclohex-1-enylen, 1,2-, 1,3- oder 1,4-Cyclohexylen, 4-Carboxy-1,2-phenylen, 2-Carboxy-1,4-phenylen oder 1-Carboxy-2,4-phenylen.

Bevorzugte Gruppen Y sind 1,2-Ethylen, 1,4-Butylen und 1,2-, 1,3- oder 1,4-Phenylen.

Selbstverständlich liegen herstellungsbedingt in der Regel Gemische vor, in denen zusätzlich niedere und höhere Oligomere enthalten sein können.

In einer weiteren bevorzugten Ausführungsform werden Reaktionsgemische von zumindest teilweise verseiften Polyesterolen als Polyalkohole A zur Herstellung des Esters F eingesetzte

Dazu werden die z.B. oben beschriebenen Polyesterole mit einer geeigneten Base zumindest teilweise verseift und anschließend, gegebenenfalls nach Abtrennung der im Reaktionsgemisch verbliebenen basischen Bestandteile, mit der Carbonsäure B verestert.

Geeignete Basen sind beispielsweise NaOH, KOH, $Ca(OH)_2$, Kalkmilch, $Na_2CO_3$ oder $K_2CO_3$, beispielsweise als Feststoff, Lösung oder Suspension, bevorzugt in Form einer 10-50 gew.-%igen Lösung, besonders bevorzugt in Form einer 20-40 gew.-%igen wässrigen Lösung.

Die Verseifung, d.h. die Spaltung der im Polyesterol enthaltenen Estergruppen, erfolgt zu beispielsweise mindestens 10% bezogen auf die Estergruppen in der Ausgangsverbindung, bevorzugt zu mindestens 25%, besonders bevorzugt zu mindestens 50%, ganz besonders bevorzugt zu mindestens 75% und insbesondere zu mindestens 90%.

Falls basische Bestandteile, wie z.B. das basische Salz der Carbonsäure, aus dem Reaktionsgemisch entfernt werden sollen, kann dies beispielsweise über Ionentauscher, z.B. saure oder stark saure Ionentauscher erfolgen.

Das Reaktionsgemisch wird anschließend angesäuert und mit der Carbonsäure B wie beschrieben verestert.

Polyester(meth)acrylate können in mehreren Stufen oder auch einstufig, wie z.B. in EP-A 279 303 beschrieben, aus (Meth)acrylsäure, Polycarbonsäure und Polyol hergestellt werden.

Ebenfalls als Polyalkohole einsetzbar sind alkoxylierte Polyole und Polyesterole, die durch Umsetzung eines Polyols oder Polesterols mit mindestens einem Alkylenoxid erhältlich sind.

Erfindungsgemäß lassen sich auch solche Verbindungen der Formel VII enthaltende Reaktionsgemische darstellen.

$$R^8\text{-}(O(CH(R^{10})CH(R^{10}O)_y\text{-}C(=O)\text{-}R^9)_x \qquad \text{(VII),}$$

worin

| | |
|---|---|
| $R^8$ | ein mehrwertiger, geradkettiger oder verzweigter $C_2$-$C_{10}$-Alkylrest, |
| $R^9$ | unabhängig voneinander ein geradkettiger oder verzweigter $C_2$-$C_{10}$-Alkenylrest, |
| $R^{10}$ | unabhängig voneinander Wasserstoff oder Methyl, |
| x | unabhängig voneinander eine positive ganze Zahl von 2 oder größer und |
| y | unabhängig voneinander für x=2 eine Zahl von 3 bis 8 und für x=3 oder größer eine Zahl von 2 bis 7 ist. |

Der zugrundeliegende zu veresternde Alkohol weist dabei die Formel VIIa auf,

$$R^8\text{-}(O(CH(R^{10})CH(R^{10})O)_y\text{-}H)_x \qquad \text{(VIIa)},$$

worin $R^8$, $R^{10}$, x und y wie oben definiert sind.

Die Verbindungen der Formel (VII) sind in der Regel 2 bis 10 Kohlenstoffatome aufweisende mehrwertige Alkohole VIIa, die mit zwischen 2 und 8 Alkylenoxideinheiten pro Hydroxygruppe alkoxyliert sind und deren endständige Hydroxygruppe jeder Alkylenoxidkette mit einer 2 bis 10 Kohlenstoffatome aufweisenden ungesättigten Carbonsäure oder deren Ester verestert ist. Bevorzugt handelt es sich bei dem Startalkohol um einen 3-6 Kohlenstoffatome aufweisenden mehrwertigen Alkohol, der bevorzugt 2 bis 4 Hydroxygruppen trägt. Besonders bevorzugt handelt es sich bei dem Startalkohol um Trimethylolpropan, Glycerin, Pentaerythrit, 1,3-Propandiol, Propylenglykol, 1,4-Butandiol oder Butylenglykol. Ganz besonders bevorzugt sind Trimethylolpropan, Glycerin und Pentaerythrit als Startalkohol.

Geeignete Alkylenoxide sind beispielsweise Ethylenoxid, Propylenoxid, *iso*-Butylenoxid, Vinyloxiran und/oder Styroloxid.

Die Alkylenoxidkette kann bevorzugt aus Ethylenoxid-, Propylenoxid- und/oder Butylenoxideinheiten zusammengesetzt sein. Eine solche Kette kann sich aus einer Spezies eines Alkylenoxides oder aus einem Gemisch von Alkylenoxiden zusammensetzen. Wird ein Gemisch verwendet, können die unterschiedlichen Alkylenoxideinheiten statistisch oder als Block oder Blöcke einzelner Spezies vorliegen. Bevorzugt ist als Alkylenoxid Ethylenoxid, Propylenoxid oder ein Gemisch daraus, besonders bevorzugt ist es Ethylenoxid oder Propylenoxid und ganz besonders bevorzugt Ethylenoxid. Somit ist bevorzugt ein Rest $R^9$ pro Alkylenoxideinheit Wasserstoff und der andere Methyl oder Wasserstoff, besonders bevorzugt sind beide Reste $R^9$ Wasserstoff.

Die bevorzugte Anzahl der Alkylenoxideinheiten in jeder Kette ist abhängig von der Anzahl der Ketten.

Das Veresterungsmittel ist eine 2 bis 10 Kohlenstoffatome aufweisende, geradkettige oder verzweigte ethylenisch ungesättigte Carbonsäure oder deren Ester, bevorzugt eine 2 bis 4 und besonders bevorzugt eine 2 bis 3 Kohlenstoffatome aufweisende ethylenisch ungesättigte Carbonsäure, ganz besonders bevorzugt Acrylsäure, Methacrylsäure oder deren Ester, insbesondere Acrylsäure.

Häufig liegen die Verbindungen der Formel VII als Gemisch von Verbindungen, die durch diese Formel beschrieben werden, und Nebenprodukten des Herstellungsprozesses vor.

Insbesondere bevorzugt sind unter diesen Verbindungen VII die je Hydroxygruppe bis zu sechsfach, besonders bevorzugt die bis zu vierfach und ganz besonders bevorzugt die vierfach ethoxylierten Verbindungen, im folgenden Verbindungen VIIb genannt. Diese weisen eine erhöhte Hydrolysestabilität auf.

Gleichermaßen bevorzugt sind Verbindungen VII, die je Hydroxygruppe

- für x=2 mehr als achtfach, besonders bevorzugt mehr als zehnfach, ganz besonders bevorzugt mehr als zwölffach und insbesondere mindestens 15fach, beziehungsweise
- für x=3 oder größer mehr als siebenfach, besonders bevorzugt mehr als neunfach, ganz besonders bevorzugt mehr als zwölffach und insbesondere mindestens 15fach ethoxyliert sind, im folgenden Verbindungen VIIc genannt, da diese in der Regel eine erhöhte Wasserlöslichkeit aufweisen.

Denkbar sind auch solche Verbindungen VII, bei denen für x = 2 y Werte von 0, 1 oder 2 und für x = 3 y Werte von 0 oder 1 annehmen kann.

Insbesondere sind Mischungen aus den Verbindungen VIIb und VIIc vorteilhaft, beispielsweise solche mit einem Gewichtsverhältnis VIIb:VIIc von 10:90 bis 90:10, bevorzugt 20:80 bis 80:20, besonders bevorzugt 30:70 bis 70:30 und ganz besonders bevorzugt von 40:60 bis 60:40. Das Mischungsverhältnis ist jedoch in erster Linie durch die gewünschten Endprodukteigenschaften des Polymerisates bestimmt.

Bevorzugte Beispiele für derartige alkoxylierte Polyole sind die Alkoxylierungsprodukte (IIa), (IIb) oder (IIc) von Polyolen der Formel (I),

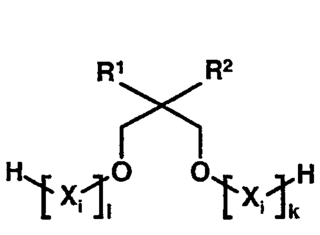
(IIa)

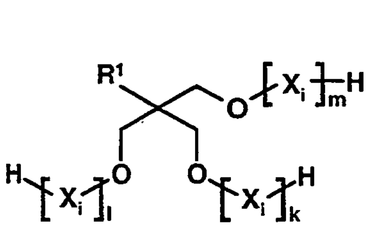
(IIb)

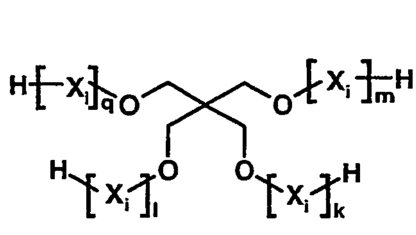
(IIc)

worin

$R^1$, $R^2$ die oben genannten Bedeutungen haben,

k, l, m, q unabhängig voneinander je für eine ganze Zahl von 1 bis 10, bevorzugt 1 bis 5, besonders bevorzugt 3 bis 5 und insbesondere 4 steht und

jedes $X_i$ für i = 1 bis k, 1 bis l, 1 bis m und 1 bis q unabhängig voneinander ausgewählt sein kann aus der Gruppe $-CH_2-CH_2-O-$, $-CH_2-CH(CH_3)-O-$, $-CH(CH_3)-CH_2-O-$, $-CH_2-C(CH_3)_2-O-$, $-C(CH_3)_2-CH_2-O-$, $-CH_2$-CHVin-O-, -CHVin-$CH_2$-O-, $-CH_2$-CHPh-O- und -CHPh-$CH_2$-O-, bevorzugt aus der Gruppe $-CH_2-CH_2-O-$, $-CH_2-CH(CH_3)-O-$ und $-CH(CH_3)-CH_2-O-$, und besonders bevorzugt $-CH_2-CH_2-O-$,

worin Ph für Phenyl und Vin für Vinyl steht.

Bevorzugt handelt es sich dabei um ein- bis fünffach, besonders bevorzugt drei- bis fünffach und ganz besonders bevorzugt vierfach ethoxyliertes, propoxyliertes oder gemischt ethoxyliertes und propoxyliertes und insbesondere ausschließlich ethoxyliertes Neopentylglykol, Trimethylolpropan, Trimethylolethan oder Pentaerythrit.

Unter diesen besonders bevorzugt sind solche mehrwertigen Alkohole der Formel (IIb).

Gleichermaßen bevorzugt ist ein ein bis 20fach, bevorzugt ein bis zehnfach, besonders bevorzugt zwei bis zehnfach, ganz besonders bevorzugt zwei bis fünffach, insbesondere drei bis fünffach und speziell drei bis vierfach alkoxyliertes, bevorzugt ethoxyliertes, propoxyliertes oder gemischt-ethoxyliert-propoxyliertes und besonders bevorzugt ethoxyliertes Glycerin (hier ausnahmsweise berechnet in mol Alkoxygruppen pro mol Glycerin).

Die angegebenen Alkoxylierungsgrade beziehen sich dabei jeweils auf den mittleren Alkoxylierungsgrad.

Das zahlenmittlere Molgewicht $M_n$ der alkoxylierten Polyole beträgt bevorzugt nicht mehr als 1000 g/mol, besonders bevorzugt nicht mehr als 800 g/mol und ganz besonders bevorzugt nicht mehr als 550 g/mol.

Die Angaben zum zahlenmittleren und gewichtsmittleren Molekulargewicht $M_n$ und $M_w$ beziehen sich hier auf gelpermeationschromatographische Messungen, wobei Polystyrol als Standard und Tetrahydrofuran als Elutionsmittel verwendet wurde. Die Methode ist im Analytiker Taschenbuch Bd. 4, Seiten 433 bis 442 , Berlin 1984 beschrieben.

Beispiele für alkoxylierten Zuckeralkohole sind solche Verbindungen, die aus Zuckeralkoholen, beispielsweise aus den oben aufgeführten Zuckeralkoholen, durch Alkoxylierung, beispielsweise mit den oben aufgeführten Alkylenoxiden, bevorzugt mit Ethylenoxid und/oder Propylenoxid und ganz besonders bevorzugt mit Ethylenoxid erhältlich sind.

Beispiele dafür sind

- die aufgeführten Tetrole, die im statistischen Mittel pro mol Zuckeralkohol 2 - 30fach, bevorzugt 2 - 20fach, besonders bevorzugt 3-10fach und insbesondere 3, 4, 5, 6, 7 oder 8fach alkoxyliert sind,
- die aufgeführten Pentole, die im statistischen Mittel pro mol Zuckeralkohol 3 - 35fach, bevorzugt 3-28fach, besonders bevorzugt 4-20fach und insbesondere 4, 5, 6, 7, 8, 9 oder 10fach alkoxyliert sind,
- höhere Zuckeralkohole, die im statistischen Mittel pro mol Zuckeralkohol 4-50fach, bevorzugt 6-40fach, besonders bevorzugt 7-30fach, ganz besonders bevorzugt 8-20fach und insbesondere 10-15fach alkoxyliert sind.

Bevorzugte alkoxylierte Zuckeralkohole sind solche, bei denen mindestens eine Hydroxygruppe des Zuckeralkohols nicht alkoxyliert ist.

Bevorzugte Beispiele für alkoxylierte Polesterole sind solche der Formel (IVa-c),

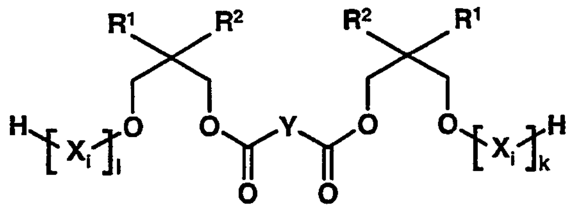

(IVa)

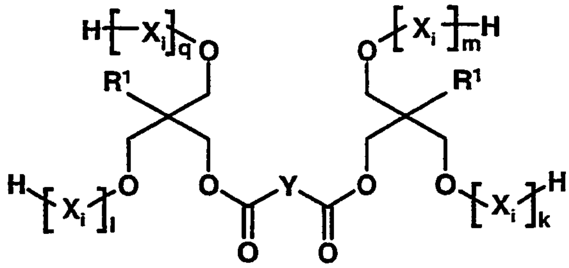

(IVb)

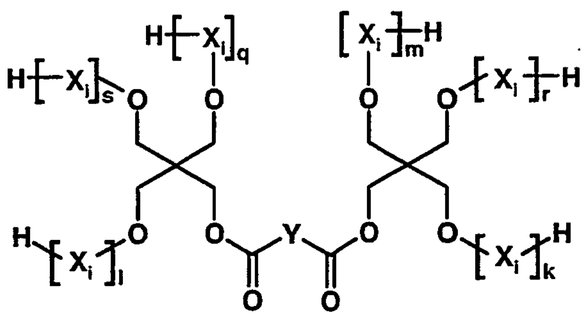

(IVc)

worin

| | |
|---|---|
| $R^1$, $R^2$, Y | die oben genannten Bedeutungen haben, |
| k, l, m, q, r, s | unabhängig voneinander je für eine ganze Zahl von 1 bis 30, bevor- zugt 1 bis 20, besonders bevorzugt 1 bis 10 und insbesondere 1 bis 5 steht und |
| jedes $X_i$ | für i = 1 bis k, 1 bis l, 1 bis m, 1 bis q, 1 bis r und 1 bis s unabhängig vonein- ander ausgewählt sein kann aus der Gruppe $-CH_2-CH_2-O-$, $-CH_2-CH(CH_3)-O-$, $-CH(CH_3)-CH_2-O-$, $-CH_2-C(CH_3)_2-O-$, $-C(CH_3)_2-CH_2-O-$, $-CH_2-CHVin-O-$, $-CHVin-CH_2-O-$, $-CH_2-CHPh-O-$ und $-CHPh-CH_2-O-$, bevorzugt aus der Grup- pe $-CH_2-CH_2-O-$, $-CH_2-CH(CH_3)-O-$ und $-CH(CH_3)-CH_2-O-$, und besonders be- vorzugt $-CH_2-CH_2-O-$, |

worin Ph für Phenyl und Vin für Vinyl steht,
bedeuten.

Bevorzugt handelt es sich dabei um unalkoxyliertes oder ein- bis zehnfach, besonders bevorzugt zwei- bis fünffach ethoxyliertes, propoxyliertes oder gemischt ethoxyliertes und propoxyliertes, mit Adipinsäure, Phthalsäure, Terephthalsäure oder Isophthalsäure verstertes Neopentylglykol, Trimethylolpropan, Trimethylolethan oder Pentaerythrit.

Die Umsetzung der Alkohole mit einem Alkylenoxid ist dem Fachmann an sich bekannt. Mögliche Durchführungsformen finden sich in Houben-Weyl, Methoden der Organischen Chemie, 4. Auflage, 1979, Thieme Verlag Stuttgart, Hrsg. Heinz Kropf, Band 6/1a, Teil 1, Seiten 373 bis 385.

Werden gemischt alkoxylierte Alkohole verwendet, so können die darin enthaltenen unterschiedlichen Alkoxygruppen zueinander im molaren Verhältnis von beispielsweise 0,05 - 20 : 1, bevorzugt 0,1 -10 : 1 und besonders bevorzugt 0,2 - 5 : 1 stehen.

An die Viskosität der erfindungsgemäß einsetzbaren Polyalkohole werden keine besonderen Ansprüche gestellt, außer dass sie bei einer Temperatur bis zu ca. 80°C problemlos pumpbar sein sollten, bevorzugt sollten sie eine Viskosität unter 1000 mPas aufweisen, bevorzugt unter 800 mPas und ganz besonders bevorzugt unter 500 mPas.

Werden als Polyalkohole drei- oder höherwertige Polyalkohole in die Veresterung eingesetzt, so kann es für deren erfindungsgemäße Verwendung als Radikalvernetzer sinnvoll sein, die Polyalkohole lediglich teilzuverestern. Dies bedeutet, dass bei einem n-wertigen Polyalkohol lediglich mindestens 2 der n Hydroxygruppen mit der Carbonsäure B verestert werden.

Für n=3 beträgt der Veresterungsgrad mindestens 2, für n=4 mindestens 2, bevorzugt mindestens 2,5 und besonders bevorzugt mindestens 3, für n=5 oder größer mindestens 2, bevorzugt mindestens 3 und besonders bevorzugt mindestens 4.

In einem solchen Fall berechnet sich der einzusetzende stöchiometrische Überschuss an Carbonsäure B auf den angestrebten Veresterungsgrad, beträgt also beispielsweise das 2/n-fache für einen Veresterungsgrad von 2 bei einem n-fachem Polyalkohol der oben angegebenen molaren Überschüsse. Selbstverständlich kann die Veresterung auch, z.B. durch Kühlung oder Verdünnung, abgebrochen werden, wenn der gewünschte Veresterungsgrad erreicht ist.

Erfindungsgemäß einsetzbare ethylenisch ungesättigte Carbonsäuren B sind solche Verbindungen, die mindestens eine Carboxylgruppe (-COOH), bevorzugt eine, und mindestens eine ethylenisch ungesättigte Gruppe aufweisen.

Die erfindungsgemäß einsetzbaren Carbonsäuren können aliphatisch, cycloaliphatisch oder aromatisch sein, bevorzugt aliphatisch oder cycloaliphatisch und ganz besonders bevorzugt aliphatisch, geradkettig oder verzweigt und gegebenenfalls substituiert mit funktionellen Gruppen.

In der Regel weisen die Carbonsäuren drei bis zehn Kohlenstoffatome auf, bevorzugt drei bis fünf und besonders bevorzugt drei bis vier.

Beispiele für ethylenisch ungesättigte Carbonsäuren B sind Acrylsäure, Methacrylsäure, Ethacrylsäure, Maleinsäure einschließlich deren Anhydrid, Fumarsäure, Itaconsäure, Citraconsäure, Mesaconsäure, Vinylessigsäure, Allylessigsäure oder Crotonsäure.

Bevorzugte Carbonsäuren B sind $\alpha,\beta$-ungesättigte Carbonsäuren.

Besonders bevorzugt sind Methacrylsäure und Acrylsäure, in dieser Schrift (Meth)acrylsäure genannt, ganz besonders bevorzugt ist Acrylsäure.

Weitere bevorzugte Ester von Polyalkoholen sind im folgenden angegeben:

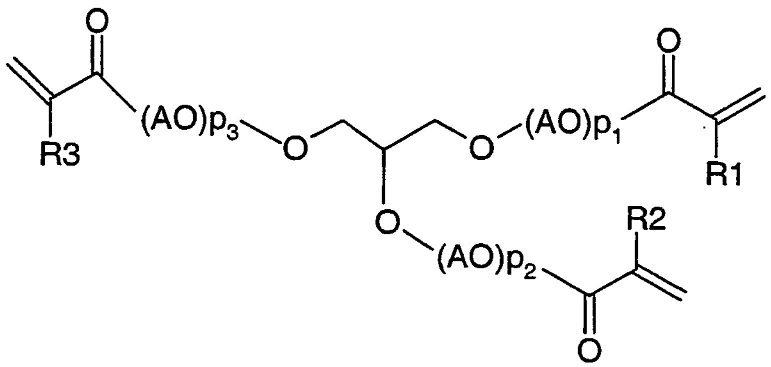

Ia,

mit

AO bedeutet für jedes AO unabhängig voneinander EO oder PO,

wobei EO bedeutet O-CH2-CH2-,

PO bedeutet unabhängig voneinander O-CH2-CH(CH3)- oder O-CH(CH3)-CH2-

p1 + p2 + p3 ist eine ganze Zahl zwischen 0 und 5, bevorzugt 0 oder 3, 4 oder 5,

R1, R2, R3 unabhängig voneinander H oder CH3.

Die EO bzw. PO Einheiten, sind so eingebaut, dass Polyether und keine Peroxide entstehen.

Bevorzugt sind die obigen Ester F, wobei AO EO oder PO, insbesondere EO bedeutet.

Besonders bevorzugt sind Ester F, wobei p1, p2 + p3 = 3 oder p1 = p2 = p3 = 0 ist.

Bevorzugt sind außerdem obige Ester F, wobei mindestens ein AO PO und mindestens ein weiteres AO EO bedeutet.

Des weiteren wird die Aufgabe durch Ester F der Formel Ib gelöst:

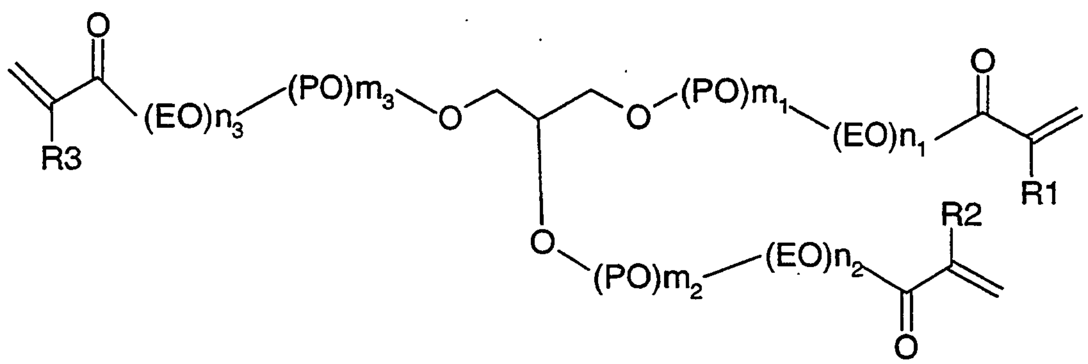

mit

EO bedeutet O-CH2-CH2-

PO bedeutet unabhängig voneinander O-CH2-CH(CH3)- oder O-CH(CH3)-CH2-

m1 +m2+m3+n1+n2+n3 ist 3,4 oder 5,

m1 + m2 + m3 ist 1, 2, 3, oder 4,

R1, R2, R3 unabhängig voneinander H oder CH3.

Oder auch durch Ester F der Formel Ic:

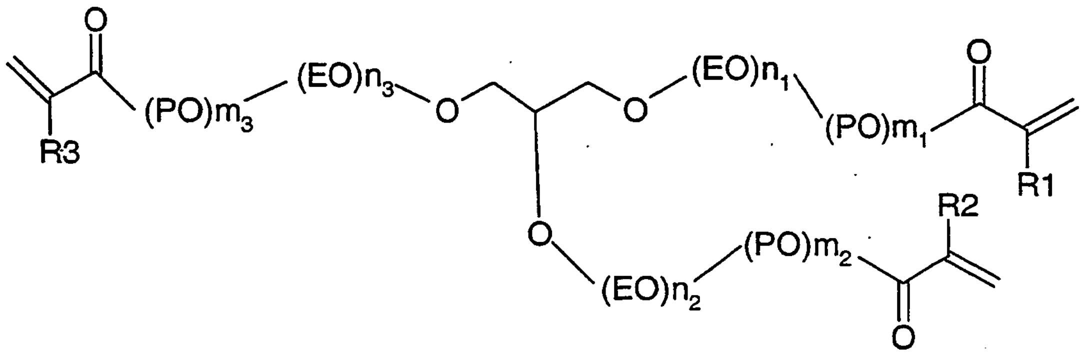

mit

EO bedeutet O-CH2-CH2-

PO bedeutet unabhängig voneinander O-CH2-CH(CH3)- oder O-CH(CH3)-CH2-

m1 +m2+m3+n1 + n2 + n3 ist 3,4 oder 5,

m1 + m2 + m3 ist 1, 2, 3, oder 4,

R1, R2, R3 unabhängig voneinander H oder CH3.

Bei den obigen Estern bevorzugt sind Ester F, wobei m1 + m2 + m3 + n1 + n2 + n3 oder p1 + p2 + p3 gleich 3 ist.
Bei den obigen Estern bevorzugt sind Ester F, wobei m1 + m2 + m3 + n1 + n2 + n3 oder p1 + p2 + p3 gleich 5 ist.
Besonders bevorzugt sind Ester F, wobei insgesamt 3 PO vorliegen.
Ganz besonders bevorzugt sind Ester F, wobei in jeder der 3 Alkoxyketten des Glycerins mindestens 1 PO vorliegt.
Ganz besonders bevorzugt sind Ester F, bei den R1, R2 und R3 identisch sind, insbesondere wenn R1, R2 und R3 H bedeuten.
Des weiteren sind folgende Ester bevorzugt:

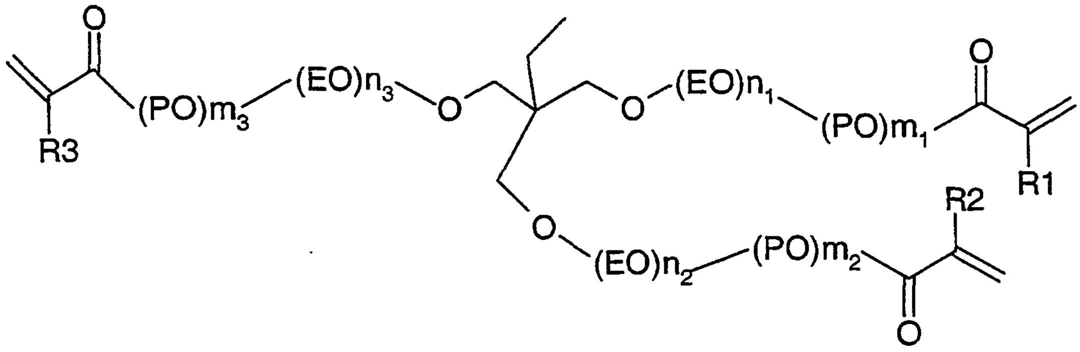

Id

oder

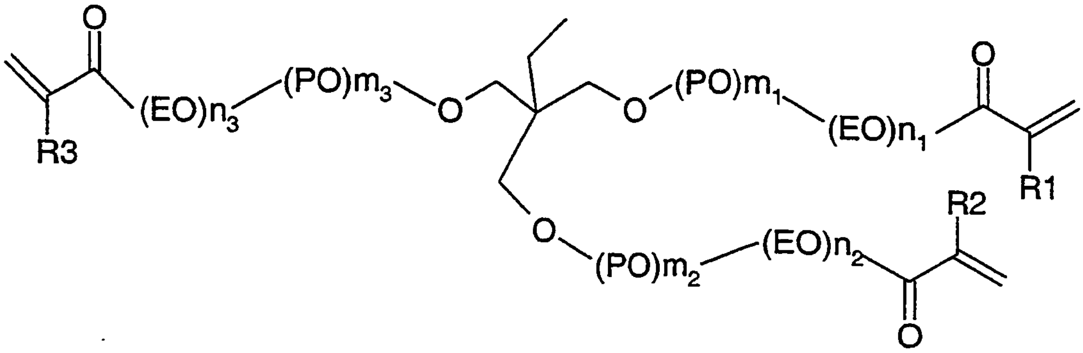

Ie

wobei jeweils in Formel Id oder Ie EO bedeutet O-CH2-CH2-, PO bedeutet unabhängig voneinander O-CH2-CH(CH3)- oder O-CH(CH3)-CH2-,

n1 + n2 + n3 ist 28, 29, 30, 31, 32, 33, 34,35, 36, 37, 38, 39, 40, 41, 42, 43, 44, 45, 46, 47, 48, 49, 50, 51, 52, 53, 54, 55, 56, 57, 58, 59 oder 60,
m1 + m2 + m3 ist 4, 5, 6, 7, 8, 9, 10, 11, 12 oder 13,
R1, R2, R3 bedeuten unabhängig voneinander H oder CH3.

Die EO bzw. PO Einheiten, sind so eingebaut, dass Polyether und keine Peroxide entstehen.

Bevorzugt sind Ester F mit obiger Bedeutung, wobei n1, n2, n3 unabhängig voneinander 9, 10, 11, 12, 13, 14, 15, 16, 17, 18, 19 oder 20 bedeuten.

Besonders bevorzugt sind Ester F mit obiger Bedeutung, wobei n1, n2, n3 unabhängig voneinander 9, 10 oder 11 bedeuten.

Besonders bevorzugt sind Ester F mit obiger Bedeutung, wobei n1, n2, n3 unabhängig voneinander 15, 16, 17, 18, 19 oder 20 bedeuten.

Bevorzugt sind Ester F mit obigen Bedeutungen, wobei n1 + n2 + n3 gleich 28, 29, 30, 31 oder 32 ist.

Bevorzugt sind Ester F mit obigen Bedeutungen, wobei n1 + n2 + n3 gleich 45, 46, 47, 48, 49, 50, 51, 52, 53, 54, 55, 56, 57, 58, 59 oder 60 ist.

Besonders bevorzugt sind Ester F mit obigen Bedeutungen, wobei n1 + n2 + n3 gleich 30 ist.

Besonders bevorzugt sind Ester F mit obigen Bedeutungen, wobei n1 + n2 + n3 gleich 50 ist.

Insbesondere bevorzugt sind Ester F mit obigen Bedeutungen, wobei n1 = n2 = n3 = 10 ist.

Insbesondere bevorzugt sind Ester F mit obigen Bedeutungen, wobei n1 = n2 =17 und n3== 16 ist.

Außerdem bevorzugt sind Ester F mit obiger Bedeutung, wobei m1, m2, m3 unabhängig voneinander 1, 2, 3, 4 oder 5 bedeuten.

Besonders bevorzugt sind Ester F mit obiger Bedeutung, wobei m1, m2, m3 unabhängig voneinander 1, 2 oder 3 bedeuten.

Besonders bevorzugt sind Ester F mit obiger Bedeutung, wobei m1, m2, m3 unabhängig voneinander 2, 3, 4 oder 5 bedeuten.

Bevorzugt sind Ester F mit obigen Bedeutungen, wobei m1 + m2 + m3 gleich 4, 5 oder 6 ist.
Bevorzugt sind Ester F mit obigen Bedeutungen, wobei m1 + m2 + m3 gleich 7, 8, 9, 10, 11, 12 oder 13 ist.
Besonders bevorzugt sind Ester F mit obigen Bedeutungen, wobei m1 + m2 + m3 gleich 5 ist.
Besonders bevorzugt sind Ester F mit obigen Bedeutungen, wobei m1 + m2 + m3 gleich 10 ist.
Insbesondere bevorzugt sind Ester F mit obigen Bedeutungen, wobei $m_i = m_k = 3$ und $m_i = 4$ ist, mit i, k, l alle verschieden und ausgewählt aus der Gruppe bestehend aus1 , 2,3.
Insbesondere bevorzugt sind Ester F mit obigen Bedeutungen, wobei $m_i = m_k = 2$ und $m_i = 1$ ist, mit i, k, l alle verschieden und ausgewählt aus der Gruppe bestehend aus1, 2, 3.
Ganz besonders bevorzugt sind Ester F, bei den R1, R2 und R3 identisch sind, insbesondere wenn R1, R2 und R3 H bedeuten.
Außerdem sind folgende Ester bevorzugt:

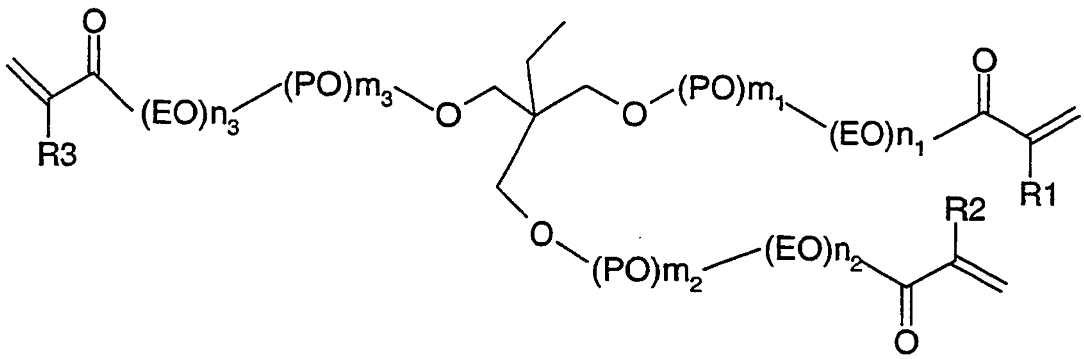

Ie

mit EO bedeutet O-CH2-CH2-,
PO bedeutet unabhängig voneinander O-CH2-CH(CH3)- oder O-CH(CH3)-CH2-,
n1, n2, n3 bedeuten unabhängig voneinander 4,5 oder 6,
n1 + n2 + n3 ist 14, 15 oder 16,
m1, m2, m3 bedeuten unabhängig voneinander 1,2 oder 3,
m1 + m2 + m3 ist 4, 5 oder 6,
R1, R2, R3 bedeuten unabhängig voneinander H oder CH3.
Die EO bzw. PO Einheiten, sind so eingebaut, dass Polyether und keine Peroxide entstehen.
Bevorzugt sind Ester F mit obigen Bedeutungen, wobei n1 + n2 + n3 gleich 15 ist.
Besonders bevorzugt sind Ester F mit obigen Bedeutungen, wobei n1 = n2 = n3 = 5 ist.
Außerdem bevorzugt sind Ester F mit obigen Bedeutungen, wobei m1 + m2 + m3 gleich 5 ist.
Außerdem besonders bevorzugt sind Ester F mit obigen Bedeutungen, wobei m1 = m2 = 2 und m3 =1 ist.
Ganz besonders bevorzugt sind Ester F, bei den R1, R2 und R3 identisch sind, insbesondere wenn R1, R2 und R3 H bedeuten.
Auch bevorzugt sind folgende Ester:

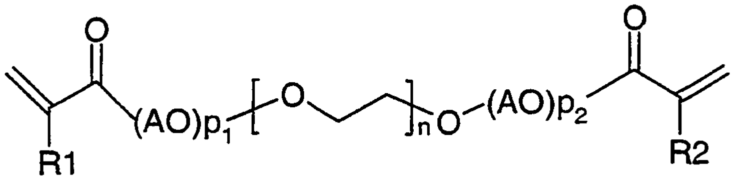

If

mit AO bedeutet für jedes AO unabhängig voneinander -O-CHR3-CHR4- oder -CHR3-CHR4-O-, mit R3 und R4 unabhängig voneinander H, lineares oder verzweigtes C1-C8-Alkyl,

p1 ist 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, oder 35,

p2 ist 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32,

33, 34, oder 35,

n ist 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 447, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100,

R1, R2 unabhängig voneinander H oder CH3,

wobei zumindest für ein AO in (AO)p1, wie auch für mindestens ein AO in (AO)p2 nicht R3 und R4 beide gleichzeitig H bedeuten.

Die AO Einheiten, sind so eingebaut, dass Polyether und keine Peroxide entstehen.

Bevorzugt sind Ester F mit obiger Bedeutung, wobei für alle AOs R3 und R4 unabhängig voneinander die selbe Bedeutung annehmen, d.h. z.B. durch Alkoxylieren von Ethylenglycol oder Polyethylenglycol mit nur einem Alkylenoxyd, z.B. Propylenoxid, zugänglich sind.

Bevorzugt sind Ester F mit obigen Bedeutungen, wobei R3 oder R4 H bedeutet.

Bevorzugt sind Ester F mit obigen Bedeutungen, wobei R3 oder R4 unabhängig voneinander CH3, CH2CH3, (CH2)2-CH3 oder (CH2)7-CH3, bevorzugt CH3 bedeutet.

Außerdem bevorzugt sind Ester F mit obiger Bedeutung, wobei p1 1, 2, 3, 4 oder 5, besonders bevorzugt 1, 2 oder 3, insbesondere 1, bedeutet oder eine Zahl zwischen 14 und 27 bedeutet.

Außerdem bevorzugt sind Ester F mit obiger Bedeutung, wobei p2 1, 2, 3, 4 oder 5, besonders bevorzugt 1, 2 oder 3, insbesondere 1, bedeutet oder eine Zahl zwischen 14 und 27 bedeutet.

Bevorzugt sind Ester F mit obigen Bedeutungen, wobei n eine Zahl zwischen 2 und 50, bevorzugt zwischen 5 und 30, insbesondere zwischen 10 und 26 bedeutet.

Bevorzugt sind Ester F mit obigen Bedeutungen, wobei die Diolkomponente (AO)p1-[-O-CH2-CH2-]n-O-(AO) p2 ein mittleres Molekulargewicht zwischen 300 und 500, insbesondere um 400 aufweist.

Bevorzugt sind Ester F mit obigen Bedeutungen, wobei die Diolkomponente (AO)p1-[-O-CH2-CH2-]n-O-(AO) p2 ein mittleres Molekulargewicht 2000 und 4000, besonders bevorzugt zwischen 2500 und 3500, aufweist.

Bevorzugt sind Diolkomponenten bei Ester F mit obigen Bedeutungen, die bezüglich (AO)p1 und (AO)p2 untereinander symmetrisch aufgebaut sind. Unter symmetrisch wird verstanden, dass p1 und p2 in etwa gleich sind, d.h. der Absolutbetrag der Differenz zwischen p1 und p2 3 oder weniger, bevorzugt 2 oder weniger, besonders bevorzugt 1 oder weniger, insbesondere gleich 0 ist.

Bevorzugt sind weiterhin Diolkomponenten bei Ester F mit obigen Bedeutungen, die bezüglich (AO)p1 und (AO)p2 untereinander strukturähnlich aufgebaut sind. Unter strukturähnlich wird verstanden, dass die jeweiligen (AO) p1- und (AO)p2-Komponenten durch gleichzeitige Synthese an dem (Poly)ethylenglycol hergestellt werden, also entweder beide aus Gemischen verschiedener Alkoxide (statistische (AO)p-Komponenten oder aus sequentieller Synthese (Block (AO)p-Komponenten) zugänglich sind. Insbesondere sind solche Diolkomponenten bevorzugt, bei denen alle AO die gleiche Bedeutung annehmen und dabei bevorzugt aus Propylenoxid entstehen.

Bevorzugt sind Ester F mit obigen Bedeutungen, wobei R1 und R2 identisch, bevorzugt H sind.

Erfindungsgemäß können die Ester F der oben genannten Formel mit den angegebenen Bedeutungen zur Herstellung von wässrige Flüssigkeiten absorbierende Hydrogel-formende Polymere verwendet werden, insbesondere als Innenvernetzer. Weitere bevorzugte Innenvernetzer sind Di(meth)acrylate, insbesondere Diacrylate von Polypropylenglycol mit 2, 3, 4 oder 5, insbesondere 2 oder 3 Propylenglycoleinheiten.

In den obigen Gemischen kann eine Verbindung durch eine der folgenden Formeln bevorzugt repräsentiert werden:

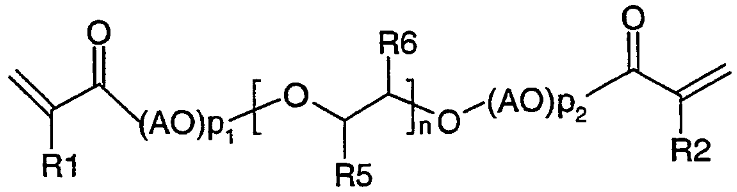

Ig,

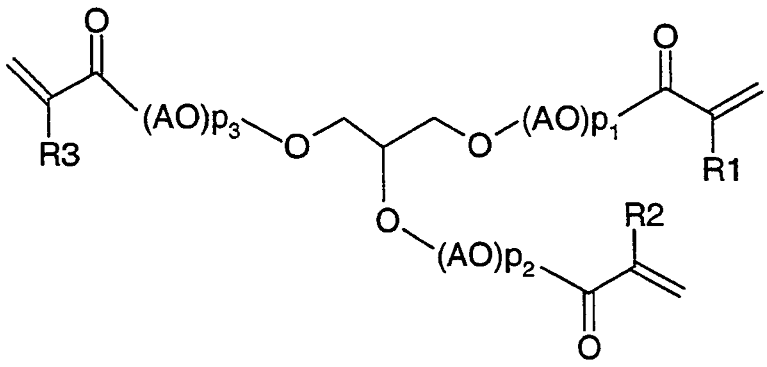

Ih,

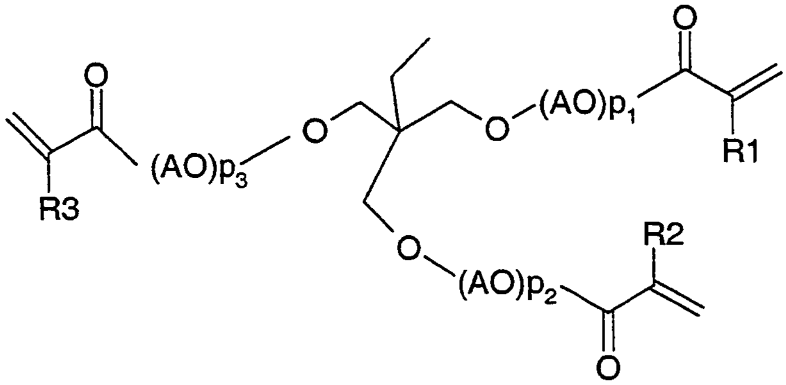

Ii,

oder

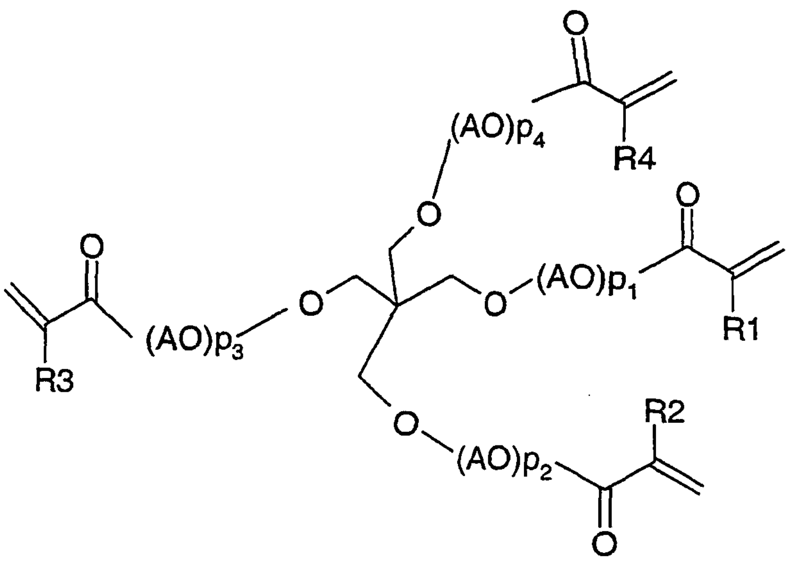

Ij

mit AO bedeutet für jedes AO unabhängig voneinander -O-CHR7-CHR8- oder -CHR7-CHR8-O-, mit R7 und R8 unabhängig voneinander H, lineares oder verzweigtes C1-C8-Alkyl,

R5 und R6 unabhängig voneinander H, lineares oder verzweigtes C1-C8-Alkyl,

n ist 1, 2 oder 3

p1 ist 0, 1 oder 2,

p2 ist 0, 1 oder 2,

p3 ist 0, 1 oder 2,

p4 ist 0, 1 oder 2,

R1, R2, R3, R4 unabhängig voneinander H oder CH3,

Auch durch eine der folgenden Formeln kann bevorzugt ein Ester im Gemisch repräsentiert werden:

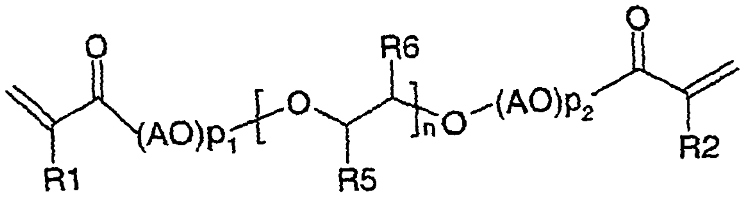

Ik,

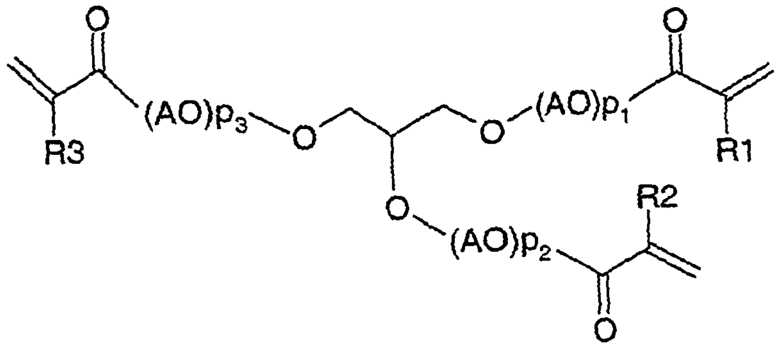

Il,

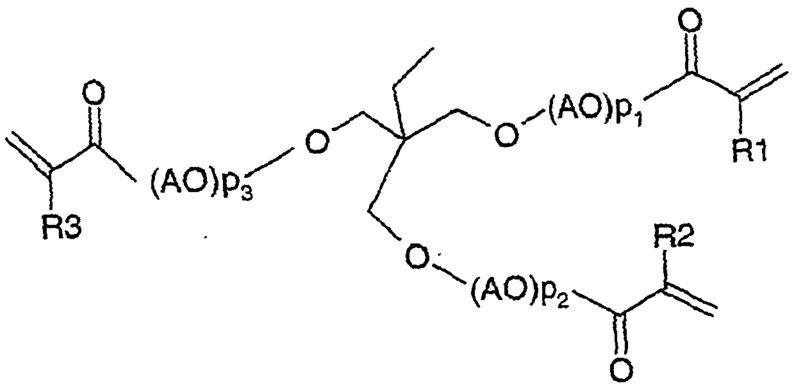

Im,

oder

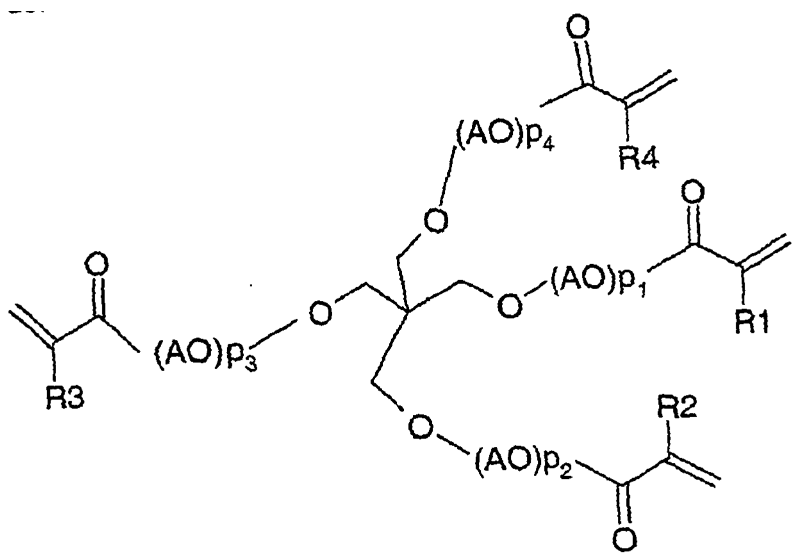

In

mit AO bedeutet für jedes AO unabhängig voneinander -O-CHR7-CHR8- oder-CHR7-CHRB-O-, mit R7 und R8 unabhängig voneinander H, lineares oder verzweigtes C1-C8-Alkyl,

R5 und R6 unabhängig voneinander H, lineares oder verzweigtes C1-C8-Alkyl,

n ist 1, 2, 3, 4, 5, 6, 7, 8, 9, 10, 11, 12, 13, 14, 15, 16, 17, 18, 19 oder 20,

p1 ist 7, 8, 9, 10, 11, 12, 13, 14, 15, 16, 17, 18, 19 oder 20,

p2 ist 7, 8, 9, 10, 11, 12, 13, 14, 15, 16, 17, 18, 19 oder 20,

p3 ist 7, 8, 9, 10, 11, 12, 13, 14, 15, 16, 17, 18, 19 oder 20,

p4 ist 7, 8, 9, 10, 11, 12, 13, 14, 15, 16, 17, 18, 19 oder 20,

R1, R2, R3, R4 unabhängig voneinander H oder CH3.

Besonders bevorzugt sind solche Gemische, bei denen AO für jedes AO unabhängig voneinander EO oder PO bedeutet, wobei EO bedeutet O-CH2-CH2- und PO bedeutet unabhängig voneinander O-CH2-CH(CH3)- oder O-CH(CH3)-CH2- und R5 und R6 unabhängig voneinander H oder CH3 bedeutet. Insbesondere sind solche bevorzugt bei denen AO Blockpolymere aus EO und PO bedeutet und die PO Einheiten endständig liegen und mit Acrylsäure verestert sind.

Insbesondere sind Gemische aus Estern ausgewählt aus der Gruppe repräsentiert durch die Formeln Ig, Ih, Ii oder Ij mit solchen ausgewählt aus der Gruppe repräsentiert durch die Formeln Ik, Il, Im oder In bevorzugt.

Weiterhin sind folgende Ester bevorzugt als eine Komponente des Gemisches: Butandioldiacrylat, Butandiol-1 EO-diacrylat, Butandiol-2EO-diacrylat, Butandiol-1 EO-1 PO-diacrylat, Butandiol-2PO-diacrylat, Butandiol-3EO-diacrylat, Butandiol-2EO-1PO-diacrylat, Butandiol-1 EO-2PO-diacrylat, Butandiol-3PO-diacrylat, Trimethylolpropantriacrylat, Trimethylolpropandiacrylat, Trimethylolpropan-3EO-triacrylat, Trimethylolpropan-3EO-diacrylat, Trimethylolpropan-2EO-1PO-triacrylat, Trimethylolpropan-2EO-1PO-diacrylat, Trimethylolpropan-1 EO-2PO-triacrylat, Trimethylolpropan-1 EO-2PO-diacrylat, Trimethylolpropan-3PO-triacrylat, Trimethylolpropan-3PO-diacrylat, Trimethylolpropan-4EO-triacrylat, Trimethylolpropan-4EO-diacrylat, Trimethylolpropan-1 PO-3EO-triacrylat, Trimethylolpropan-1 PO-3EO-diacrylat, Trimethylolpropan-2EO-2PO-triacrylat, Trimethylolpropan-2EO-2PO-diacrylat, Trimethylolpropan-1EO-2PO-triacrylat, Trimethylolpropan-1EO-3PO-diacrylat, Trimethylolpropan-4PO-triacrylat, Trimethylolpropan-4PO-diacrylat, Glycerintriacrylat, Glycerindiacrylat, Glycerin-3EO-triacrylat, Glycerin-3EO-diacrylat, Glycerin-2EO-1PO-triacrylat, Glycerin-2EO-1 PO-diacrylat, Glycerin-1 EO-2PO-triacrylat, Glycerin-1 EO-2PO-diacrylat, Glycerin-3PO-triacrylat, Glycerin-3PO-diacrylat, Glycerin-4EO-triacrylat, Glycerin-4EO-diacrylat, Glycerin-1PO-3EO-triacrylat, Glycerin-1PO-3EO-diacrylat, Glycerin-2EO-2PO-triacrylat, Glycerin-2EO-2PO-diacrylat, Glycerin-1EO-2PO-triacrylat, Glycerin-1 EO-3PO-diacrylat, Glycerin-4PO-triacrylat, Glycerin-4PO-diacrylat, Pentaerytrittriacrylat, Pentaerytritdiacrylat, Pentaerytrit-3EO-triacrylat, Pentaerytrit-3EO-diacrylat, Pentaerytrit-2EO-1 PO-triacrylat, Pentaerytrit-2EO-1 PO-diacrylat, Pentaerytrit-1 EO-2PO-triacrylat, Pentaerytrit-1 EO-2PO-diacrylat, Pentaerytrit-3PO-triacrylat, Pentaerytrit-3PO-diacrylat, Pentaerytrit-4EO-triacrylat, Pentaerytrit-4EO-diacrylat, Pentaerytrit-1PO-3EO-triacrylat, Pentaerytrit-1PO-3EO-diacrylat, Pentaerytrit-2EO-2PO-triacrylat, Pentaerytrit-2EO-2PO-diacrylat, Pentaerytrit-1EO-2PO-triacrylat, Pentaerytrit-1EO-3PO-diacrylat, Pentaerytrit-4PO-triacrylat, Pentaerytrit-4PO-diacrylat, Pentaerytrittetraacrylat, Pentaerytrit-5EO-tetraacrylat, Pentaerytrit-3EO-tetraacrylat, Pentaerytrit-4EO-1PO-tetraacrylat, Pentaerytrit-2EO-1PO-tetraacrylat, Pentaerytrit-3EO-2PO-tetraacrylat, Pentaerytrit-1EO-2PO-tetraacrylat, Pentaerytrit-3PO-2EO-tetraacrylat, Pentaerytrit-3PO-tetraacrylat, Pentaerytrit-4EO-tetraacrylat, Pentaerytrit-1EO-4PO-tetraacrylat, Pentaerytrit-5PO-tetraacrylat, Pentaerytrit-1 PO-3EO-tetraacrylat, Pentaerytrit-2EO-2PO-tetraacrylat, Pentaerytrit-1EO-3PO-tetraacrylat, Pentaerytrit-4PO-tetraacrylat.

Besonders bevorzugt sind Estermischungen bei denen die eine Komponente durch eine der folgenden Verbindungen repräsentiert ist: Glycerin-3EO-triacrylat (G3EOTA), Glycerin-3PO-triacrylat (G3POTA), Glycerintriacrylat (GTA), Trimethylolpropan-3EO-triacrylat (TMP3EOTA), Trimethylolpropropan-3PO-triacrylat (TMP3POTA) und Trimethylolpropantriacrylat (TMPTA). Die andere Komponente ist bevorzugt Trimethylpropan, das zuerst mit 5PO und anschließend mit 30EO alkoxyliert und anschließend vollständig mit Acrylsäure verestert ist (TMP5PO30EOTA), sowie das Trimethylpropan, das zuerst mit 5PO und anschließend mit 30EO alkoxyliert und anschließend vollständig mit Acrylsäure verestert ist (TMP30EO5POTA)

Ganz besonders bevorzugt sind die Mischungen:

G3EOTA mit TMP30EO5POTA G3POTA mit TMP30EO5POTA, GTA mit TMP30EO5POTA.

Diese Vernetzer werden besonders bevorzugt mit solchen der Formel- Im kombiniert. Die weiteren Ausführungen gelten sowohl für die Herstellung der einzelnen Ester aus Polyalkoholen, wie auch für die Herstellung des Estergemisches aus Gemischen von Polyalkoholen. Im weiteren wird stellvertretend von einem Ester F gesprochen.

Die weitere Aufgabe wird gelöst durch ein Verfahren zur Herstellung eines Esters F eines Polyalkohols A mit (Meth)acrylsäure, umfassend die Schritte

a) Umsetzung eines Polyalkohols A mit (Meth)acrylsäure in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Esters F,

b) gegebenenfalls Entfernen zumindest eines Teils des in a) entstehenden Wassers aus dem Reaktionsgemisch, wobei b) während und/oder nach a) erfolgen kann, f) gegebenenfalls Neutralisation des Reaktionsgemischs,

h) falls ein Lösungsmittel E eingesetzt wurde gegebenenfalls Entfernen dieses Lösungsmittels durch Destillation und/oder

i) Strippen mit einem unter den Reaktionsbedingungen inerten Gas.

Bevorzugt beträgt hierbei

- der molare Überschuss (Meth)acrylsäure zu dem Polyalkohol A je zu veresternder Hydroxygruppe in A mindestens 1,05:1 und
- verbleibt die in dem nach dem letzten Schritt erhaltenen Reaktionsgemisch enthaltene, gegebenenfalls neutralisierte (Meth)acrylsäure im wesentlichen im Reaktionsgemisch.

Unter (Meth)acrylsäure wird bei der vorliegenden Erfindung Methacrylsäure, Acrylsäure oder Gemische aus Methacrylsäure und Acrylsäure verstanden. Bevorzugt ist Acrylsäure.

Wird der Ester F in Reinform gewünscht kann er durch bekannte Auftrennungsverfahren gereinigt werden.

Der molare Überschuss von (Meth)acrylsäure zu zu A beträgt (je zu veresternder Hydroxygruppe im Polyalkohol A) mindestens 1,05 : 1, bevorzugt mindestens 1,1:1, besonders bevorzugt mindestens 1,25:1, ganz besonders bevorzugt mindestens 1,5:1 und insbesondere mindestens 2,5:1.

In einer bevorzugten Ausführungsform wird (Meth)acrylsäure in einem Überschuss von beispielsweise größer als 10:1, bevorzugt größer als 20:1, besonders bevorzugt größer als 40:1, ganz besonders bevorzugt größer 100:1, insbesondere größer 150:1 und speziell größer als 200:1 eingesetzt.

Die so erhältlichen Veresterungsprodukte können im wesentlichen ohne weitere Reinigung, besonders ohne wesentliche Abtrennung des Überschusses an (Meth)acrylsäure und des Gehalts an Veresterungskatalysator C, als Radikalvernetzer in Hydrogelen eingesetzt werden.

Unter Vernetzung wird in dieser Schrift, wenn nicht anders erwähnt, die Radikalvernetzung (Gelvernetzung, Innenvernetzung, Quervernetzung von linearem oder schwach vernetzten Polymer) verstanden. Diese Vernetzung kann

über radikalische oder kationische Polymerisationsmechanismen oder andere, beispielsweise Michael-Addition, Veroder Umesterungsmechanismen erfolgen, bevorzugt durch radikalische Polymerisation.

Wässrige Flüssigkeiten absorbierende Hydrogel-formende Polymere sind bevorzugt solche mit einer Absorption von destilliertem Wasser von mindestens dem Eigengewicht, bevorzugt dem 10-fachen Eigengewicht, insbesondere dem 20-fachen Eigengewicht, diese Absorption wird bevorzugt auch unter einem Druck von 0,7 psi erreicht.

Erfindungsgemäß einsetzbare Polyalkohole A sind Verbindungen, die mindestens zwei Hydroxyfunktionen (-OH) aufweisen, bevorzugt mindestens drei, besonders bevorzugt drei bis zehn, ganz besonders bevorzugt drei bis sechs und insbesondere drei bis vier.

Die Polyalkohole können aliphatisch, cycloaliphatisch oder aromatisch sein, bevorzugt aliphatisch oder cycloaliphatisch und ganz besonders bevorzugt aliphatisch, geradkettig oder verzweigt und gegebenenfalls substituiert mit funktionellen Gruppen.

In der Regel weisen die Polyalkohole zwei bis 50 Kohlenstoffatome auf und bevorzugt drei bis 40.

Die Umsetzung von Polyalkoholen mit einem Alkylenoxid ist dem Fachmann an sich bekannt. Mögliche Durchführungsformen finden sich in Houben-Weyl, Methoden der Organischen Chemie, 4. Auflage, 1979, Thieme Verlag Stuttgart, Hrsg. Heinz Kropf, Band 6/1a, Teil 1, Seiten 373 bis 385.

Zur Herstellung der Polyalkohole A kann z.B. zuerst das Polyol mit EO abreagiert um das erwünschte Polyethylenpolyol zu erhalten und dann anschließend wird mit PO umgesetzt.

Dies kann z.B. geschehen in dem etwa 72 g Glycol (Beispiel für Polyol) oder die entsprechende Menge Polyethylenglycol mit 0,5 g KOH, 45% in Wasser, in einem Autoklaven vorgelegt wird und zusammen bei 80°C und reduziertem Druck (ca. 20 mbar) entwässert wird. Dann wird bei 145 bis 155°C die entsprechende Menge an Propylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 150°C nachgerührt. Anschließend wird die entsprechende Menge an Propylenoxid bei 120 bis 130°C über längere Zeit bei erhöhtem Druck zudosiert und ebenfalls abreagieren gelassen. Nach Spülen mit Inertgas und Abkühlen auf 60°C wird der Katalysator durch Zugabe von Natriumpyrophosphat und anschließende Filtration abgetrennt.

Es können aber auch kommerziell erhältliche alkoxylierte Glycole, wie z.B. Di- und Tripropylenglycol oder Triblockpolymere vom PO-EO-PO Typ, wie z.B. die Pluronic® RPE Polymere, vom Typ 1720, 1740, 2035,2510,2520, 2525 oder 3110 eingesetzt werden.

An die Viskosität der erfindungsgemäß einsetzbaren Polyalkohole werden keine besonderen Ansprüche gestellt, außer dass sie bei einer Temperatur bis zu ca. 80°C problemlos pumpbar sein sollten, bevorzugt sollten sie eine Viskosität unter 1000 mPas aufweisen, bevorzugt unter 800 mPas und ganz besonders bevorzugt unter 500 mPas.

Erfindungsgemäß einsetzbare Veresterungskatalysatoren C sind Schwefelsäure, Aryl- oder Alkylsulfonsäuren oder Gemische davon. Beispiele für Arylsulfonsäuren sind Benzolsulfonsäure, para-Toluolsulfonsäure oder Dodecylbenzolsulfonsäure, Beispiele für Alkylsulfonsäuren sind Methansulfonsäure, Ethansulfonsäure oder Trifluormethansulfonsäure. Auch stark saure Ionentauscher oder Zeolithe sind als Veresterungskatalysatoren einsetzbar. Bevorzugt sind Schwefelsäure und Ionentauscher.

Erfindungsgemäß einsetzbare Polymerisationsinhibitoren D sind beispielsweise Phenole wie Alkylphenole, beispielsweise o-, m- oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), 4,4'-Oxydiphenyl, 3,4-Methylendioxydiphenol (Sesamol), 3,4-Dimethylphenol, Hydrochinon, Brenzcatechin (1,2-Dihydroxybenzol), 2-(1'-Methylcyclohex-1'-yl)-4,6-dimethylphenol, 2- oder 4-(1'-Phenyl-eth-1'-yl)-phenol, 2-tert-Butyl-6-methylphenol, 2,4,6-Tris-tert-Butylphenol, 2,6-Di-tert.-butylphenol, 2,4-Di-tert.-butylphenol, 4-tert.-Butylphenol, Nonylphenol [11066-49-2], Octylphenol [140-66-9], 2,6-Dimethylphenol, Bisphenol A, Bisphenol F, Bisphenol B, Bisphenol C, Bisphenol S, 3,3',5,5'-Tetrabromobisphenol A, 2,6-Di-tert-Butyl-p-kresol, Koresin® der BASF AG, 3,5-Di-tert-Butyl-4-hydroxybenzoesäuremethylester, 4-tert-Butylbrenzcatechin, 2-Hydroxybenzylalkohol, 2-Methoxy-4-methylphenol, 2,3,6-Trimethylphenol, 2,4,5-Trimethylphenol, 2,4,6-Trimethylphenol, 2-Isopropylphenol, 4-Isopropylphenol, 6-Isopropyl-m-Kresol, n-Octadecyl-beta-(3,5-di-tert-butyl-4-hydroxyphenyl)propionat, 1,1,3-Tris-(2-methyl-4-hydroxy-5-tert-butylphenyl)butan, 1,3,5-Trimethyl-2,4,6-tris-(3,5-di-tert-butyl-4-hydroxybenzyl)benzol, 1,3,5,-Tris-(3,5-di-tert-butyl-4-hydroxybenzyl)isocyanurat, 1,3,5,-Tris-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrit-tetrakis-[beta-(3,5,-di-tert-butyl-4-hydroxyphenyl)-propionat], 2,6-Di-*tert*.-butyl-4-dimethylaminomethyl-phenol, 6-sek.-Butyl-2,4-dinitrophenol, Irganox® 565, 1141, 1192, 1222 und 1425 der Firma Ciba Spezialitätenchemie, 3-(3',5'-Di-*tert*.-butyl-4'-hydroxyphenyl)propionsäureoctadecylester, 3-(3',5'-Di-*tert*-butyl-4'-hydroxyphenyl)propionsäurehexadecylester, 3-(3',5'-Di-*tert*.-butyl-4'-hydroxyphenyl)propionsäureoctylester, 3-Thia-1,5-pentandiol-bis-[(3',5'-di-*tert*-butyl-4'-hydroxyphenyl)propionat], 4,8-Dioxa-1,11-undecandiol-bis-[(3',5'-di-*tert*.-butyl-4'-hydroxyphenyl)propionat], 4,8-Dioxa-1,11-undecandiol-bis-[(3'-*tert*.-butyl-4'-hydroxy-5'-methylphenyl)propionat], 1,9-Nonandiol-bis-[(3',5'-di-*tert*.-butyl-4'-hydroxyphenyl)propionat], 1,7-Heptandiamin-bis[3-(3',5'-di-*tert*-butyl-4'-hydroxyphenyl)propionsäureamid], 1,1-Methandiamin-bis[3-(3',5'-di-*tert*-butyl-4'-hydroxyphenyl)propionsäureamid],

3-(3',5'-di-*tert*.-Butyl-4'-hydroxyphenyl)propionsäurehydrazid, 3-(3',5'-di-Methyl-4'-hydroxyphenyl)propionsäurehydrazid, Bis(3-*tert*.-Butyl-5-ethyl-2-hydroxy-phen-1-yl)methan, Bis(3,5-di-*tert*-Butyl-4-hydroxy-phen-1-yl)methan, Bis[3-(1'-methylcycl*ohex-1'-yl)-5-methyl-2-hydroxy-phen-1-yl]methan, Bis(3-*tert*-Butyl-2-hydroxy-5-methyl-phen-1-yl)methan, 1,1-Bis(5-*tert*.-Butyl-4-hydroxy-2-methyl-phen-1-yl)ethan, Bis(5-*tert*.-Butyl-4-hydroxy-2-methyl-phen-1-yl)sulfid, Bis (3-*tert*.-Butyl-2-hydroxy-5-methyl-phen-1-yl)sulfid, 1,1-Bis(3,4-Dimethyl-2-hydroxy-phen-1-yl)-2-methylpropan, 1,1-Bis (5-*tert*.-Butyl-3-methyl-2-hydroxy-phen-1-yl)-butan, 1,3,5-Tris[1'-(3",5"-di-*tert*.-Butyl-4"-hydroxyphen-1"-yl)-meth-1'-yl]-2,4,6-trimethylbenzol, 1,1,4-Tris(5'-*tert*: Butyl-4'-hydroxy-2'-methyl-phen-1'-yl)butan, Aminophenole, wie z.B. para-Aminophenol, Nitrosophenole, wie z.B. para-Nitrosophenol, p-Nitroso-o-Kresol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, 3,5-Di-tert-butyl-4-hydroxyanisol, 3-Hydroxy-4-methoxybenzylalkohol, 2,5-Dimethoxy-4-hydroxybenzylalkohol (Syringaalkohol), 4-Hydroxy-3-methoxybenzaldehyd (Vanillin), 4-Hydroxy-3-ethoxybenzaldehyd (Ethylvanillin), 3-Hydroxy-4-methoxybenzaldehyd (Isovanillin), 1-(4-Hydroxy-3-methoxyphenyl)ethanon (Acetovanillon), Eugenol, Dihydroeugenol, Isoeugenol, Tocopherole, wie z.B. alpha-, beta-, gamma-, delta- und epsilon-Tocopherol, Tocol, alpha-Tocopherolhydrochinon, sowie 2,3-Dihydro-2,2-dimethyl-7-hydroxybenzofuran (2,2-Dimethyl-7-hydroxycumaran), Chinone und Hydrochinone wie Hydrochinon oder Hydrochinonmonomethylether, 2,5-Di-*tert*.-Butylhydrochinon, 2-Methyl-p-hydrochinon, 2,3-Dimethylhydrochinon, Trimethylhydrochinon, 4-Methylbrenzcatechin, tert-Butylhydrochinon, 3-Methylbrenzcatechin, Benzochinon, 2-Methyl-p-hydrochinon, 2,3-Dimethylhydrochinon, Trimethylhydrochinon, 3-Methylbrenzcatechin, 4-Methylbrenzcatechin, tert-Butylhydrochinon, 4-Ethoxyphenol, 4-Butoxyphenol, Hydrochinonmonobenzylether, p-Phenoxyphenol, 2-Methylhydrochinon, 2,5-Di-tert.-Butylhydrochinon, Tetramethyl-p-benzochinon, Diethyl-1,4-cyclohexandion-2,5-dicarboxylat, Phenyl-p-benzochinon, 2,5-Dimethyl-3-benzyl-p-benzochinon, 2-Isopropyl-5-methyl-p-benzochinon (Thymochinon), 2,6-Diisopropyl-p-benzochinon, 2,5-Dimethyl-3-hydroxy-p-benzochinon, 2,5-Dihydroxy-p-benzochinon, Embelin, Tetrahydroxy-p-benzochinon, 2,5-Dimethoxy-1,4-benzochinon, 2-Amino-5-methyl-p-benzochinon, 2,5-Bisphenylamino-1,4-benzochinon, 5,8-Dihydroxy-1,4-naphthochinon, 2-Anilino-1,4,naphthochinon, Anthrachinon, N,N-Dimethylindoanilin, N,N-Diphenyl-p-benzochinondiimin, 1,4-Benzochinondioxim, Coerulignon, 3,3'-Di-tert-butyl-5,5'-dimethyldiphenochinon, p-Rosolsäure (Aurin), 2,6-Di-tert-butyl-4-benzylidenbenzochinon, 2,5-Di-tert.-Amylhydrochinon, N-Oxyle wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit, 3-Oxo-2,2,5,5-tetramethyl-pyrrolidin-N-oxyl, 1-Oxyl-2,2,6,6-tetramethyl-4-methoxypiperidin, 1-Oxyl-2,2,6,6-tetramethyl-4-trimethylsilyloxypiperidin, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-Oxyl-2,2,6,6-tetramethylpiperidin-4-yl)-succinat, Bis(1-Oxyl-2,2,6,6-tetramethylpiperidin-4-yl)-adipat, 1,10-Dekandisäure-bis(1-Oxyl-2,2,6,6-tetramethylpiperidin-4-yl)ester, Bis(1-Oxyl-2,2,6,6-tetramethylpiperidin-4-yl)-n-butylmalonat, Bis (1-Oxyl-2,2,6,6-tetramethylpiperidin-4-yl)-phthalat, Bis(1-Oxyl-2,2,6,6-tetramethylpiperidin-4-yl)-isophthalat, Bis(1-Oxyl-2,2,6,6-tetramethylpiperidin-4-yl)-terephthalat, Bis(1-Oxyl-2,2,6,6-tetramethylpiperidin-4-yl)-hexahydroterephthalat, N,N'-Bis(1-Oxyl-2,2,6,6-tetramethylpiperidin-4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-Oxyl-2,2,6,6-tetramethylpiperidin-4-yl]triazin, N,N'-Bis(1-Oxyl-2,2,6,6-tetramethylpiperidin-4-yl)-N,N'-bis-formyl-1,6-diaminohexan, 4,4'-Ethylenbis(1-Oxyl-2,2,6,6-tetramethylpiperazin-3-on) aromatische Amine wie Phenylendiamine, N,N-Diphenylamin, N-Nitroso-diphenylamin, Nitrosodiethylanilin, N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, beispielsweise N,N'-Di-*iso*-butyl-p-phenylendiamin, N,N'-Di-*iso*-propyl-p-phenylendiamin, Irganox 5057 der Firma Ciba Spezialitätenchemie, N,N'-Di-*iso*-butyl-p-phenylendiamin, N,N'-Di-*iso*-propyl-p-phenylendiamin, p-Phenylendiamin, N-Phenyl-p-Phenylendiamin, N,N'-Diphenyl-p-phenylendiamin, N-Isopropyl-N-phenyl-p-phenylendiamin, N,N'Di-sec-butyl-p-phenylendiamin (Kerobit® BPD der BASF AG), N-Phenyl-N'-isopropyl-p-phenylendiamin (Vulkanox® 4010 der Bayer AG), N-(1,3-Dimethylbutyl)-N'-phenyl-p-phenylendiamin, N-Phenyl-2-naphthylamin, Imoinodibenzyl, N,N'-Diphenylbenzidin, N-Phenyltetraanilin, Acridon, 3-Hydroxydiphenylamin, 4-Hydroxydiphenylamin, Hydroxylamine wie N,N-Diethylhydroxylamin, Harnstoffderivate wie Harnstoff oder Thioharnstoff, phosphorhaltige Verbindungen, wie Triphenylphosphin, Triphenylphosphit, Hypophosphorige Säure oder Triethylphosphit, schwefelhaltige Verbindungen, wie Diphenylsulfid, Phenothiazin oder Metallsalze, wie beispielsweise Kupfer-, Mangan-, Cer-, Nickel-, Chrom-, -chlorid, -dithiocarbamat, -sulfat, -salicylat oder -acetat oder Gemische davon. Bevorzugt sind die genannten Phenole und Chinone, besonders bevorzugt sind Hydrochinon, Hydrochinonmonomethylether, 2-tert.-Butyl-4-methylphenol, 6-tert.-Butyl-2,4-dimethyl-phenol, 2,6-Di-tert.-Butyl-4-methylphenol, 2,4-di-tert.-Butylphenol, Triphenylphosphit, Hypophosphorige Säure, $CuCl_2$ und Guajacol, ganz besonders bevorzugt sind Hydrochinon und Hydrochinonmonomethylether.

Besonders bevorzugt sind Hydrochinonmonomethylether, Hydrochinon, und Alkylphenole, gegebenenfalls in Kombination mit Triphenylphosphit und/oder Hypophosphoriger Säure.

Ganz besonders bevorzugt sind α-Tocopherol (Vitamin E), β-Tocopherol, γ-Tocopherol, oder δ-Tocopherol,

gegebenenfalls in Kombination mit Triphenylphosphit und/oder Hypophosphoriger Säure

Ganz besonders bevorzugt werden nur sterisch gehinderte Phenole als Stabilisatoren eingesetzt, welche während der Veresterungsreaktion -im Vergleich mit Hydrochinonmonomethylether-.bei gleicher Säurezahl des Endproduktes weniger stark dunkel gefärbte Produktgemische ergeben. Ein Beispiel für solche am meisten bevorzugten Stabilisatoren ist das α-Tocopherol.

Zur weiteren Unterstützung der Stabilisierung kann ein sauerstoffhaltiges Gas, bevorzugt Luft oder ein Gemisch aus Luft und Stickstoff (Magerluft) anwesend sein.

Unter den aufgeführten Stabilisatoren sind solche bevorzugt, die aerob sind, d.h. solche, die zur Entfaltung ihrer vollen Inhibitorwirkung die Anwesenheit von Sauerstoff erfordern.

Erfindungsgemäß einsetzbare Lösungsmittel E sind besonders solche, die sich zur azeotropen Entfernung des Reaktionswassers, falls gewünscht, eignen, vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Für die Veresterung können die dem Fachmann bekannten Herstell- und/oder Aufarbeitungsverfahren von mehrwertigen Alkoholen angewendet werden, beispielsweise die eingangs erwähnten oder die in DE-A 199 41 136, DE-A 38 43 843, DE-A 38 43 854, DE-A 199 37 911, DE-A 199 29 258, EP-A 331 845, EP 554 651 oder US 4 187 383 beschriebenen.

Im allgemeinen kann die Veresterung wie folgt durchgeführt werden:

Die Veresterungsapparatur besteht aus einem gerührten Reaktor, bevorzugt aus einem Reaktor mit Umlaufverdampfer und einer aufgesetzten Destillationseinheit mit Kondensator und Phasentrenngefäß.

Bei dem Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf, d.h. unter Verwendung einer Pumpe, besonders bevorzugt Naturumlauf, bei dem der Kreislaufstrom ohne mechanische Hilfsmittel bewerkstelligt wird, eingesetzt.

Selbstverständlich kann die Reaktion auch in mehreren Reaktionszonen, beispielsweise einer Reaktorkaskade aus zwei bis vier, bevorzugt zwei bis drei Reaktoren durchgeführt werden.

Geeignete Umlaufverdampfer sind dem Fachmann bekannt und beispielsweise beschrieben in R. Billet, Verdampfertechnik, HTB-Verlag, Bibliographisches Institut Mannheim, 1965, 53. Beispiele für Umlaufverdampfer sind Rohrbündelwärmetauscher, Plattenwärmetauscher, etc.

Selbstverständlich können im Umlauf auch mehrere Wärmetauscher vorhanden sein.

Die Destillationseinheit ist von an sich bekannter Bauart. Dabei kann es sich um eine einfache Destillation handeln, die gegebenenfalls mit einem Spritzschutz ausgestattet ist, oder um eine Rektifikationskolonne. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator und das Trenngefäß sind von herkömmlicher Bauart.

(Meth)acrylsäure und alkoxyliertes Glycol werden in der Veresterung a) in der Regel im molaren Überschuss wie oben angegeben eingesetzt. Der eingesetzte Überschuss kann bis zu ca. 3000:1 betragen, falls gewünscht.

Als Veresterungskatalysatoren C kommen die oben angeführten in Frage.

Sie werden in der Regel in einer Menge von 0,1 - 5 Gew.-%, bezogen auf das Veresterungsgemisch, eingesetzt, bevorzugt 0,5 - 5, besonders bevorzugt 1 - 4 und ganz besonders bevorzugt 2 - 4 Gew.-%.

Falls erforderlich kann der Veresterungskatalysator aus dem Reaktionsgemisch mit Hilfe eines Ionenaustauschers entfernt werden. Der Ionenaustauscher kann dabei direkt in das Reaktionsgemisch gegeben und anschließend abfiltriert oder das Reaktionsgemisch kann über eine Ionenaustauscherschüttung geleitet werden.

Bevorzugt wird der Veresterungskatalysator im Reaktionsgemisch belassen. Handelt es sich jedoch bei dem Katalysator um einen Ionentauscher, so wird dieser bevorzugt entfernt, beispielsweise durch Filtration.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert und/oder durch das Reaktionsgemisch und/oder über dieses geleitet.

Das Polymerisationsinhibitor(gemisch) D (wie oben angeführt) wird in einer Gesamtmenge von 0,01 -11 Gew.-%, bezogen auf das Veresterungsgemisch, eingesetzt, bevorzugt 0,02 - 0,8, besonders bevorzugt 0,05 - 0,5 Gew.-%.

Das Polymerisationsinhibitor(gemisch) D kann beispielsweise als wässrige Lösung oder als Lösung in einem Edukt oder Produkt eingesetzt werden.

b) Das bei der Reaktion entstehende Reaktionswasser kann während oder nach der Veresterung a) abdestilliert werden, wobei dieser Vorgang durch ein mit Wasser ein Azeotrop bildendes Lösungsmittel unterstützt werden kann.

Als Lösungsmittel E zur azeotropen Entfernung des Reaktionswassers, falls gewünscht, eignen sich die oben angeführten Verbindungen.

Bevorzugt ist die Durchführung der Veresterung in Gegenwart eines Lösungsmittels.

Die eingesetzte Menge an Lösungsmittel beträgt 10 - 200 Gew.-%, vorzugsweise 20 - 100 Gew.-%, besonders bevorzugt 30 bis 100 Gew% bezogen auf die Summe von alkoxyliertem Glycol und (Meth)acrylsäure.

Denkbar ist jedoch auch eine Durchführung ohne Schleppmittel, wie sie z.B. in der DE-A1 38 43 854, Sp. 2, Z. 18 bis Sp. 4, Z. 45 beschrieben ist, jedoch im Unterschied dazu mit den oben genannten Stabilisatoren.

Falls das im Reaktionsgemisch enthaltene Wasser nicht über ein azeotropbildendes Lösungsmittel entfernt wird, so ist es möglich, dieses über Strippen mit einem inerten Gas, bevorzugt einem sauerstoffhaltigen Gas, besonders bevorzugt mit Luft oder Magerluft zu entfernen, beispielsweise wie in der DE-A 38 43 843 beschrieben.

Die Reaktionstemperatur der Veresterung a) liegt allgemein bei 40 -160°C, bevorzugt 60 -140°C und besonders bevorzugt 80 -120°C. Die Temperatur kann im Reaktionsverlauf gleichbleiben oder ansteigen, bevorzugt wird sie im Reaktionsverlauf angehoben. In diesem Fall ist die Endtemperatur der Veresterung um 5 - 30°C höher als die Anfangstemperatur. Die Temperatur der Veresterung kann durch Variation der Lösungsmittelkonzentration im Reaktionsgemisch bestimmt und geregelt werden, wie in DE-A 199 41 136 und der deutschen Anmeldung mit dem Aktenzeichen 100 63 175.4 beschrieben.

Falls ein Lösungsmittel eingesetzt wird, so kann dieses über die dem Reaktor aufgesetzte Destillationseinheit vom Reaktionsgemisch abdestilliert werden.

Das Destillat kann wahlweise entfernt werden, oder, nach Kondensation, in einen Phasentrennapparat geführt werden. Die so erhaltene wässrige Phase wird in der Regel ausgeschleust, die organische Phase kann als Rücklauf in die Destillationseinheit geführt und/oder direkt in die Reaktionszone geleitet werden und/oder in einen Umlaufverdampfer geführt werden, wie in der deutschen Patentanmeldung mit dem Aktenzeichen 100 63175.4 beschrieben.

Bei Verwendung als Rücklauf kann die organische Phase, wie in der DE-A 199 41 136 beschrieben, zur Steuerung der Temperatur in der Veresterung verwendet werden.

Die Veresterung a) kann drucklos aber auch bei Überdruck oder Unterdruck durchgeführt werden, vorzugsweise wird bei normalem Druck gearbeitet.

Die Reaktionszeit beträgt in der Regel 2 - 20 Stunden, vorzugsweise 4 -15 und besonders bevorzugt 7 bis 12 Stunden.

Die Reihenfolge der Zugabe der einzelnen Reaktionskomponenten ist erfindungsgemäß nicht wesentlich. Es können alle Komponenten gemischt vorgelegt und anschließend aufgeheizt werden oder es können eine oder mehrere Komponenten nicht oder nur teilweise vorgelegt und erst nach dem Aufheizen zugegeben werden.

Die einsetzbare (Meth)acrylsäure ist in ihrer Zusammensetzung nicht beschränkt und kann beispielsweise folgende Komponenten aufweisen:

| | |
|---|---|
| (Meth)acrylsäure | 90 - 99,9 Gew.-% |
| Essigsäure | 0,05 - 3 Gew.-% |
| Propionsäure | 0,01 - 1 Gew.-% |
| Diacrylsäure | 0,01 - 5 Gew.-% |
| Wasser | 0,05 - 5 Gew.-% |
| Carbonylhaltige | 0,01 - 0,3 Gew.-% |
| Inhibitoren | 0,01 - 0,1 Gew.-% |
| Maleinsäure(-anhydrid) | 0,001 - 0,5 Gew.-% |

Die eingesetzte Roh-(Meth)acrylsäure ist in der Regel stabilisiert mit 200 - 600 ppm Phenothiazin oder anderen Stabilisatoren in Mengen, die eine vergleichbare Stabilisierung ermöglichen. Unter dem Ausdruck Carbonylhaltige werden hier beispielsweise Aceton und niedere Aldehyde, wie z.B. Formaldehyd, Acetaldehyd, Crotonaldehyd, Acrolein, 2- und 3- Furfural und Benzaldehyd, verstanden.

Unter Roh-(Meth)acrylsäure wird hier das (meth)acrylsäurehaltige Gemisch verstanden, das nach Absorption der Reaktionsgase der Propan/Propen/Acrofein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew.-% |

(fortgesetzt)

| | |
|---|---|
| Essigsäure | 50 - 1000 Gew.ppm |
| Propionsäure | 10 - 500 Gew.ppm |
| Diacrylsäure | 10 - 500 Gew.ppm |
| Wasser | 50 - 1000 Gew.ppm |
| Carbonylhaltige | 1 - 500 Gew.ppm |
| Inhibitoren | 1 - 300 Gew.ppm |
| Maleinsäure(-anhydrid) | 1 - 200 Gew.ppm |

Die eingesetzte Rein-(Meth)acrylsäure ist in der Regel stabilisiert mit 100 - 300 ppm Hydrochinonmonomethylether oder anderen Lagerstabilisatoren in Mengen, die eine vergleichbare Stabilisierung ermöglichen. Beispielsweise kann die Rein-(Meth)acrylsäure auch mit Vitamin E oder anderen sterisch gehinderten Phenolen stabilisiert sein.

Unter reiner bzw. vorgereinigter (Meth)acrylsäure wird allgemein (Meth)acrylsäure verstanden, deren Reinheit mind. 99,5 Gew% beträgt und die im wesentlichen frei von den aldehydischen, anderen carbonylhaltigen und hochsiedenden Komponenten ist.

Die während der Veresterung abdestillierte wässrige Phase des über die aufgesetzte Kolonne, wenn vorhanden, abgetrennten Kondensats, die in der Regel 0,1 - 10 Gew.-% (Meth)acrylsäure enthalten kann, wird abgetrennt und ausgeschleust. Vorteilhaft kann die darin enthaltene (Meth)acrylsäure mit einem Extraktionsmittel, bevorzugt dem gegebenenfalls in der Veresterung verwendeten Lösungsmittel, beispielsweise mit Cyclohexan, bei einer Temperatur zwischen 10 und 40 °C und einem Verhältnis von wässriger Phase zu Extraktionsmittel von 1 : 5 - 30, bevorzugt 1 : 10 - 20, extrahiert und in die Veresterung zurückgeführt werden.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) in den Umlauf, durch oder über das Reaktionsgemisch geleitet werden, beispielsweise in Mengen von 0,1 -1, bevorzugt 0,2 - 0,8 und besonders bevorzugt 0,3 - 0,7 $m^3/m^3h$, bezogen auf das Volumen des Reaktionsgemisches.

Der Verlauf der Veresterung a) kann durch Verfolgung der ausgetragenen Wassermenge und/oder die Abnahme der Carbonsäurekonzentration im Reaktor verfolgt werden.

Die Reaktion kann beispielsweise beendet werden, sobald 90 % der theoretisch zu erwartenden Wassermenge durch das Lösungsmittel ausgetragen worden sind, bevorzugt bei mindestens 95% und besonders bevorzugt bei mindestens 98%.

Das Ende der Reaktion kann beispielsweise dadurch festgestellt werden, dass im wesentlichen kein weiteres Reaktionswasser mehr über das Schleppmittel entfernt wird. Wird (Methacryl)säure zusammen mit dem Reaktionswasser ausgetragen, so ist deren Anteil beispielsweise durch Rücktitration eines Aliquots der wässrigen Phase bestimmbar.

Auf ein Entfernen des Reaktionswassers kann beispielsweise verzichtet werden, wenn die (Methacryl)säure in einem hohen stöchiometrischen Überschuss eingesetzt wird, beispielsweise von mindestens 3:1, bevorzugt mindestens 5:1 und ganz besonders bevorzugt mindestens 10:1. In diesem Fall verbleibt ein wesentlicher Teil der entstandenen Wassermenge im Reaktionsgemisch. Während oder nach der Reaktion wird lediglich der Anteil an Wasser aus dem Reaktionsgemisch entfernt, der durch die Flüchtigkeit bei der angelegten Temperatur bestimmt ist und es werden darüber hinaus keine Maßnahmen zur Abtrennung des entstandenen Reaktionswassers durchgeführt. So können beispielsweise mindestens 10 Gew.-% des entstandenen Reaktionswassers im Reaktionsgemisch verbleiben, bevorzugt mindestens 20 Gew%, besonders bevorzugt mindestens 30 Gew.-%, ganz besonders bevorzugt mindestens 40 und insbesondere mindestens 50 Gew.-%.

c) Nach Beendigung der Veresterung kann das Reaktorgemisch auf übliche Weise auf eine Temperatur von 10 bis 30°C abgekühlt werden und gegebenenfalls durch Zugabe von Lösungsmittel, das das gleiche wie das gegebenenfalls zur azeotropen Entfernung von Wasser verwendete Lösungsmittel oder ein anderes sein kann, eine beliebige Zielesterkonzentration eingestellt werden.

In einer weiteren Ausführungsform kann die Reaktion mit einem geeigneten Verdünnungsmittel G gestoppt werden und auf eine Konzentration von beispielsweise 10 - 90 Gew.-%, bevorzugt 20 - 80%, besonders bevorzugt 20 bis 60%, ganz besonders bevorzugt 30 bis 50% und insbesondere ca. 40% verdünnt, beispielsweise um die Viskosität zu verringern.

Wichtig ist dabei, dass sich nach Verdünnung eine im wesentlichen homogene Lösung bildet.

Dies erfolgt bevorzugt erst relativ kurz vor dem Einsatz in der Herstellung des Hydrogels, beispielsweise nicht mehr als 24 Stunden vorher, bevorzugt nicht mehr als 20, besonders bevorzugt nicht mehr als 12, ganz besonders bevorzugt nicht mehr als 6 und insbesondere nicht mehr als 3 Stunden vorher.

Das Verdünnungsmittel G ist ausgewählt aus der Gruppe bestehend aus Wasser, ein Gemisch von Wasser mit einem oder mehreren in Wasser unbegrenzt löslichen organischen Lösemittel oder ein Gemisch von Wasser mit

einem oder mehreren einfachen oder mehrfachfunktionellen Alkoholen, z.B. Methanol und Glycerin. Die Alkohole tragen bevorzugt 1, 2 oder 3 Hydroxygruppen und weisen bevorzugt zwischen 1 bis 10, insbesondere bis 4 C-Atome auf. Bevorzugt sind primäre und sekundäre Alkohole.

Bevorzugte Alkohole sind Methanol, Ethanol, Isopropanol, Ethylenglykol, Glycerin, 1,2-Propandiol oder 1,3-Propandiol.

d) Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew.-%, bevorzugt 0,5 bis 25 Gew.-%, besonders bevorzugt 1 - 10 Gew.-% bei Temperaturen von beispielsweise 10 bis 100°C, bevorzugt 20 bis 80°C und besonders bevorzugt 30 bis 60°C unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Vorwäsche, Neutralisation, Wäsche oder Lösungsmittelentfernung.

Das Reaktionsgemisch kann weiterhin einer Vorwäsche e) und/oder einer Neutralisation f) und/oder einer Nachwäsche g) unterworfen werden, bevorzugt lediglich einer Neutralisation f). Gegebenenfalls können Neutralisation f) und Vorwäsche e) in der Reihenfolge auch vertauscht werden.

Aus der wässrigen Phase der Wäschen e) und g) und/oder Neutralisation f) kann enthaltene (Meth)acrylsäure und/oder Katalysator C durch Ansäuern und Extraktion mit einem Lösungsmittel zumindest teilweise wiedergewonnen und von Neuem eingesetzt werden.

Zur Vor- oder Nachwäsche e) oder g) wird das Reaktionsgemisch in einem Waschapparat mit einer Waschflüssigkeit, beispielsweise Wasser oder einer 5 - 30 gew.-%igen, bevorzugt 5 - 20, besonders bevorzugt 5 -15 gew.-%igen Kochsalz-, Kaliumchlorid-, Ammoniumchlorid-, Natriumsulfat- oder Ammoniumsulfatlösung, bevorzugt Wasser oder Kochsalzlösung, behandelt.

Das Mengenverhältnis Reaktionsgemisch : Waschflüssigkeit beträgt in der Regel 1: 0,1 -1, bevorzugt 1 : 0,2 - 0,8, besonders bevorzugt 1 : 0,3 - 0,7.

Die Wäsche oder Neutralisation kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche oder Neutralisation im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Vorzugsweise werden Siebboden- oder gepackte beziehungsweise Füllkörperkolonnen, Rührbehälter oder Mixer-Settler-Apparate, sowie gepulste Kolonnen oder solche mit rotierenden Einbauten eingesetzt.

Die Vorwäsche e) wird bevorzugt dann eingesetzt, wenn Metallsalze, bevorzugt Kupfer oder Kupfersalze als Inhibitoren (mit)verwendet werden.

Eine Nachwäsche g) kann zur Entfernung von Base- oder Salzspuren aus dem in f) neutralisierten Reaktionsgemisch vorteilhaft sein.

Zur Neutralisation f) kann das gegebenenfalls vorgewaschene Reaktionsgemisch, das noch geringe Mengen an Katalysator und die Hauptmenge an überschüssiger (Meth)acrylsäure enthalten kann, mit einer 5 - 25, bevorzugt 5 - 20, besonders bevorzugt 5 - 15 gew.-%igen wässrigen Lösung einer Base, wie beispielsweise Alkali- oder Erdalkalimetalloxide, -hydroxide, -carbonate oder -hydrogencarbonate, bevorzugt Natronlauge, Kalilauge, Natriumhydrogencarbonat, Natriumcarbonat, Kaliumhydrogencarbonat, Kalziumhydroxid, Kalkmilch, Ammoniak, Ammoniakwasser oder Kaliumcarbonat, der gegebenenfalls 5 -15 Gew.-% Kochsalz, Kaliumchlorid, Ammoniumchlorid oder Ammoniumsulfat zugesetzt sein können, besonders bevorzugt mit Natronlauge oder Natronlauge-Kochsalz-Lösung, neutralisiert werden. Der Neutralisationsgrad beträgt bevorzugt 5 bis 60 mol-%, vorzugsweise 10 bis 40 mol-%, besonders bevorzugt 20 bis 30 mol-%, bezogen auf die Säuregruppen enthaltenden Monomere. Diese Neutralisation kann vor und/oder während der Polymerisation erfolgen, bevorzugt vor der Polymerisation. Ein anderer bevorzugter Neutralisationsgrad liegt bei 50 bis 100 mol%, besonders bevorzugt bei 55 - 80 mol-%, insbesondere bei 60 bis 75 mol-%. Bevorzugt wird die Vernetzerlösung in reiner Acrylsäure erst kurz vor der Polymerisation mit der Acrylatlösung zur Einstellung des Neutralisationsgrades zusammengemischt.

Die Zugabe der Base erfolgt in einer Weise, dass die Temperatur im Apparat nicht über 60°C ansteigt, bevorzugt zwischen 20 und 35°C beträgt und der pH-Wert 4 -13, bevorzugt 4,5 -10 beträgt. Die Abfuhr der Neutralisationswärme erfolgt vorzugsweise durch Kühlung des Behälters mit Hilfe von innenliegenden Kühlschlangen oder über eine Doppelwandkühlung.

Das Mengenverhältnis Reaktionsgemisch : Neutralisationsflüssigkeit beträgt in der Regel 1: 0,1 -1, bevorzugt 1 : 0,2 - 0,8, besonders bevorzugt 1 : 0,3 - 0,7.

Hinsichtlich der Apparatur gilt das oben Gesagte.

h) Falls ein Lösungsmittel im Reaktionsgemisch enthalten ist, so kann dieses durch Destillation im wesentlichen entfernt werden. Bevorzugt wird gegebenenfalls enthaltenes Lösungsmittel nach Wäsche und/oder Neutralisation aus dem Reaktionsgemisch entfernt, falls gewünscht kann dieses aber auch vor der Wäsche beziehungsweise Neutralisation erfolgen.

Dazu wird das Reaktionsgemisch mit einer derartigen Menge an Lagerstabilisator, bevorzugt Hydrochinonmonomethylether versetzt, dass nach Abtrennung des Lösungsmittels 100 - 500, bevorzugt 200 - 500 und besonders bevorzugt 200 - 400 ppm davon im Zielester (Rückstand) enthalten sind. Anstatt Hydrochinonmonomethylether können aber auch die oben beschriebenen sterisch gehinderten Phenole bevorzugt allein oder im Gemisch mit anderen Stabilisatoren eingesetzt werden.

Die destillative Abtrennung der Hauptmenge an Lösungsmittel erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50 -150 mbar und einer Temperatur von 40 - 80°C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500 und besonders bevorzugt 50 -150 mbar und einer Temperatur von 40 - 80°C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 -1, bevorzugt 0,2 - 0,8 und besonders bevorzugt 0,3-0,7 $m^3/m^3h$, bezogen auf das Volumen des Reaktionsgemisches.

Der Restlösungsmittelgehalt im Rückstand beträgt nach der Destillation in der Regel unter 5 Gew.-%, bevorzugt 0,5 - 5 % und besonders bevorzugt 1 bis 3 Gew.-%.

Das abgetrennte Lösungsmittel wird kondensiert und bevorzugt wiederverwendet.

Falls erforderlich kann zusätzlich oder anstelle der Destillation eine Lösungsmittelstrippung i) durchgeführt werden.

Dazu wird der Zielester, der noch geringe Lösungsmittelmengen enthält, auf 50 - 90°C, bevorzugt 80 - 90°C erwärmt und die restlichen Lösungsmittelmengen mit einem geeigneten Gas in einer geeigneten Apparatur entfernt. Zur Unterstützung kann gegebenenfalls auch ein Vakuum angelegt werden.

Geeignete Apparaturen sind beispielsweise Kolonnen von an sich bekannter Bauart, die die üblichen Einbauten, z.B. Böden, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie z.B. ein Luwa-, Rotafilm- oder Sambayverdampfer, der als Spritzschutz beispielsweise mit einem Demister ausgerüstet sein kann.

Geeignete Gase sind unter den Strippbedingungen inerte Gase, bevorzugt sauerstoffhaltige Gase, besonders bevorzugt Luft oder Gemische aus Luft und Stickstoff (Magerluft) oder Wasserdampf, insbesondere solche, die auf 50 bis 100°C temperiert sind.

Die Strippgasmenge beträgt beispielsweise 5 - 20, besonders bevorzugt 10 - 20 und ganz besonders bevorzugt 10 bis 15 $m^3/m^3h$, bezogen auf das Volumen des Reaktionsgemisches.

Falls notwendig kann der Ester in einem beliebigen Stadium des Aufarbeitungsverfahrens, bevorzugt nach Wäsche/Neutralisation und gegebenenfalls erfolgter Lösungsmittelentfernung einer Filtration j) unterworfen werden, um ausgefallene Spuren an Salzen sowie gegebenenfalls enthaltenem Entfärbungsmittel zu entfernen.

In einer denkbaren Ausführungsform wird die Veresterung a) von alkoxyliertem Glycol mit der (Methacryl)säure in einem wie oben angeführten molaren Überschuss von mindestens 10:1 in Gegenwart mindestens eines Veresterungskatalysators C und mindestens eines Polymerisationsinhibitors D ohne ein mit Wasser ein Azeotrop bildendes Lösungsmittel durchgeführt.

Die im Überschuss eingesetzte (Methacryl)säure wird in einer bevorzugten Ausführungsform im wesentlichen nicht abgetrennt, d.h. es wird lediglich der Anteil an (Methacryl)säure aus dem Reaktionsgemisch entfernt, der durch die Flüchtigkeit bei der angelegten Temperatur bestimmt ist und es werden darüberhinaus keine Maßnahmen zur Abtrennung der Carbonsäure durchgeführt, wie beispielsweise destillative, rektifikative, extraktive, wie z.B. Wäschen, absorptive, wie z.B. Überleiten über Aktivkohle oder über Ionentauscher, und/oder chemische Schritte, wie z.B. Abfangen der Carbonsäure mit Epoxiden.

Bevorzugt wird die im Reaktionsgemisch enthaltene (Methacryl)säure zu nicht mehr als 75 Gew.-%, besonders bevorzugt zu nicht mehr als 50 Gew.-%, ganz besonders bevorzugt zu nicht mehr als 25 Gew.-%, insbesondere zu nicht

mehr als 10 Gew.-% und speziell zu nicht mehr als 5 Gew.-% aus dem Reaktionsgemisch abgetrennt, bezogen auf die nach Reaktionsende im Reaktionsgemisch enthaltene (Methacryl)säure. In einer besonders bevorzugten Ausführungsform kann auf die Stufe b) verzichtet werden, so dass lediglich der Anteil an Reaktionswasser und (Methacryl)säure aus dem Reaktionsgemisch entfernt wird, der durch die Flüchtigkeit bei der angelegten Temperatur bestimmt ist. Dies kann bevorzugt durch im wesentlichen vollständige Kondensation verhindert werden.

Weiterhin verbleibt auch der eingesetzte Veresterungskatalysator C im wesentlichen im Reaktionsgemisch.

Das so erhältliche Reaktionsgemisch weist bevorzugt eine Säurezahl gem. DIN EN 3682 von mindestens 25 mg KOH/g Reaktionsgemisch auf, bevorzugt mind. 35 mg KOH/g, besonders bevorzugt mind. 45 mg KOH/g aufweist. Als Bereich ist besonders bevorzugt von 25 bis 80 und ganz besonders bevorzugt von 35 bis 50 mg KOH/g auf.

Auf eine Vor- oder Nachwäsche e) oder g) wird bevorzugt verzichtet, lediglich ein Filtrationsschritt j) kann sinnvoll sein.

Anschließend kann das Reaktionsgemisch im Schritt c) verdünnt werden, in diesem Fall wird es bevorzugt innerhalb von 6 Stunden, besonders bevorzugt innerhalb von 3 Stunden zum Hydrogel umgesetzt. Bevorzugt kann es in einem Schritt f) neutralisiert werden.

Die Abfolge der Schritte c), j) und f) ist dabei beliebig.

Die Erfindung betrifft außerdem ein Stoffgemisch enthaltend

- mindestens einen Ester F, erhältlich nach einem der oben beschriebenen Veresterungsverfahren,
- (Methacryl)säure und
- Verdünnungsmittel G.

Als weitere Komponenten können enthalten sein

- Veresterungskatalysator C in protonierter oder unprotonierter Form,
- Polymerisationsinhibitor D sowie
- gegebenenfalls Lösungsmittel E, falls ein solches in der Versterung verwendet wurde.

Das Stoffgemisch kann gegebenenfalls neutralisiert sein und einen pH-Wert aufweisen, wie oben unter f) aufgeführt.

Wenn das Stoffgemisch neutralisiert ist, so ist zumindest ein Teil der (Methacryl)säure in deren wasserlösliche Alkalimetall-, Erdalkalimetall- oder Ammoniumsalze überführt.

Bevorzugtes Stoffgemisch enthält an

- Ester F im Stoffgemisch 0,1 bis 40 Gew.%, besonders bevorzugt 0,5 bis 20, ganz besonders bevorzugt 1 bis 10, insbesondere 2 bis 5 und speziell 2 bis 4 Gew.%,
- Monomer M 0,5 - 99,9 Gew%, besonders bevorzugt 0,5 - 50 Gew.-%, ganz besonders bevorzugt 1 - 25, insbesondere 2 - 15 und speziell 3 bis 8, oder 4 - 6 Gew.-%,
- Veresterungskatalysator C 0 -10 Gew.-%, besonders bevorzugt 0,02 - 5, ganz besonders bevorzugt 0,05 - 2,5 Gew.-% und insbesondere 0,1 -1 Gew.-%,
- Polymerisationsinhibitor D 0 - 5 Gew.-%, besonders bevorzugt 0,01 -1,0, ganz besonders bevorzugt 0,02 - 0,75, insbesondere 0,05 - 0,5 und speziell 0,075 - 0,25 Gew.-%,
- Lösungsmittel E0 -10 Gew.-%, besonders bevorzugt 0 - 5 Gew.-%, ganz besonders bevorzugt 0,05-1,5 Gew.-% und insbesondere 0,1 -0,5 Gew.-%, mit der Maßgabe, dass die Summe immer 100 Gew.-% beträgt, sowie
- gegebenenfalls Verdünnungsmittel G ad 100 Gew.-%.

Die nach dem obigen Verfahren erhältlichen Reaktionsgemische und erfindungsgemäßen Stoffgemische können Verwendung finden

- als Radikalvernetzer von wasserabsorbierenden Hydrogelen,
- als Ausgangsstoff für die Herstellung von Polymerdispersionen,
- als Ausgangsstoff für die Herstellung von Polyacrylaten (außer Hydrogelen),
- als Lackrohstoff oder
- als Zementadditiv.

Zur Verwendung als Radikalvernetzer von wasserabsorbierenden Hydrogelen sind insbesondere solche erfindungsgemäßen Stoffgemische geeignet, die eine Wasserlöslichkeit (bei 25°C in destilliertem Wasser) von mindestens 0,5 Gew.-%, bevorzugt mindestens 1 Gew.-%, mehr bevorzugt mindestens 2 Gew.%, noch mehr bevorzugt mindestens 5 Gew.-, besonders bevorzugt mindestens 10 Gew.-%, ganz besonders bevorzugt mindestens 20 Gew.-% und insbesondere von mindestens 30 Gew.-% aufweisen.

k) Das Reaktionsgemisch aus der Veresterung, inklusive deren Aufarbeitungsschritte, soweit diese durchlaufen werden, beispielsweise das Reaktionsgemisch aus f), bzw, wenn auf f) verzichtet wird, aus b), beziehungsweise, wenn auf b) verzichtet wird, das Reaktionsgemisch aus a), kann gegebenenfalls mit zusätzlichen monoethylenisch ungesättigten Verbindungen N versetzt werden, die keine Säuregruppen tragen, aber mit den hydrophilen Monomeren M copolymerisierbar sind, kann dann zur Herstellung von wasserabsorbierenden Hydrogelen in Gegenwart mindestens eines Radikalstarters K und gegebenenfalls mindestens einer Pfropfgrundlage L polymerisiert werden.

Vorteilhaft kann

l) das Reaktionsgemisch aus k) nachvernetzt werden.

Zur Herstellung k) dieser hydrophilen, hochquellfähigen Hydrogele geeignete hydrophile Monomere M sind beispielsweise polymerisationsfähige Säuren, wie Acrylsäure, Methacrylsäure, Ethacrylsäure, α-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2methylpropansulfonsäure, 2-Acrylamido-2-methylpropanphosphonsäure sowie deren Amide, Hydroxyalkylester und aminogruppen- oder ammoniumgruppenhaltige Ester und Amide. Die Monomeren können allein oder in Mischung untereinander eingesetzt werden. Des weiteren wasserlösliche N-Vinylamide oder auch Diallyldimethylammoniumchlorid. Bevorzugte hydrophile Monomere sind Verbindungen der Formel V

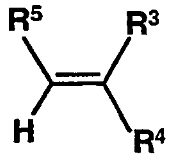

worin

- $R^3$ Wasserstoff, Methyl oder Ethyl,
- $R^4$ die Gruppe -COOR$^6$, eine Sulfonylgruppe oder Phosphonylgruppe, eine mit ei- nem ($C_1$-$C_4$)-Alkylalkohol veresterte Phosphonylgruppe oder eine Gruppe der Formel VI

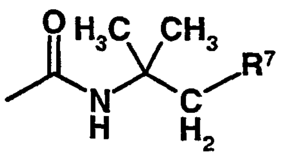

- $R^5$ Wasserstoff, Methyl, Ethyl oder eine Carboxylgruppe,
- $R^6$ Wasserstoff, Amino oder Hydroxy-($C_1$-$C_4$)-Alkyl und
- $R^7$ eine Sulfonylgruppe, eine Phosphonylgruppe oder eine Carboxylgruppe bedeu- ten.

Beispiele für ($C_1$-$C_4$)- Alkylalkohol sind Methanol, Ethanol, n-Propanol oder n-Butanol.

Besonders bevorzugte hydrophile Monomere sind Acrylsäure und Methacrylsäure, insbesondere Acrylsäure.

Zur Optimierung von Eigenschaften kann es sinnvoll sein, zusätzliche monoethylenisch ungesättigte Verbindungen N einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäuren, z. B. Acrylamid, Methacrylamid und N-Vinylformamid, N-Vinylacetamid, N-Methylvinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z. B. Ethylvinylether oder Butylvinylether, Ester von monoethylenisch ungesättigten $C_3$- bis $C_6$-Carbonsäuren, z. B. Ester aus

einwertigen $C_1$- bis $C_{18}$-Alkoholen und Acrylsäure, Methacrylsäure oder Maleinsäure, Halbester von Maleinsäure, z. B. Maleinsäuremono-methylester, N-Vinyllactame wie N-Vinylpyrrolidon oder N-Vinylcaprolactam, Acrylsäure- und Methacrylsäureester von alkoxylierten einwertigen, gesättigten Alkoholen, z. B. von Alkoholen mit 10 bis 25 C-Atome, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, z. B. Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-% zugesetzt.

Bevorzugt bestehen die vernetzten (Co)Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 - 40 Gew.-% bezogen auf ihr Gesamtgewicht keine säuregruppentragenden monoethylenisch ungesättigten Monomeren.

Die Herstellung, Testung und Verwendung (meth)acrylsäurehaltiger (Co)Polymere, Polyacrylsäuren und Superabsorbern ist vielfach vorbeschrieben und daher hinreichend bekannt, siehe z.B. "Modern Superabsorbent Polymer Technology", F.L. Buchholz and A.T. Graham, Wiley-VCH, 1998 oder in Markus Frank "Superabsorbents" in Ullmann's Handbuch der technischen Chemie Band 35 2003.

Bevorzugt sind solche Hydrogele, die durch vernetzende Polymerisation oder Copolymerisation von säuregruppentragenden monoethylenisch ungesättigten Monomeren M oder deren Salzen erhalten werden.

Die erhältlichen Polymere zeichnen sich durch einen verbesserten Verseifungsindex (VSI) aus.

Bei dem Verfahren zur Nachvernetzung wird das Ausgangspolymer mit einem Nachvernetzer behandelt und bevorzugt während oder nach dem Behandeln durch Temperaturerhöhung nachvernetzt und getrocknet, wobei der Vernetzer bevorzugt in einem inerten Lösemittel enthalten ist. Unter inerten Lösemittel werden solche verstanden, die bei der Reaktion weder mit dem Ausgangspolymer noch mit dem Nachvernetzer im wesentlichen reagieren. Bevorzugt sind solche Lösemittel, die zu mehr als 90%, bevorzugt mehr als 95%, besonders bevorzugt mehr als 99%, insbesondere zu mehr als 99,5% nicht chemisch mit dem Ausgangspolymer oder Nachvernetzer reagieren.

Bevorzugt zur Nachvernetzung l) und Trocknung m) ist dabei der Temperaturbereich zwischen 30 und 250°C, insbesondere 50 - 200°C, ganz besonders bevorzugt ist der Bereich zwischen 100 -180°C. Die Aufbringung der Oberflächennachvernetzungslösung erfolgt bevorzugt durch Aufsprühen auf das Polymere in geeigneten Sprühmischern. Im Anschluss an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX-Mischer, NAUTA-Mischer, SHUGGI-Mischer oder PROCESSALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch z.B. eine azeotrope Destillation als Trocknungsverfahren benutzt werden. Die bevorzugte Verweilzeit bei dieser Temperatur im Reaktionsmischer oder Trockner beträgt unter 60 min., besonders bevorzugt unter 30 min.

Bevorzugt sind obige Verfahren, wobei das Ausgangspolymere eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Bevorzugt werden solche Verfahren in denen das Stoffgemisch enthaltend Radikalvernetzer, also den Ester F, und Verdünnungsmittel G in einem Verhältnis von 0,1 - 20 Gew.-%, insbesondere 0,5 -10 Gew.-% bezogen auf die Masse des Ausgangspolymeren eingesetzt wird.

Bevorzugt werden solche Verfahren in denen der Radikalvernetzer in einer Dosierung von 0,01 - 5,0 Gew.-%, bevorzugt 0,02 - 3,0 Gew.-%, ganz besonders bevorzugt 0,03 - 2,5 Gew.-%, insbesondere 0,05 -1,0 und speziell 0,1 bis 0,75 Gew.-% bezogen auf das Ausgangspolymer verwendet wird.

Gegenstand der Erfindung sind auch Polymere, hergestellt nach einem der oben genannten Verfahren und deren Verwendung in Hygieneartikeln, Verpackungsmaterialien und in Nonwovens, sowie die Verwendung von einem oben genannten Stoffgemisch zur Herstellung von vernetzten oder durch Wärmebehandlung vernetzungsfähigen Polymeren, insbesondere in Lacken und Farben.

Die dabei einzusetzenden hydrophilen, hochquellfähigen Hydrogele (Ausgangspolymere) sind insbesondere Polymere aus (co)polymerisierten hydrophilen Monomeren M, Pfropf(co)polymere von einem oder mehreren hydrophilen Monomeren M auf eine geeignete Pfropfgrundlage L, vernetzte Cellulose- oder Stärkeether oder in wässrigen Flüssigkeiten quellbare Naturprodukte, wie beispielsweise Guarderivate. Diese Hydrogele sind dem Fachmann bekannt und beispielsweise beschrieben in US-4 286 082, DE-C-27 06 135, US-4 340 706, DE-C-3713 601, DE-C-28 40 010, DE-A-43 44 548, DE-A40 20 780, DE-A-4015 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-4418 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780 EP-A-0 20 5674, US-5145 906, EP-A-0 530 438, EP-A-0 670 073, US4 057 521, US-4 062 817, US-4 525 527, US-4 295 987, US-5 011 892, US-4 076 663 oder US-4 931 497. Besonders geeignet sind auch hochquellfähige Hydrogele aus einem Herstellprozess wie in WO 01/38402 beschrieben, sowie anorganisch-organische hybride hochquellfähige Hydrogele wie in DE 198 54 575 beschrieben.

Geeignete Pfropfgrundlagen L für hydrophile Hydrogele, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das wasserabsorbierende Polymer kann über radikalische Pfropfcopolymerisation von Acrylsäure oder Acrylat auf eine wasserlösliche Polymermatrix erhalten werden. Geeignete wasserlösliche Polymermatrices sind beispielsweise, aber nicht ausschließlich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikalvernetzer eingesetzt.

Das wasserabsorbierende Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozeß ein in Wasser lösliches Silikat oder lösliches Aluminat oder ein Gemisch von beiden eingesetzt wurde.

Bevorzugte Hydrogele sind insbesondere Polyacrylate, Polymethacrylate sowie die in US-4 931 497, US-5 011 892 und US-5 041 496 beschriebene Pfropfpolymere. Ganz besonders bevorzugte Hydrogele sind die in WO 01/38402 beschriebenen Kneterpolymere und die in DE 198 545 75 beschriebenen hybriden organisch-anorganischen Hydrogele auf Basis von Polyacrylaten.

Die erfindungsgemäß hergestellten, als Radikalvernetzer in Hydrogelen verwendbaren Substanzen können allein oder in Kombination mit anderen Vernetzern, beispielsweise Innen- oder Oberflächenvernetzern verwendet werden, beispielsweise den folgenden:

Geeignete weitere Vernetzer sind insbesondere Methylenbisacryl- bzw. -methacrylamid, Ester ungesättigter Mono- oder Polycarbonsäuren von Polyolen, wie Diacrylat , Triacrylat oder Tetraacrylat, z. B. Butandiol- oder Ethylenglykoldiacrylat bzw. -methacrylat sowie Trimethylolpropantriacrylat oder Glycerindiacrylat und -triacrylat oder Pentaerythritoltetraacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Besonders bevorzugt werden im erfindungsgemäßen Verfahren jedoch Hydrogele, die unter Verwendung von Polyallylethern als weitere Vernetzer und durch saure Homopolymerisation von Acrylsäure hergestellt werden. Geeignete Vernetzer sind Pentaerythritoltri- und -tetraallylether, Trimethylolpropandiallylether, Polyethylenglykoldiallylether, Monoethylenglykoldiallylether, Glyceroldi- und Triallylether, Polyallylether auf Basis Sorbitol, sowie ethoxylierte Varianten davon. Weiterhin besonders bevorzugte Vernetzer sind die Polyethylenglykoldiacrylate, ethoxylierte Derivate von Trimethylolpropantriacrylat z.B. Sartomer SR 9035, sowie ethoxylierte Derivate von Glycerindiacrylat und Glycerintriacrylat. Selbstverständlich können auch Gemische der obigen Vernetzer eingesetzt werden.

Insbesondere bevorzugt sind Kombinationen von Vernetzern, bei denen in den erfindungsgemäßen Vernetzern F weitere Vernetzer dispergiert werden können. Beispiele für solche Vernetzerkombinationen sind die erfindungsgemäßen Vernetzer F zusammen mit Di- oder Tripropylenglycoldiacrylat und propoxylierten Glycerintriacrylaten. Weitere Beispiele solcher Vernetzerkombinationen sind die erfindungsgemäßen Vernetzer zusammen mit Butandioldiacrylat oder Trimethylolpropantriacrylat oder Pentaerythritoltriallylether.

Ganz besonders bevorzugt sind solche Hydrogele, die mit einem erfindungsgemäß hergestellten Ester F als Radikalvernetzer hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001 -10 mol-%), ganz besonders bevorzugt sind jedoch Polymere, die über radikalische Polymerisation erhalten wurden und bei denen ein mehrfunktioneller ethylenisch ungesättigter Radikalvernetzer verwendet wurde. Die erfindungsgemäßen Estergemisches erlauben bevorzugt jede Esterkomponente $F_i$ bezogen auf die Gesamtmenge der Monomere zu weniger als 2 Gew.-%, bevorzugt 1 Gew.-%, einzusetzen, Besonders bevorzugt ist die Summe aller Esterkomponenten unter 2 Gew.-, bevorzugt unter 1 Gew.-%.

Die hydrophilen, hochquellfähigen Hydrogele können durch an sich bekannte Polymerisationsverfahren hergestellt werden. Bevorzugt ist die Polymerisation in wässriger Lösung nach dem Verfahren der sogenannten Gelpolymerisation. Dabei werden wie oben angeführt verdünnte, bevorzugt wässrige, besonders bevorzugt 15 bis 50 gew.-% ige wässrige Lösungen eines oder mehrerer hydrophiler Monomerer und gegebenenfalls einer geeigneten Pfropfgrundlage L in Gegenwart eines Radikalinitiators bevorzugt ohne mechanische Durchmischung unter Ausnutzung des Trommsdorff-Norrish-Effektes (Makromol. Chem. 1,169 (1947)), polymerisiert. Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren K herangezogen werden, z. B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$.

Sie können gegebenenfalls in Kombination mit Reduktionsmitteln wie z.B. Ascorbinsäure, Natriumhydrogensulfit, und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie z. B. Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich 50° bis 130°C, vorzugsweise 70° bis 100°C, können die Qualitätseigenschaften der Polymerisate noch verbessert werden.

Die erhaltenen Gele werden zu 0 - 100 mol%, bevorzugt zu 25 - 100 mol%, und besonders bevorzugt zu 50 - 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat. Bei Verwendung von Natriumhydroxid wird besonders bevorzugt die durch Membranelektrolyse erhältliche Qualität eingesetzt.

Üblicherweise wird die Neutralisation durch Einmischung des Neutralisationsmittels als wässrige Lösung oder bevorzugt auch als Feststoff erreicht. Das Gel wird hierzu mechanisch zerkleinert, z.B. mittels eines Fleischwolfes, und das Neutralisationsmittel wird aufgesprüht, übergestreut oder aufgegossen, und dann sorgfältig untergemischt.

Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden, wie oben im Schritt f) beschrieben. Unabhängig davon ob die Neutralisation vor, während, oder nach der Polymerisation stattfindet wird besonders bevorzugt Acrylsäure mit einem Dimerengehalt unter 2000 ppm, ganz besonders bevorzugt unter 1000 ppm, und am meisten bevorzugt unter 500 ppm eingesetzt. Ganz besonders bevorzugt wird Natriumhydroxid aus einer Membranelektrolyse eingesetzt. Dieses zeichnet sich gegenüber anderen Verfahren durch seine hohe Reinheit (beispielsweise niedriger Chloridgehalt und Abwesenheit von Quecksilberspuren) aus. Natürlich kann aber auch Natriumhydroxid aus dem Amalgam- oder Diaphragmaverfahren verwendet werden. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels UV-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

n) Das getrocknete Hydrogel kann hiernach gemahlen und gesiebt werden, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 - 1000 μm, bevorzugt bei 45 - 850 μm, besonders bevorzugt bei 200 - 850 μm, und ganz besonders bevorzugt bei 300 - 850 μm. Ein weiterer besonders bevorzugter Bereich liegt bei 150 - 850 μm, insbesondere 150 - 700 μm, und besonders bevorzugt bei 200 - 600 μm, und ganz besonders bevorzugt bei 150 - 550 μm. Ein weiterer besonderer Bereich liegt bei 200 - 800 μm und ein besonders bevorzugter Bereich bei 250 - 650 μm, ein ganz besonders bevorzugter Bereich liegt bei 300 - 600 μm. Besonders bevorzugte Bereiche liegen bei 200 - 500 μm, bei 100 - 450 μm sowie bei 150 - 400 μm. In diesen Bereichen liegen bevorzugt 80 Gew.-% der Teilchen, insbesondere 90 Gew.-% der Teilchen. Die Größenverteilung kann mit etablierten Siebmethoden oder vorzugsweise auch mit optischen Methoden (Photographien) bestimmt werden.

Gegenstand der vorliegenden Erfindung sind weiterhin vernetzte Hydrogele, die mindestens ein hydrophiles Monomer M in einpolymerisierter Form enthalten und vernetzt sind mit einem Ester F von Polyol mit (Methacryl)säure. Der Ester kann erfindungsgemäß oder auf eine im Stand der Technik bekannte Weise hergestellt werden, bevorzugt auf erfindungsgemäße Weise.

Als Ester F sind solche Verbindungen einsetzbar, wie sie vorstehend beschrieben sind.

Der CRC-Wert [g/g] der erfindungsgemäßen Hydrogel-formenden Polymere kann nach den in der Beschreibung angegebenen Methoden gemessen werden und liegt bevorzugt größer 10, insbesondere 11, 12, 13, 14, 15, 16, 18, 20 , 22, 24, oder höher, besonders bevorzugt bei 25 insbesondere bei 26, 27, 28, 29, insbesondere bevorzugt bei 30, 31, 32, 33, 34, 35, 36, 37, 38, 39, 40, 41, 42, 43, 44, 45 oder höher.

Der AUL-0,7psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Der AUL-0,5psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22,23, 24, 25, 26, 27, 28, oder höher.

Der Verseifungsindex VSI der erfindungsgemäßen Hydrogel-formenden Polymere kann nach den in der Beschreibung angegebenen Methoden gemessen werden und liegt bevorzugt kleiner 10, insbesondere 9.5, 9, oder 8.5 oder geringer, besonders bevorzugt kleiner 8, insbesondere 7.5, 7, 6.5, 6, 5.5 oder geringer, insbesondere bevorzugt kleiner 5, insbesondere 4.5, 4, 3.5 oder geringer.

Der Restvernetzergehalt der erfindungsgemäßen Hydrogel-formenden Polymere kann nach den in der Beschreibung angegebenen Methoden gemessen werden und liegt bevorzugt kleiner 30 ppm, besonders bevorzugt kleiner 20 ppm, ganz besonders bevorzugt aber kleiner 10 ppm, insbesondere 9.5 ppm, 9 ppm, oder 8.5 ppm oder geringer, besonders bevorzugt kleiner 8 ppm, insbesondere 7.5 ppm, 7 ppm, 6.5 ppm, 6 ppm, 5.5 ppm oder geringer, insbesondere bevorzugt kleiner 5 ppm, insbesondere 4.5 ppm, 4 ppm, 3.5 ppm oder geringer. Bei Einsatz mehrerer Vernetzer im Gemisch beziehen sich diese Maximalwerte auf jeden einzelnen Vernetzer im Gemisch.

Einsatz und Verwendung der erfindungsgemäßen Hydrogel-formenden Polymere

Die vorliegende Erfindung betrifft ferner die Verwendung der oben genannten Hydrogel-formenden Polymeren in Hygieneartikeln, umfassend

(P) eine obere flüssigkeitsdurchlässige Abdeckung
(Q) eine untere im wesentlichen flüssigkeitsundurchlässige Schicht
(R) einen zwischen (P) und (Q) befindlichen Kern, enthaltend
10 -100 Gew.-% des erfindungsgemäßem Hydrogel-formenden Polymer
0 - 90 Gew.-% hydrophiles Fasermaterial
bevorzugt 20 -100 Gew.-% des erfindungsgemäßem Hydrogel-formenden Polymer, 0 - 80 Gew.-% hydrophiles Fasermaterial
mehr bevorzugt 30 -100 Gew.-% des erfindungsgemäßem Hydrogel-formenden Polymer, 0 - 70 Gew.-% hydrophiles Fasermaterial
noch mehr bevorzugt 40 -100 Gew.-% des erfindungsgemäßem Hydrogel-formenden Polymer, 0 - 60 Gew.-% hydrophiles Fasermaterial
besser bevorzugt 50 -100 Gew.-% des erfindungsgemäßem Hydrogel-formenden Polymer 0 - 50 Gew.-% hydrophiles Fasermaterial
besonders bevorzugt 60 -100 Gew.-% des erfindungsgemäßem Hydrogel-formenden Polymer, 0 - 40 Gew.-% hydrophiles Fasermaterial
insbesondere bevorzugt 70 -100 Gew.-% des erfindungsgemäßem Hydrogel-formenden Polymer, 0 - 30 Gew.-% hydrophiles Fasermaterial
außerordentlich bevorzugt 80 -100 Gew.-% des erfindungsgemäßem Hydrogel-formenden Polymer, 0 - 20 Gew.-% hydrophiles Fasermaterial
am meisten bevorzugt 90 -100 Gew.-% des erfindungsgemäßem Hydrogel-formenden Polymer, 0 -10 Gew.-% hydrophiles Fasermaterial
(S) gegebenenfalls eine sich unmittelbar oberhalb und unterhalb des Kerns (R) sich befindende Tissueschicht und
(T) gegebenenfalls eine zwischen (P) und (R) sich befindende Aufnahmeschicht.

Die Prozentangaben sind so zu verstehen, dass bei 10 -100 Gew.-%, 11, 12, 13, 14, 15, 16, 17, 18, 19 bis jeweils 100 Gew.-% an erfindungsgemäßem Hydrogel-formenden Polymer und alle dazwischenliegenden %-Angaben (z.B. 12,2%) möglich sind und entsprechend hydrophiles Fasermaterial von 0 bis jeweils 89, 88, 87, 86, 85, 83, 82, 81 Gew.-% und dazwischen liegende Prozentangaben (z.B. 87,8%) möglich sind. Liegen weitere Materialien im Kern vor, so verringern sich entsprechend die Prozentwerte an Polymer und Faser. Das analoge gilt für die bevorzugten Bereiche, so z.B. bei außerordentlich bevorzugt können 81, 82, 83, 84, 85, 86, 87, 88, 89 Gew.-% für das erfindungsgemäßem Hydrogel-formenden Polymer und entsprechend 19, 18,17, 16, 15, 14, 13, 12, 11 Gew.-% des Fasermaterials vorliegen. So können im bevorzugten Bereich 20, 21, 22, 23, 24, 25, 26, 27, 28, 29 bis 100 Gew.-% erfindungsgemäßes Hydrogel-formendes Polymer, im mehr bevorzugten Bereich 30, 31, 32, 33, 34, 35, 36, 37, 38, 39 bis 100 Gew.-% erfindungsgemäßes Hydrogel-formendes Polymer, im noch mehr bevorzugten Bereich 40, 41, 42, 43, 44, 45, 46, 47, 48, 49 bis 100 Gew.-% erfindungsgemäßes Hydrogel-formendes Polymer, im besser bevorzugten Bereich 50, 51, 52, 53, 54, 55, 56,

57, 58, 59 bis 100 Gew.-% erfindungsgemäßes Hydrogel-formendes Polymer, im besonders bevorzugten Bereich 60, 61, 62, 63, 64, 65, 66, 67, 68, 69 bis 100 Gew.-% erfindungsgemäßes Hydrogel-formendes Polymer, im insbesonders bevorzugten Bereich 70, 71, 71, 72, 73, 74, 75, 76, 77, 78, 79 bis 100 Gew.-% erfindungsgemäßes Hydrogel-formendes Polymer und im am meisten bevorzugten Bereich 90, 91, 92, 93, 94, 95, 96, 97, 98, 99 oder 100 Gew.-% erfindungsgemäßes Hydrogel-formendes Polymer vorliegen.

Unter Hygieneartikeln sind dabei sowohl Inkontinenzeinlagen und Inkontinenzhosen für Erwachsene als auch Windeln für Babys zu verstehen.

Bei der flüssigkeitsdurchlässigen Abdeckung (P) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 102 388 3 A2.

Die flüssigkeitsundurchlässige Schicht (Q) besteht in der Regel aus einer Folie aus Polyethylen oder Polypropylen. Es können jedoch auch alle anderen Materialien verwendet werden, die man zu flüssigkeitsundurchlässigen Folien verarbeiten kann. Flüssigkeitsundurchlässig bedeutet in diesem Zusammenhang eine Undurchlässigkeit für kondensierte Flüssigkeiten. Gleichzeitig kann die Folie jedoch Durchlässigkeit für den Dampf der Flüssigkeit zeigen, und oftmals vereinen moderne Windelkonstruktionen eine hohe Dampfdurchlässigkeit mit maximaler Undurchlässigkeit für die zugrunde liegende kondensierte Flüssigkeit welche in der Regel Wasser bzw. Urin ist.

Der Kern (R) enthält neben dem erfindungsgemäßen Hydrogel-formendem Polymer hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 - 200 $\mu$m, bevorzugt 10 -100 $\mu$m. Darüber hinaus haben die Fasern eine Mindestlänge von 1 mm.

Der Aufbau und die Form von Windeln ist allgemein bekannt und beispielsweise in der WO 95 / 26 209 S. 66 Zeile 34 bis S. 69 Zeile 11, DE 196 04 601 A1, EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 -17, WO 00/35502, insbesondere Seiten 3-9, DE 19737434, WO 98/8439 beschrieben. Hygieneartikel für die Damenhygiene werden in folgenden Literaturstellen beschrieben. Die erfindungsgemäßen wässrige Flüssigkeiten absorbierende Hydrogel-formende Polymere können dort eingesetzt werden. Literaturstellen Damenhygiene: WO 95/24173: Absorption Article for Controlling Odour, WO 91/11977: Body Fluid Odour Control, EP 389023: Absorbent Sanitary Articles, WO 94/25077: Odour Control Material, WO 97/01317: Absorbent Hygienic Article, WO 99/18905, EP 834297, US 5,762,644, US 5,895,381, WO 98/57609, WO 2000/065083, WO 2000/069485, WO 2000/069484, WO 2000/069481, US 6,123,693, EP 1104666, WO 2001/024755, WO 2001/000115, EP 105373, WO 2001/041692, EP 1074233. Tampons werden in folgenden Schriften beschrieben: WO 98/48753, WO 98/41179, WO 97/09022, WO 98/46182, WO 98/46181, WO 2001/043679, WO 2001/043680, WO 2000/061052, EP 1108408, WO 2001/033962, DE 200020662, WO 2001/001910, WO 2001/001908, WO 2001/001909, WO 2001/001906, WO 2001/001905, WO 2001/24729. Inkontinenzartikel werden in folgenden Schriften beschrieben: Disposable Absorbent Article for Incontinent Individuals: EP 311344 Beschreibung S. 3 - 9; Disposable Absorbent Article: EP 850623; Absorbent Article: WO 95/26207; Absorbent Article: EP 894502; Dry Laid Fibrous Structure: EP 850 616; WO 98/22063; WO 97/49365; EP 903134; EP 887060; EP 887059; EP 887058; EP 887057; EP 887056; EP 931530; WO 99/25284; WO 98/48753. Damenhygiene- und Inkontinenzartikel wird in folgenden Schriften beschrieben: Catamenial Device: WO 93/22998 Beschreibung S. 26 - 33; Absorbent Members for Body Fluids: WO 95/26209 Beschreibung S. 36 - 69; Disposable Absorbent Article: WO 98/20916 Beschreibung S. 13 - 24; Improved Composite Absorbent Structures: EP 306262 Beschreibung S. 3 -14; Body Waste Absorbent Article: WO 99/45973.

Die erfindungsgemäßen Hydrogel-formenden Polymere eignen sich hervorragend als Absorptionsmittel für Wasser und wässrige Flüssigkeiten, so dass sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Einlagerung und Fixierung der erfindungsgemäßen hochquellfähigen Hydrogele

Zusätzlich zu den oben beschriebenen hochquellfähigen Hydrogelen liegen in der absorbierenden Zusammensetzung gemäß der vorliegenden Erfindung Kompositionen vor, welche die hochquellfähigen Hydrogele enthalten oder an denen sie fixiert sind. Jede Komposition ist geeignet, die die hochquellfähigen Hydrogele aufnehmen kann und die darüber hinaus in die Absorptionsschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (airlaid web, wet laid web) oder aus synthetischen

Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines air-laid web ist beispielsweise geschildert in WO 98/28 478. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. Ein derartiges Kammersystem ist detailliert geschildert in EP 0 615 736 A1 S. 7 Zeile 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die hochquellfähigen Hydrogelpartikel zurückzuhalten. Obige Beispiele zur Komposition der absorbierenden Zusammensetzung schließen auch Laminate aus mindestens zwei Schichten mit ein, zwischen die die hochquellfähigen Hydrogele eingebaut und fixiert werden.

Generell besteht die Möglichkeit, Hydrogelpartikel innerhalb des Absorbent Cores zur Verbesserung der sog. Dry- und Wet-Integrity zu fixieren. Unter Dry- und Wet-Integrity versteht man die Fähigkeit, hochquellfähige Hydrogele derart in die absorbierende Zusammensetzung einzubauen, dass sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in WO 95/26 209 S. 37 Zeile 36 bis S. 41 Zeile 14. Methoden zur Erhöhung der Wet Strength finden sich auch in WO 2000/36216 A1.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die hochquellfähigen Hydrogele mit einem Gewichtsanteil von 10 bis 100 Gew.-%, bevorzugt 20 - 100 Gew.-%, mehr bevorzugt 30 -100 Gew.-%, noch mehr bevorzugt 40 -100 Gew.-%, besser bevorzugt 50 -100 Gew.-%, besonders bevorzugt 60 -100 Gew.-%, insbesondere bevorzugt 70 -100 Gew.-%, außerordentlich bevorzugt 80 -100 Gew.-% und am meisten bevorzugt 90 -100 Gew.-% basierend auf dem Gesamtgewicht der Komposition und der hochquellfähigen Hydrogele eingebaut.

Fasermaterialien der absorbierenden Zusammensetzung

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt Patent WO 95/26 209 S. 28 Zeile 9 bis S. 36 Zeile 8.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DAC-RON® oder KODEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75°C und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9 000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner aus 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozess wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikel werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene®557 H, Hercules, Inc. Wilmington Delaware, USA), Polyacrylamid- Überzüge (beschrieben in U.S. Patent 3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Versteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren

zu verhindern.

Chemisch vernetzte Cellulosefasern sind bekannt und in WO 91/11162, U.S. Patent 3,224,926, U.S. Patent 3,440,135, U.S. Patent 3,932,209, U.S. Patent 4,035,147, U.S. Patent 4,822,453, U.S. Patent 4,888,093, U.S. Patent 4,898,642 und U.S. Patent 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Herstellungsverfahren der absorbierenden Zusammensetzung

Die absorbierende Zusammensetzung setzt sich zusammen aus Kompositionen, welche hochquellfähige Hydrogele enthalten, und den hochquellfähigen Hydrogelen, die in besagten Kompositionen vorliegen oder daran fixiert sind.

Beispiele für Verfahren, mit denen man eine absorbierende Zusammensetzung erhält, die beispielsweise aus einem Trägermaterial bestehen, an den ein- oder beidseitig hochquellfähige Hydrogele fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteten hochquellfähigen Hydrogelen (c) besteht, wobei das Mischungsverhältnis von (100 bis 0) synthetische Faser: (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunde bis 3 Minuten, bevorzugt 1 Sekunde bis 1 Minute.

Die absorbierende Zusammensetzung wird im allgemeinen beispielsweise mit einer für Flüssigkeit durchlässigen Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in WO 95/26 209.

Der Vorteil der vorliegenden Erfindung beruht darin, dass die als Vernetzer verwendbaren Ester F nach ihrer Herstellung nicht aufgereinigt werden müssen, besonders dass nicht die (Meth)acrylsäure, bevorzugt Acrylsäure, abgetrennt werden muss, da diese in der Regel ein Monomer zur Herstellung der Hydrogele darstellt.

Experimenteller Teil

In dieser Schrift verwendete ppm- und Prozentangaben beziehen sich, falls nicht anders angegeben, auf Gewichtsprozente und -ppm.

Das erfindungsgemäße Verfahren wird durch das nachfolgende Beispiel näher erläutert.

Beispiele

Herstellung von Acrylat-Rohestern als Superabsorbervernetzer

Die Herstellung der Superabsorbervernetzer erfolgt in den Beispielen durch Veresterung von Polyol oder POlolgemischen mit Acrylsäure, wobei die Abtrennung des Wassers in einer azeotropen Destillation erfolgt. Veresterungskatalysator ist in den Beispielen Schwefelsäure. Die Reaktanden werden zusammen mit einer Stabilisatormischung bestehend aus Hydrochinonmonomethylether, Triphenylphosphit und Hypophosphorige Säure in den Beispielen in Methylcyclohexan als Schleppmittel vorgelegt. Die Reaktionsmischung wird dann auf ca. 98°C erwärmt bis die azeotrope

Destillation beginnt. Während der azeotropen Destillation steigt die Temperatur in der Reaktionsmischung an. Die abgetrennte Wassermenge wird bestimmt. Die Destillation wird abgebrochen, wenn mindestens die theoretische Wassermenge abgetrennt wurde. Anschließend wird das Schleppmittel in einer Vakuumdestillation entfernt. Das Produkt wird abgekühlt und als Vernetzer in der Superabsorberherstellung eingesetzt.

Umsatz und Ausbeute der Umsetzung wird nicht genau bestimmt, da das in der Veresterung abgetrennte Wasser auch Acrylsäure enthält und auch während der Vakuumdestillation des Schleppmittels Acrylsäure entfernt wird. Auch der Rohester enthält noch freie Acrylsäure, die zusammen mit dem Katalysator titriert wird (Säurezahl). Alle Mengenangaben sind, soweit nicht anders angegeben, Gewichtsteile.

Herstellung des Esters

Säurezahlen wurden gem. DIN EN 3682 bestimmt.

Beispiel 1 Herstellung des alkoxylierten Glycols als Grundlage für Ester F

a) Trimethylolpropan - 30 EO - 5 PO
77 g Trimethylolpropan werden mit 0,5 g KOH, 45 % in Wasser, in einem Autoklaven vorgelegt und zusammen bei 80°C und reduziertem Druck (ca. 20 mbar) entwässert. Dann werden bei 145 bis 155°C 759 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150°C nachgerührt. Anschließend werden 167 g Propylenoxid bei 120 bis 130°C über längere Zeit bei erhöhtem Druck zudosiert und ebenfalls abreagieren gelassen. Nach Spülen mit Inertgas und Abkühlen auf 60°C wird der Katalysator durch Zugabe von Natriumpyrophosphat und anschließende Filtration abgetrennt.

b) Analog wird TMP-15-EO hergestellt.

c) Tripropylenglykol ist eine kommerziell verfügbare Diolkomponente.

d) Glycerin - 3 EO wird analog zu Beispiel 1 a) hergestellt.

e) Trimethylolpropan - 3 EO wird analog zu Beispiel 1a) hergestellt.

f) Trimethylolpropan - 1PO - 3 EO wird analog zu Beispiel 1a) hergestellt - zuerst wird aber bei 120 - 130°C Propylenoxid addiert und dann erst bei 145 - 155°C Ethylenoxid.

g) Glycerin - 30 EO - 5 PO wird analog zu Beispiel 1a) hergestellt.

h) Butandiol ist kommerziell verfügbar.

Beispiel 2 Herstellung des Acrylsäureesters

a) Trimethylolpropan - 30 EO - 5 PO - Triacrylat (TMP30EO5POTA)
1427 Teile ca. 30-fach ethoxyliertes und 5-fach propoxyliertes Trimethylolpropan (gemäß Beispiel 1a) wird mit 216 Teilen Acrylsäure und 5 Teilen Schwefelsäure in 345 Teilen Methylcyclohexan verestert. Als Hilfsstoffe werden 2 Teilen Hydrochinonmonomethylether, und 2 Teile $\alpha$-Tocopherol zugesetzt. Es werden 44 Teile Wasser abgetrennt bevor das Schleppmittel durch Vakuumdestillation entfernt wird. Das Produkt wird über K300-Filter gereinigt. Die Säurezahl wird bestimmt. Durch Zugabe von 96 Teilen Acrylsäure wird die Viskosität eingestellt. Die Viskosität des fast farblosen Produktes (Jodfarbzahl 0-1) beträgt ca. 330 mPas.

b) Analog wird TMP-15-EO-triacrylat (TMP15EOTA) hergestellt.

c) Tripropylenglykoldiacrylat (TPGDA) ist kommerziell erhältlich als Laromer TPGDA (BASF AG). Es kann aber auch vollkommen analog zu obigen Beispielen hergestellt werden.

d) Analog wird Glycerin-3 EO-Triacrylat (G3EOTA) hergestellt.

e) Analog wird Trimethylolpropan-3 EO-Triacrylat (TMP3EOTA) hergestellt.

f) Analog wird Trinethylolpropan-1 PO-3 EO-Triacrylat (TMP1 PO3EOTA)hergestellt.

g) Analog wird Glycerin-30 EO-5 PO-Triacrylat (G30EO5POTA) hergestellt.

h) Butandioldiacrylat (BDDA) ist kommerziell erhältlich (BASF AG)

Die in den folgenden Beispielen verwendeten Vernetzergemische sind durch einfaches Mischen der fertigen Acrylatester im angegebenen Gewichtsverhältnis herstellbar. Alternativ können die zugrunde liegenden Polyether gemischt und wie oben beschrieben gemeinsam verestert werden.

Herstellung von Hydrogelen

Zur Bestimmung der Güte der Oberflächenvernetzung kann das getrocknete Hydrogel mit folgenden Testmethoden untersucht werden.

Testmethoden

a) Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 +/- 0,0050 g getrocknetes Hydrogel (Kornfraktion 106 - 850 µm) in einem 60 x 85 mm großen Teebeutel eingewogen, der anschließend verschweißt wird. Der Teebeutel wird für 30 Minuten in einen Überschuss von 0,9 Gew.%igen Kochsalzlösung gegeben (mindestens 0,83 l Kochsalz-Lösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

b) Absorption unter Druck (AUL Absorbency Under Load) (0,7 psi)

Die Messzelle zur Bestimmung der AUL 0,7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 µm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1345 g. Zur Durchführung der Bestimmung der AUL 0,7 psi wird das Gewicht des leeren Plexiglas-Zylinders und der Plastikplatte ermittelt und als Wo notiert. Dann werden 0,900 +/- 0,005 g Hydrogel-formendes Polymer (Korngrößenverteilung 150 - 800 µm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm, einer Höhe von 10 mm und einer Porosität 0 (Duran, Fa. Schott) gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 µm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0,7 psi [g/g]} = [W_b - W_a] / [W_a - W_0]$$

Der AUL 0,5psi wird analog mit geringerem Druck gemessen.

c) Die Bestimmung des nach 16 h extrahierbare Anteils (Extract. 16h) erfolgte analog, wie in EP-A1 811 636, S. 13, Z. 1 bis Z. 19 beschrieben.

Beispiel 3: Herstellung von Superabsorber unter Verwendung der Acrylsäureester aus Beispiel 2 und Gemischen daraus.

Beispiel A (Vergleichsbeispiel)

In einen Lödige-Pflugscharkneter Typ VT 5R-MK (5 l Volumen) werden 180 g entionisiertes Wasser, 220 g Acrylsäure, 2201 g einer 37,3 gew.-%igen Natriumacrylatlösung (100 mol-% neutralisiert) sowie 5,1 g (= 0,60 Gew.-% bezogen auf Acrylsäuremonomer) des Vernetzers Trimethylolpropan-15 EO-Triacrylat vorgelegt und unter Durchperlen von Stickstoff 20 Minuten inertisiert. Dann wird durch Zusatz (verdünnte wässrige Lösungen) von 2,112 g Natriumpersulfat, 0,045 g Ascorbinsäure sowie 0,126 g Wasserstoff-peroxid bei ca. 23°C gestartet. Nach dem Start wird die Temperatur des Heizmantels der Reaktionstemperatur im Reaktor mittels Regelung nachgeführt. Es wird unter Rühren und guter Durchmischung im Kneter polymerisiert. Das letztlich erhaltene krümelige Gel wird dann bei 160°C ca. 3 h im Umlufttrockenschrank getrocknet. Anschliessend wird gemahlen und auf 300-850 Mikrometer abgesiebt. Das erhaltene Hydrogel wird schliesslich charakterisiert.

70 Analog zu Beispiel 3A werden folgende weitere Beispiele hergestellt:

Tab. 1

| Bsp. Nr. | Vernetzertyp | Einsatzmenge bez. Acrylsäure-monomer | Einsatz menge in g |
|---|---|---|---|
| A | Trimethylolpropan-15 EO-Triacrylat | 0,60 Gew.-% | 5,1 g |
| B | Trimethylolpropan-15 EO-Triacrylat | 2,00 Gew.-% | 17,0 g |
| C | TMP - 30 EO - 5 PO - triacrylat (69,3 Gew.-%)<br>Laromer TPGDA (30,7 Gew.-) | 0,60 Gew.-% | 5,1 g |
| D | Glycerin-3 EO-Triacrylat (25,2 Gew.-%)<br>Trimethylolpropan-30 EO-5 PO-triacrylat (74,8 Gew.-%) | 0,60 Gew.-% | 5,1 g |
| E | Trimethylolpropan-3 EO-Triacrylat (28,8 Gew.-%)<br>Trimethylolpropan-30 EO-5 PO-Triacrylat (71,2 Gew.-%) | 2,00 Gew.-% | 17,0 g |
| F | Trimethylolpropan-1 PO-3 EO-Triacrylat (34,0 Gew.-%)<br>Trimethylolpropan-30 EO-5 PO-Triacrylat (66,0 Gew.-%) | 0,60 Gew.-% | 5,1 g |
| G | Butandioldiacrylat (18,3 Gew.-%)<br>Trimethylolpropan-30 EO-5 PO-Triacrylat (81,7 Gew.%) | 0,60 Gew.-% | 5,1 g |
| H | Glycerin-30 EO-5 PO-Triacrylat (69,8 Gew.-%)<br>Laromer TPGDA (30,2 Gew.-%) | 2,00.Gew.-% | 17,0 g |
| I | TMP - 30 EO - 5 PO - triacrylat (7,7 Gew.-%)<br>Laromer TPGDA (92,3 Gew.-%) | 0,30 Gew.-% | 2,6 g |

Die Eigenschaften dieser Hydrogele werden in Tab. 2 zusammengefasst

| Bsp | | CRC | Extrahierbare 16h | AUL 0.3 psi |
|---|---|---|---|---|
| | | [g/g] | [Gew%.-%] | [g/g] |
| A | TMP15EOTA 0.6 | 35,8 | 9,5% | 16,6 |
| B | TMP15EOTA2.0 | 26,4 | 5,1% | 25,0 |
| C | TMP30EO5POTA TPGDA 0.6 | 36,0 | 10,1% | 16,7 |
| D | TMP30EO5POTA G3EOTA 0.6 | 36,7 | 8,6% | 17,0 |
| E | TMP30EO5POTA TMP3EOTA 2.0 | 27,0 | 4,3% | 25,3 |
| F | TMP30EO5POTA TMP1P03EOTA 0.6 | 37,1 | 8,8% | 15,9 |
| G | TMP30EO5POTA BDDA 0.6 | 36,6 | 8,6% | 16,2 |
| H | G30EO5POTA TPGDA 2.0 | 27,6 | 4,5% | 24,7 |
| I | TMP30EO5POTA TPGDA 0.3 | 35,0 | 10,0% | 18,0 |

Wie aus Tab. 2 ersichtlich können die Eigenschaften des Vernetzers der Vergleichsbeispiele mit den erfindungsgemäß berechneten Mengen einer Vielzahl anderer Vernetzer in ähnlicher Weise erhalten werden.
Darüber hinaus offenbart Beispiel 3I wie auf erfindungsgemäße Weise durch Veränderung der Gemischanteile die Vernetzerstärke eingestellt werden kann. Das kann technisch vorteilhaft genutzt werden, indem man durch geeignete Mischung der Komponenten die Gesamtdosiermenge des Vernetzers konstant hält und die Stärke der Vernetzung nur durch das Komponentenverhältnis regelt. Dies ist technisch von Vorteil weil man so die Mischung des Vernetzers in die Monomerlösung im Großmaßstab optimal mit konstanten Mengenströmen einstellen kann.

Nachvernetzung:

Das trockene normale Grundpolymerpulver wird mit einer Lösung aus 0,12 Gew. % N-Hydroxyethyl-2-Oxazolidinon, 3,35 Gew.-% Wasser und 1,65 Gew.-% Propandiol-1,2 -jeweils bezogen auf eingesetztes Polymer- unter Rühren homogen besprüht.
Die Ansatzgröße beträgt jeweils 1,2 kg, das Aufsprühen erfolgt mittels Zweistoffdüse durch Zerstäuben der Lösung mit Stickstoff. Verwendet wird ein Pflugscharmischer der Firma Lödige mit 5 l Arbeitsvolumen.
Das feuchte Pulver wird dann im Trockenschrank bei 180° C für 60 min getempert. Danach wird nochmals bei 850 Mikrometer gesiebt, um Agglomerate zu entfernen.
Für die Trocknung werden nur ca. 100 g feuchtes Polymer benötigt.
Die Eigenschaften dieses nachvemetzten Polymers werden bestimmt.

| Bsp | Grundpolymer | CRC | AUL 0.7 psi |
|---|---|---|---|
| A | 3A (Vgl. Bsp.) | 29,9 | 24,0 |
| A1 | 3B (Vgl. Bsp.) | 24,5 | 21,9 |
| A2 | 3C | 30,2 | 24,5 |
| B | 3E | 25,0 | 22,4 |

In einem weiteren Experiment wurde eine Fraktion mit einer Teilchengröße 300 bis 850 µm hergestellt und diese mit Ethylenglycoldiglycidylether (0,10 Gew.-% bezogen auf Polymer) oberflächen-nachvernetzt. Apparatur und

Einsatzmengen aller Hilfsstoffe sind exakt die selben wie oben beschrieben. Das feuchte Polymer wurde jedoch 1 Stunde bei nur 150°C im Umluftschrank getrocknet.

Verschiedene Performance Daten werden erhoben, um die verschiedenen Vernetzer unter konstanten Bedingungen vergleichen zu können.

| Vernetzer | Gew.-% Vernetzer bezogen auf Acrylsäure | CRC g/g | AUL 0.3psi g/g | AUL 0.7psi g/g | Extrahierbare 16h |
|---|---|---|---|---|---|
| TMP-15EO-Triacrylat | 1.0 | 30,0 | 29,0 | 23,0 | 9,0 |
| | 2,0 | 25,3 | 26,4 | 22,6 | 5,1 |
| | 3,0 | 23,7 | 25,3 | 21,6 | 3,2 |
| TPGDA | 1,0 | 33,8 | 32,3 | 24,3 | 17,3 |
| | 2,0 | 26,6 | 27,8 | 23,8 | 5,0 |
| | 3,0 | 24,8 | 26,3 | 22,5 | 4,4 |
| TMP-30EO-5PO-Triacrylat | 1,0 | 32,9 | 31,8 | 25,0 | 13,7 |
| | 2,0 | 32,2 | 30,0 | 22,8 | 11,7 |
| | 3,0 | 29,3 | 29,1 | 23,2 | 8,0 |
| 70 Gew.-% TMP-30EO-5PO-triacrylat 30 Gew.-% TPGDA | 0,25 | 31,6 | 30,5 | 23,6 | 11,1 |
| | 0,35 | 32,1 | 30,9 | 23,8 | 11,4 |
| | 0,50 | 31,2 | 30,8 | 24,5 | 9,1 |
| | 0,70 | 29,9 | 30,2 | 24,5 | 6,9 |
| | 1,00 | 28,7 | 29,5 | 23,9 | 5,9 |
| | 1,50 | 27,4 | 28,1 | 23,6 | 4,8 |
| | 2,00 | 26,6 | 27,7 | 23,4 | 4,4 |

Aus der vorstehenden Tabelle ist zu erkennen, dass die erfindungsgemäße Mischung aus zwei Vernetzern, vorteilhaft ist gegenüber der Verwendung der einzelnen Vernetzerkomponenten und auch gegenüber einem einzigen ähnlichen Vernetzer mit ähnlichem GFV.

So wird z.B. der Wert von etwa 9 bei den Extrahierbaren schon bei 0,5 Gew.-% erfindungsgemäßer Vernetzerkombination gegenüber über 1,0 oder sogar über 2,0 Gew.-% Vernetzeranteil der Einzelkomponenten und etwa 1,0 Gew.-% Anteil des ähnlichen Einzelvernetzers erreicht. Die CRC- und AUL-Werte der Vernetzerkombination sind ebenfalls bei gleichen Extrahierbaren denen des ähnlichen Einzelvernetzers überlegen.

In einem weiteren Experiment werden 6 verschiedene Gemische hergestellt, um die Erfindung zu demonstrieren. Wie in Beispiel A beschrieben werden damit hydrogelbildende Polymere hergestellt, nur dass an Stelle des reinen Vernetzers dort eine gleiche Menge jeweils eines der Gemische eingesetzt wird. Die Nachvernetzung wird dann wie oben beschrieben mit Ethylengylycoldiglycidylether unter Verwendung der 300 - 850 μm Fraktion durchgeführt.

| Mischung | Vernetzer | Gew.% in Mischung | Funktionalität | $M_w$ | $M_w/F$ | Molenbruch ($\alpha$) | Einsatzmenge bez. auf Acrylsäure | CRC | AUL 0.7 psi | Extrahierbare 16h |
|---|---|---|---|---|---|---|---|---|---|---|
| 1 | **Trimethylolpropantrimethacrylat** | **21,4%** | 3 | 338 | 113 | 0,61 | 0,6 Gew.% | 30,5 | 24,7 | 9,0% |
| | **TMP-30 EO-5 PO-Triacrylate** | **78,6%** | 3 | 1906 | 635 | 0,39 | | | | |
| 2 | **Glycerindiacrylat** | **18,6%** | 2 | 200 | 100 | 0,59 | 0,6 Gew.% | 31,0 | 25 | 8,9% |
| | **TMP-30 EO-5 PO-Triacrylat** | **81,4%** | 3 | 1906 | 635 | 0,41 | | | | |
| 3 | **Polyethylenglykol-300-Diacrylat** | **47,0%** | 2 | 408 | 204 | 0,73 | 0,6 Gew.% | 30,3 | 24,3 | 8,2% |
| | **TMP-30 EO-5 PO-Triacrylat** | **53,0%** | 3 | 1906 | 635 | 0,27 | | | | |
| 4 | **Glycerin-3 PO-Triacrylat** | **28,8%** | 3 | 428 | 143 | 0,64 | 0,6 Gew.%. | 30,1 | 25,2 | 7,9% |
| | **TMP-30 EO-5 PO-Triacrylat** | **71,2%** | 3 | 1906 | 635 | 0,36 | | | | |
| 5 | **Glycerin-3 EO-Triacrylat** | **26,6%** | 3 | 386 | 129 | 0,66 | 0,6 Gew.% | 30,8 | 25,6 | 9,7% |
| | **TMP- 40EO - TA** | **73,4%** | 3 | 2056 | 685 | 0,34 | | | | |
| 6 | **Glycerin-3 EO-Triacrylat** | **26,2%** | 3 | 386 | 129 | 0,65 | 0,6 Gew.% | 30,1 | 24,9 | 8,0% |
| | **Glycerin - 40 EO - Triacrylat** | **73,8%** | 3 | 2014 | 671 | 0,35 | | | | |

**Patentansprüche**

1. Gemisch von mindestens zwei Verbindungen mit jeweils mindestens zwei Doppelbindungen, wobei das Gemisch ein GFV von 240 bis 600 g/mol Doppelbindung aufweist, und zumindest zwei der Verbindungen jeweils mindestens zwei (Meth)acrylsäureester als Doppelbindungskomponente enthalten, und GFV bedeutet:

$$\sum_{i=1}^{n} \alpha_i \times MW_i / Z_i = GFV$$

mit

$$\sum_{i=1}^{n} \alpha_i = 1$$

$\alpha_i$ entspricht dem molaren Anteil der Verbindung (i) im Gemisch,
n entspricht der Anzahl der Verbindungen im Gemisch und n ist $\geq 2$,
$Z_i$ entspricht der Anzahl der Doppelbindungen in der Verbindung (i),
$MW_i$ entspricht dem Molekulargewicht der Verbindung (i).

2. Gemisch gemäß Anspruch 1, wobei das Gemisch ein GFV zwischen 240 und 400 g/mol Doppelbindung, bevorzugt ein GFV zwischen 250 und 350 g/mol Doppelbindung aufweist.

3. Gemisch gemäß einem der Ansprüche 1 oder 2, wobei n 2, 3 oder 4 bevorzugt 2 bedeutet.

4. Gemisch gemäß einem der Ansprüche 1 bis 3, wobei zwischen den Verhältnissen MW/Z von zwei Verbindungen mindestens ein Unterschied von mindestens 50 g/mol Doppelbindung, bevorzugt von mindestens 100 g/mol Doppelbindung, besonders bevorzugt mindestens 250 g/mol Doppelbindung liegt.

5. Gemisch gemäß einem der Ansprüche 1 bis 4, wobei eine Verbindung ein Verhältnis MW/Z von unter 400 g/mol Doppelbindung, bevorzugt von unter 300 g/mol Doppelbindung, besonders bevorzugt unter 200 g/mol Doppelbindung, insbesondere unter 150 g/mol Doppelbindung aufweist.

6. Gemisch gemäß einem der Ansprüche 1 bis 5, wobei eine Verbindung ein Verhältnis MW/Z von über 400 g/mol Doppelbindung und unter 10000 g/mol Doppelbindung, bevorzugt von über 600 g/mol Doppelbindung und unter 1000 g/mol Doppelbindung aufweist.

7. Gemisch gemäß einem der Ansprüche 1 bis 6, wobei Z mindestens einer Verbindung zwischen 2 und 6, bevorzugt bei 2, 3 oder 4 liegt.

8. Gemisch gemäß einer der Ansprüche 1 bis 7, wobei die Verbindungen Ester $F_i$ sind die durch die Veresterung von Polyalkoholen $A_i$ mit (Meth)acrylsäure zugänglich sind und jeder Polyalkohol $A_i$ $Z_i$ Hydroxyfunktionen und 2 bis 50 Kohlenstoffatome aufweist.

9. Gemisch gemäß einer der Ansprüche 1 bis 8, wobei eine Verbindung durch eine der folgenden Formeln repräsentiert wird:

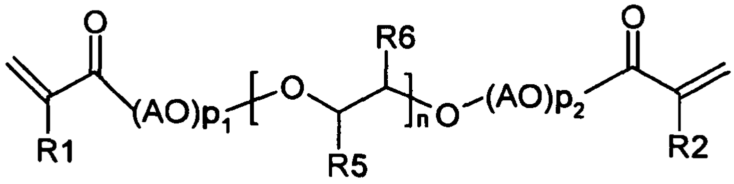

Ia,

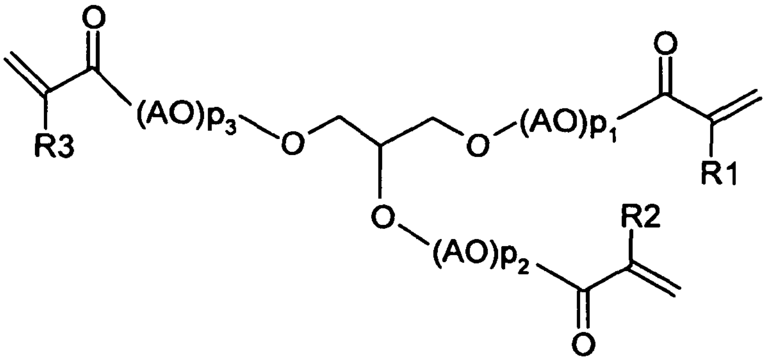

Ib,

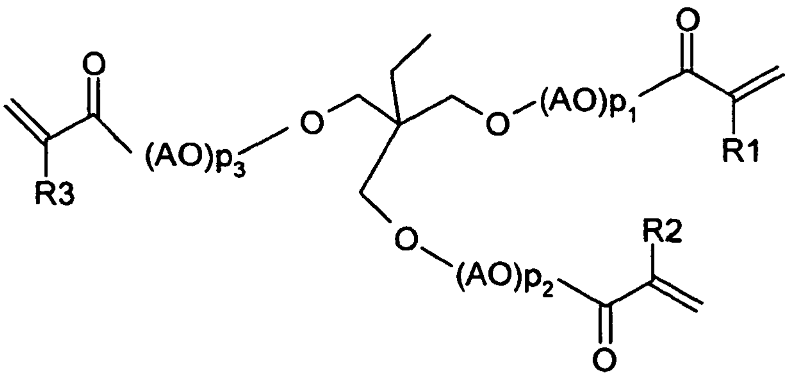

Ic,

oder

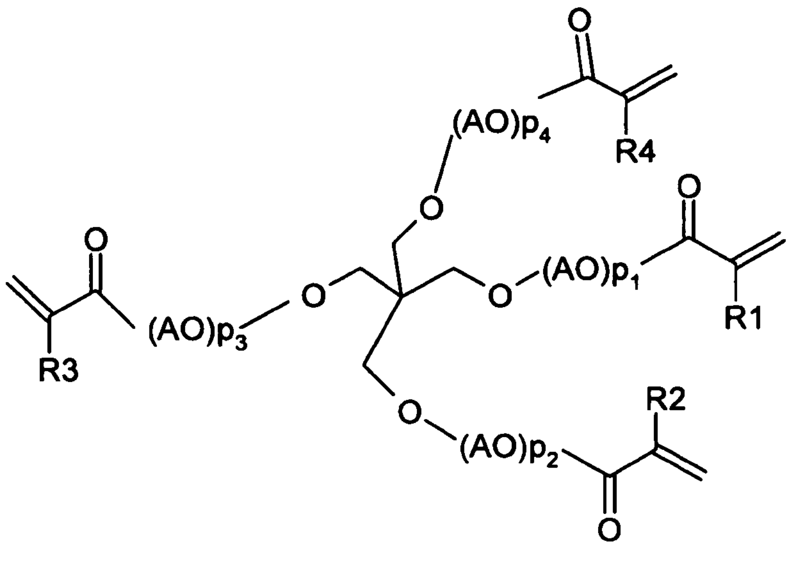

Id

mit AO bedeutet für jedes AO unabhängig voneinander -O-CHR7-CHR8- oder -CHR7-CHR8-O-, mit R7 und R8 unabhängig voneinander H, lineares oder verzweigtes C1-C8-Alkyl,
R5 und R6 unabhängig voneinander H, lineares oder verzweigtes C1-C8-Alkyl,
n ist 1, 2 oder 3
p1 ist 0, 1 oder 2,
p2 ist 0, 1 oder 2,
p3 ist 0, 1 oder 2,
p4 ist 0, 1 oder 2,
R1, R2, R3, R4 unabhängig voneinander H oder CH3,

**10.** Gemisch gemäß einer der Ansprüche 1 bis 9, wobei eine Verbindung durch eine der folgenden Formeln repräsentiert wird:

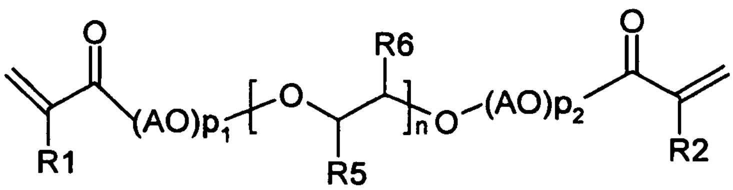

Ia,

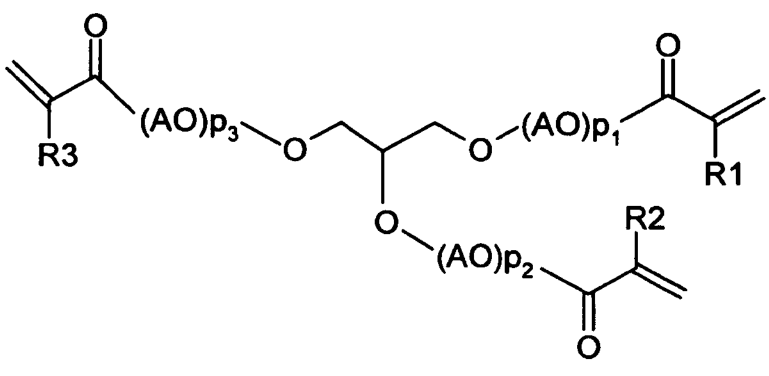

Ib,

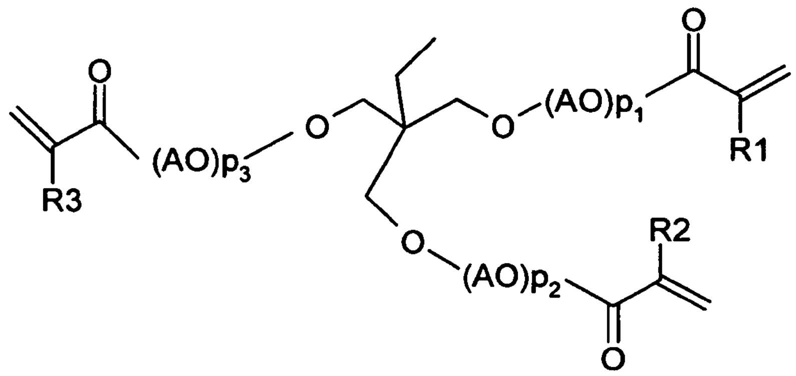

Ic,

oder

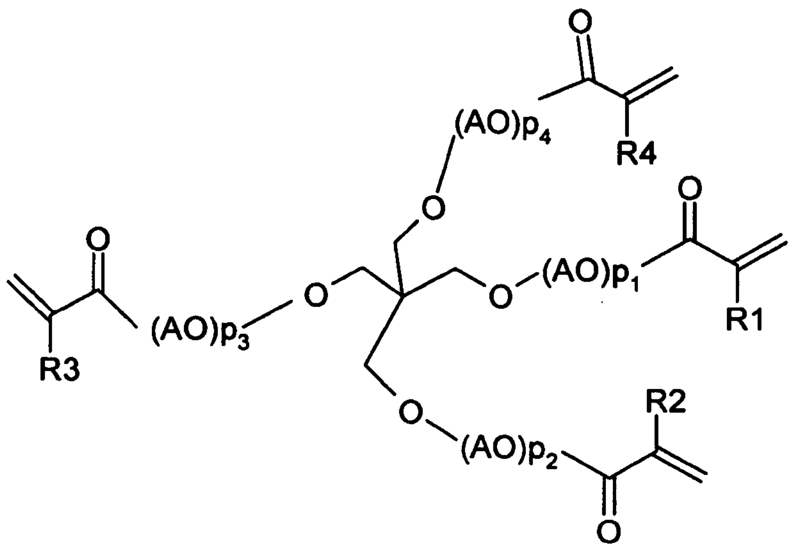

Id

mit AO bedeutet für jedes AO unabhängig voneinander -O-CHR7-CHR8- oder -CHR7-CHR8-O mit R7 und R8 unabhängig voneinander H, lineares oder verzweigtes C1-C8-Alkyl,
R5 und R6 unabhängig voneinander H, lineares oder verzweigtes C1-C8-Alkyl,
n ist 1, 2, 3, 4, 5, 6, 7, 8, 9, 10, 11, 12, 13, 14, 15, 16, 17, 18, 19 oder 20,
p1 ist 7, 8, 9, 10, 11, 12, 13, 14, 15, 16, 17, 18, 19 oder 20,
p2 ist 7, 8, 9, 10, 11, 12, 13, 14, 15, 16, 17, 18, 19 oder 20,
p3 ist 7, 8, 9, 10, 11, 12, 13, 14, 15, 16, 17, 18, 19 oder 20,
p4 ist 7, 8, 9, 10, 11, 12, 13, 14, 15, 16, 17, 18, 19 oder 20,
R1, R2, R3, R4 unabhängig voneinander H oder CH3.

**11.** Gemisch gemäß einer der Ansprüche 9 oder 10, wobei AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
R5 und R6 unabhängig voneinander H oder CH3

**12.** Verfahren zur Herstellung eines Estergemisches aus den Estern $F_i$ gemäß einem der Ansprüche 1 bis 11 ausgehend von einem Alkoholgemisch aus den Polyalkoholen $A_i$ umfassend die Schritte

a) Umsetzung von den Polyalkoholen $A_i$ mit (Meth)acrylsäure in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Estergemisches aus den Estern $F_i$,
b) gegebenenfalls Entfernen zumindest eines Teils des in a) entstehenden Wassers aus dem Reaktionsgemisch, wobei b) während und/oder nach a) erfolgen kann,
f) gegebenenfalls Neutralisation des Reaktionsgemischs,
h) falls ein Lösungsmittel E eingesetzt wurde gegebenenfalls Entfernen dieses Lösungsmittels durch Destillation und/oder
i) Strippen mit einem unter den Reaktionsbedingungen inerten Gas.

**13.** Verfahren zur Herstellung eines Estergemischs aus den Estern $F_i$ nach Anspruch 12, **dadurch gekennzeichnet, dass**

- der molare Überschuss (Meth)acrylsäure zu den Polyalkoholen $A_i$ mindestens $5^*Z_i$ mol-% beträgt und
- die in dem nach dem letzten Schritt erhaltenen Reaktionsgemisch enthaltene, gegebenenfalls neutralisierte (Meth)acrylsäure im wesentlichen im Reaktionsgemisch verbleibt.

**14.** Verfahren zur Herstellung eines Estergemischs aus den Estern $F_i$ nach einem der Ansprüche 12 oder 13, **dadurch gekennzeichnet, dass** die (Meth)acrylsäure aus dem nach dem letzten Schritt erhaltenen Estergemisch enthaltende Reaktionsgemisch zu nicht mehr als 75 Gew.-% abgetrennt wird.

**15.** Verfahren zur Herstellung eines Estergemischs aus den Estern $F_i$ nach einem der Ansprüche 12 bis 14, **dadurch gekennzeichnet, dass** das nach dem letzten Schritt erhaltene Estergemisch enthaltende Reaktionsgemisch eine Säurezahl gem. DIN EN 3682 von mindestens 25 mg KOH/g aufweist.

**16.** Verfahren zur Herstellung eines Estergemischs aus den Estern $F_i$, nach einem der Ansprüche 12 bis 15, **dadurch gekennzeichnet, dass** das nach dem letzten Schritt erhaltene Estergemisch enthaltende Reaktionsgemisch einen Gehalt an (Meth)acrylsäure von mindestens 0,5 Gew.-% aufweist.

**17.** Verfahren zur Herstellung eines Estergemischs aus den Estern $F_i$ nach einem der Ansprüche 12 bis 16, **dadurch gekennzeichnet, dass** in der Umsetzung a) das molare Verhältnis der (Meth)acrylsäure zum Alkoholgemisch $A_i$ mindestens $5^*Z_i$ :1 beträgt.

**18.** Verfahren zur Herstellung eines vernetzten Hydrogels, umfassend die Schritte

k) Polymerisieren eines Estergemisches aus den Estern $F_i$ gemäß einem der Ansprüche 1 bis 11 mit (Meth) acrylsäure, mit gegebenenfalls zusätzlichen monoethylenisch ungesättigten Verbindungen N, sowie gegebenenfalls mindestens einem weiteren copolymerisierbaren hydrophilen Monomer M in Gegenwart mindestens eines Radikalstarters K und gegebenenfalls mindestens einer Pfropfgrundlage L,
l) gegebenenfalls Nachvernetzung des aus k) erhaltenen Reaktionsgemisches,
m) Trocknung des aus k) oder l) erhaltenen Reaktionsgemisches und
n) gegebenenfalls Mahlen und/oder Sieben des aus k), l) oder m) erhaltenen Reaktionsgemisches.

**19.** Verfahren zur Herstellung eines vernetzten Hydrogels, umfassend die Schritte a) bis i) gemäß einem der Ansprüche 12 bis 17 und zusätzlich

k) Polymerisieren des Reaktionsgemischs aus einer der Stufen a) bis i), soweit durchlaufen, mit gegebenenfalls zusätzlichen monoethylenisch ungesättigten Verbindungen N, sowie gegebenenfalls mindestens einem weiteren copolymerisierbaren hydrophilen Monomer M in Gegenwart mindestens eines Radikalstarters K und gegebenenfalls mindestens einer Pfropfgrundlage L,
l) gegebenenfalls Nachvernetzung des aus k) erhaltenen Reaktionsgemisches,
m) Trocknung des aus k) oder l) erhaltenen Reaktionsgemisches und
n) gegebenenfalls Mahlen und/oder Sieben des aus k), l) oder m) erhaltenen Reaktionsgemisches.

**20.** Polymer, erhältlich nach einem Verfahren gemäß einem der Ansprüche 18 oder 19.

**21.** Vernetztes Hydrogel, enthaltend mindestens ein hydrophiles Monomer M in einpolymerisierter Form, vernetzt mit einem Estergemisch aus den Estern $F_i$ gemäß einem der Ansprüche 1 bis 11.

**22.** Vernetztes Hydrogel, enthaltend mindestens ein hydrophiles Monomer M in einpolymerisierter Form, vernetzt mit einem Estergemisch aus den Estern $F_i$ enthaltenden Reaktionsgemisch, wie es erhältlich nach einem Verfahren der Ansprüche 12 bis 15 ist.

**23.** Verwendung eines Polymers gemäß einem der Ansprüche 20 bis 22 in Hygieneartikeln, Verpackungsmaterialien und in Nonwovens.

**24.** Stoffgemisch, enthaltend

- 0,1 bis 40 Gew.-% mindestens eines Estergemisches aus den Estern $F_i$ gemäß einem der Ansprüche 1 bis 11, und (Meth)acrylsäure,
- 0,5 - 99,9 Gew.-% mindestens eines hydrophilen Monomers M,
- 0 - 10 Gew.-% mindestens eines Veresterungskatalysators C,
- 0 - 5 Gew.-% mindestens einen Polymerisationsinhibitors D und
- 0 - 10 Gew.-% eines Lösungsmittels E,

mit der Maßgabe, dass die Summe immer 100 Gew.-% beträgt.

**25.** Stoffgemisch gemäß Anspruch 24, enthaltend zusätzlich

- Verdünnungsmittel G ad 100 Gew.-%.

**26.** Vernetztes Hydrogel, erhältlich aus einem Stoffgemisch gemäß Anspruch 24 oder 25 und zusätzlich

l) gegebenenfalls Nachvernetzung des erhaltenen Reaktionsgemisches,
m) Trocknung des direkt erhaltenen oder aus l) erhaltenen Reaktionsgemisches und
n) gegebenenfalls Mahlen und/oder Sieben des direkt erhaltenen oder aus l) oder m) erhaltenen Reaktionsgemisches.

**27.** Verwendung eines Reaktionsgemisches erhältlich nach einem der Ansprüche 12 bis 17 oder eines Stoffgemisches nach Anspruch 24 oder 25

- als Radikalvernetzer von wasserabsorbierenden Hydrogelen,
- als Ausgangsstoff für die Herstellung von Polymerdispersionen,
- als Ausgangsstoff für die Herstellung von Polyacrylaten,
- als Lackrohstoff oder
- als Zementadditiv.

**28.** Vernetztes Hydrogel gemäß einem der Ansprüche 20, 21, 22 oder 26 mit einem Restvernetzergehalt kleiner 10 ppm, bevorzugt kleiner 8 ppm, besonders bevorzugt kleiner 5 ppm.

**29.** Verwendung eines Estergemisches aus den Estern $F_i$ gemäß einem der Ansprüche 1 bis 11 zur Herstellung von wässrige Flüssigkeiten absorbierende Hydrogel-formende Polymere.

**30.** Verwendung eines Estergemisches gemäß Anspruch 29, wobei jede Esterkomponente $F_i$ bezogen auf die Gesamtmenge der Monomere zu weniger als 2 Gew.-%, bevorzugt 1 Gew.-%, vorliegt.

## Claims

**1.** A mixture of at least two compounds each having at least two double bonds, said mixture having a WFR from 240 to 600 g/mol of double bond and at least two of said compounds each comprising at least two (meth)acrylic esters as double bond component, WFR being given by:

$$\sum_{i=1}^{n} \alpha_i \times MW_i / Z_i = WFR$$

where

$$\sum_{i=1}^{n} \alpha_i = 1$$

$\alpha_i$ is equal to the molar fraction of compound (i) in said mixture,
n is equal to the number of compounds in said mixture and n is ≥ 2,
$Z_i$ is equal to the number of double bonds in said compound (i),
$MW_i$ is equal to the molecular weight of said compound (i).

**2.** The mixture according to claim 1 which has a WFR between 240 and 400 g/mol of double bond and preferably a WFR between 250 and 350 g/mol of double bond.

**3.** The mixture according to either of claims 1 and 2
wherein n is 2, 3 or 4 preferably 2.

**4.** The mixture according to any of claims 1 to 3
wherein the MW/Z ratios of two compounds differ at least by at least 50 g/mol of double bond, preferably by at least 100 g/mol of double bond and more preferably by at least 250 g/mol of double bond.

**5.** The mixture according to any of claims 1 to 4 wherein one compound has an MW/Z ratio of below 400 g/mol of double bond, preferably below 300 g/mol of double bond, more preferably below 200 g/mol of double bond and especially below 150 g/mol of double bond.

**6.** The mixture according to any of claims 1 to 5
wherein one compound has an MW/Z ratio of above 400 g/mol of double bond and below 10 000 g/mol of double bond and preferably of above 600 g/mol of double bond and below 1000 g/mol of double bond.

**7.** The mixture according to any of claims 1 to 6
wherein Z of at least one compound is between 2 and 6 and preferably is 2, 3 or 4.

**8.** The mixture according to any of claims 1 to 7
wherein said compounds are esters $F_i$ which are obtainable by esterification of polyalcohols $A_i$ with (meth)acrylic acid and each polyalcohol $A_i$ has $Z_i$ hydroxyl functions and from 2 to 50 carbon atoms.

**9.** The mixture according to any of claims 1 to 8
wherein one compound is represented by one of the following formulae:

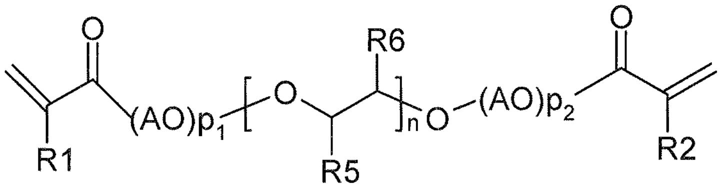

Ia,

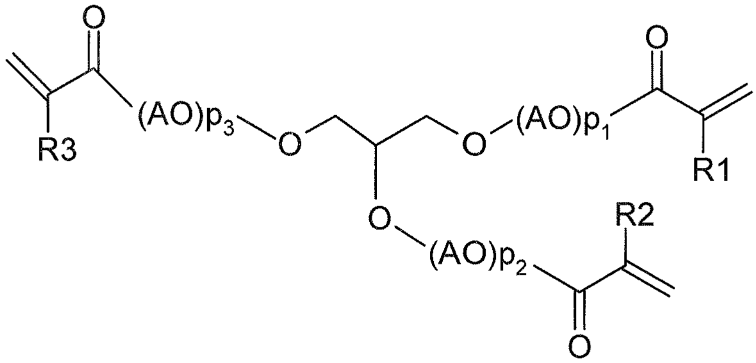

Ib,

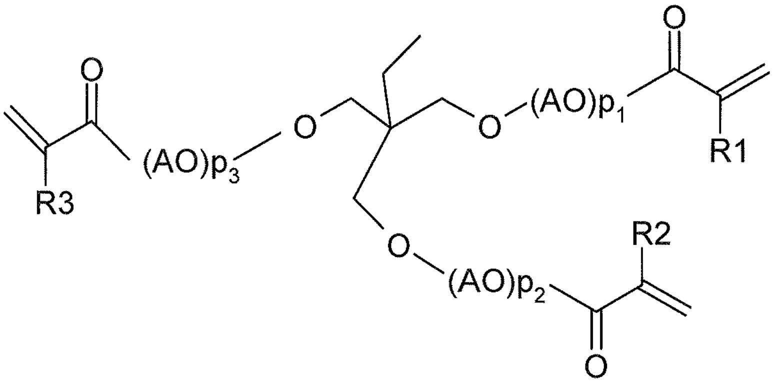

Ic,

or

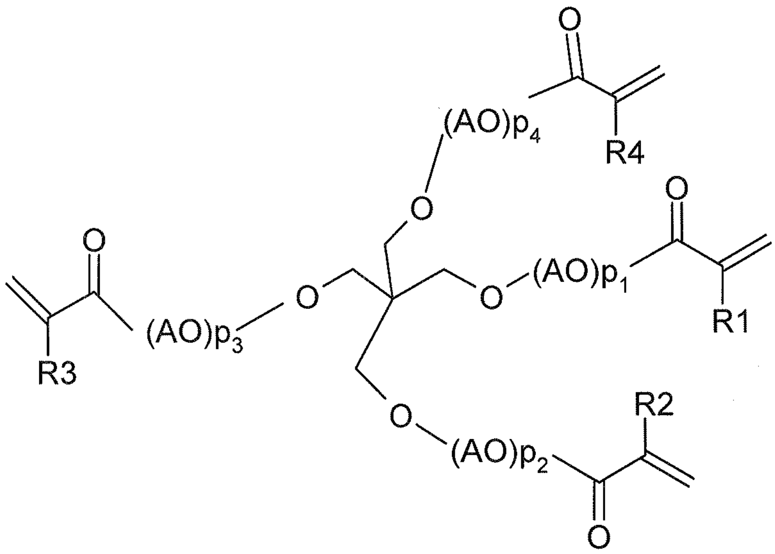

Id

where AO is independently at each instance - O-CHR7-CHR8- or -CHR7-CHR8-O- where R7 and R8 are independently H, linear or branched C1-C8-alkyl,
R5 and R6 are independently H, linear or branched C1-C8-alkyl,
n is 1, 2 or 3
p1 is 0, 1 or 2,
p2 is 0, 1 or 2,
p3 is 0, 1 or 2,
p4 is 0, 1 or 2,
R1, R2, R3, R4 are independently H or CH3.

**10.** The mixture according to any of claims 1 to 9 wherein one compound is represented by one of the following formulae:

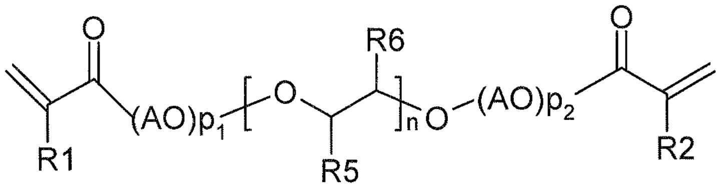

Ia,

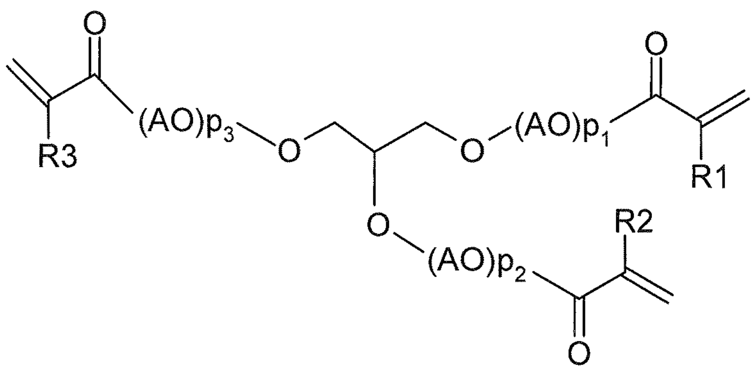

Ib,

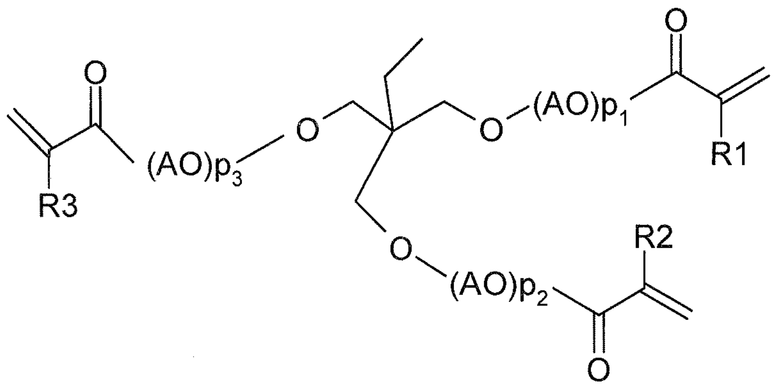

Ic,

or

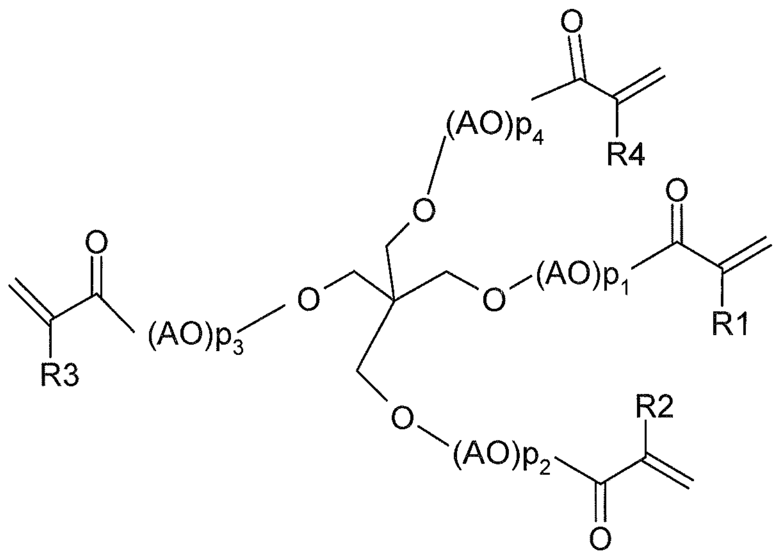

Id

where AO is independently at each instance - O-CHR7-CHR8- or -CHR7-CHR8-O where R7 and R8 are independently H, linear or branched C1-C8-alkyl,
R5 and R6 are independently H, linear or branched C1-C8-alkyl,
n is 1, 2, 3, 4, 5, 6, 7, 8, 9, 10, 11, 12, 13, 14, 15, 16, 17, 18, 19 or 20,
p1 is 7, 8, 9, 10, 11, 12, 13, 14, 15, 16, 17, 18, 19 or 20,
p2 is 7, 8, 9, 10, 11, 12, 13, 14, 15, 16, 17, 18, 19 or 20,
p3 is 7, 8, 9, 10, 11, 12, 13, 14, 15, 16, 17, 18, 19 or 20,
p4 is 7, 8, 9, 10, 11, 12, 13, 14, 15, 16, 17, 18, 19 or 20,
R1, R2, R3, R4 are independently H or CH3.

**11.** The mixture according to either of claims 9 and 10 wherein AO is independently at each instance EO or PO, where EO is O-CH2-CH2-,
PO is independently O-CH2-CH(CH3)- or O-CH(CH3)-CH2-
R5 and R6 are independently H or CH3

**12.** A process for preparing an ester mixture of said esters $F_i$ according to any of claims 1 to 11 by starting from an alcohol mixture of said polyalcohols $A_i$, comprising the steps of

a) reacting said polyalcohols $A_i$ with (meth)acrylic acid in the presence of at least one esterification catalyst C

and of at least one polymerization inhibitor D and optionally also of a water-azeotroping solvent E to form an ester mixture of said esters $F_i$,
b) optionally removing from the reaction mixture some or all of the water formed in a), during and/or after a),
f) optionally neutralizing said reaction mixture,
h) when a solvent E was used, optionally removing this solvent by distillation, and/or
i) stripping with a gas which is inert under the reaction conditions.

**13.** The process for preparing an ester mixture of said esters $F_i$ according to claim 12 wherein

- the molar excess of (meth)acrylic acid over said polyalcohols $A_i$ is at least $5*Z_i$ mol% and
- the optionally neutralized (meth)acrylic acid present in said reaction mixture after the last step substantially remains in said reaction mixture.

**14.** The process for preparing an ester mixture of said esters $F_i$ according to either of claims 12 and 13 wherein the (meth)acrylic acid is not more than 75% by weight removed from said reaction mixture obtained after said last step, which reaction mixture comprises ester mixture.

**15.** The process for preparing an ester mixture of said esters $F_i$ according to any of claims 12 to 14 wherein said reaction mixture obtained after said last step, which comprises ester mixture, has a DIN EN 3682 acid number of at least 25 mg KOH/g.

**16.** The process for preparing an ester mixture of said esters $F_i$ according to any of claims 12 to 15 wherein said reaction mixture obtained after said last step, which comprises ester mixture, has a (meth)acrylic acid content of at least 0.5% by weight.

**17.** The process for preparing an ester mixture of said esters $F_i$ according to any of claims 12 to 16 wherein the molar ratio of (meth)acrylic acid to alcohol mixture $A_i$ in reaction a) is at least $5*Z_i$ :1.

**18.** A process for preparing a crosslinked hydrogel, comprising the steps of

k) polymerizing an ester mixture of said esters $F_i$ according to any of claims 1 to 11 with (meth)acrylic acid, with optionally additional monoethylenically unsaturated compounds N and optionally also at least one further copolymerizable hydrophilic monomer M in the presence of at least one free-radical initiator K and optionally of at least one grafting base L,
l) optionally postcrosslinking the reaction mixture obtained from k),
m) drying the reaction mixture obtained from k) or 1), and
n) optionally grinding and/or sieving the reaction mixture obtained from k), 1) or m).

**19.** A process for preparing a crosslinked hydrogel, comprising steps a) to i) according to any of claims 12 to 17 and additionally

k) polymerizing the reaction mixture from one of stages a) to i) if performed, with optionally additional monoethylenically unsaturated compounds N and optionally also at least one further copolymerizable hydrophilic monomer M in the presence of at least one free-radical initiator K and optionally of at least one grafting base L,
l) optionally postcrosslinking the reaction mixture obtained from k),
m) drying the reaction mixture obtained from k) or 1), and
n) optionally grinding and/or sieving the reaction mixture obtained from k), 1) or m).

**20.** Polymer obtainable according to a process according to either of claims 18 and 19.

**21.** Crosslinked hydrogel comprising at least one hydrophilic monomer M in copolymerized form crosslinked with an ester mixture of said esters $F_i$ according to any of claims 1 to 11.

**22.** Crosslinked hydrogel comprising at least one hydrophilic monomer M in copolymerized form crosslinked with a reaction mixture which comprises an ester mixture of said esters $F_i$ and is obtainable according to a process of claims 12 to 15.

**23.** The use of a polymer according to any of claims 20 to 22 in hygiene articles, packaging materials and in nonwovens.

**24.** A composition of matter comprising

- from 0.1% to 40% by weight of at least one ester mixture of said esters $F_i$ according to any of claims 1 to 11 and (meth)acrylic acid,
- 0.5 - 99.9% by weight of at least one hydrophilic monomer M,
- 0 - 10% by weight of at least one esterification catalyst C,
- 0 - 5% by weight of at least one polymerization inhibitor D, and
- 0 - 10% by weight of at least one solvent E, with the proviso that the sum total is always 100% by weight.

**25.** The composition of matter according to claim 24, further comprising

- a diluent G ad 100% by weight.

**26.** Crosslinked hydrogel obtainable from a composition of matter according to claim 24 or 25 and additionally

l) optionally postcrosslinking the reaction mixture obtained,
m) drying the reaction mixture obtained directly or obtained from 1), and
n) optionally grinding and/or sieving the reaction mixture obtained directly or obtained from 1) or m).

**27.** The use of a reaction mixture obtainable according to any of claims 12 to 17 or of a composition of matter according to claim 24 or 25

- as a free-radical crosslinker of water-absorbing hydrogels,
- as a starting material for preparing polymer dispersions,
- as a starting material for preparing polyacrylates,
- as a paint raw material, or
- as a cement additive.

**28.** The crosslinked hydrogel according to any of claims 20, 21, 22 or 26 which has a residual crosslinker content of less than 10 ppm, preferably less than 8 ppm and more preferably less than 5 ppm.

**29.** The use of an ester mixture of said esters $F_i$ according to any of claims 1 to 11 for preparing hydrogel-forming polymers capable of absorbing aqueous fluids.

**30.** The use of an ester mixture according to claim 29 wherein each ester component $F_i$ is present at less than 2% by weight and preferably 1% by weight based on the total amount of monomers.

**Revendications**

**1.** Mélange d'au moins deux composés comportant chacun au moins deux doubles liaisons, le mélange présentant un rapport pondéral de fonctionnalité GFV de 240 à 600 g/mole de double liaison, et au moins deux des composés contenant chacun au moins deux esters d'acide (méth)acrylique en tant que composant à doubles liaisons, et GFV signifiant :

$$\sum_{i=1}^{n} \alpha_i \times MW_i / Z_i = GFV$$

où

$$\sum_{i=1}^{n} \alpha_i = 1$$

$\alpha_i$ correspond à la proportion molaire du composé (i) dans le mélange,
n correspond au nombre des composés dans le mélange et n est ≥ 2,
Z correspond au nombre des doubles liaisons dans le composé (i),
$MW_i$ correspond à la masse moléculaire du composé (i).

2. Mélange selon la revendication 1, le mélange présentant un rapport GFV compris entre 240 et 400 g/mole, de préférence un rapport GFV compris entre 250 et 350 g/mole de double liaison.

3. Mélange selon l'une quelconque des revendications 1 ou 2, dans lequel n représente 2, 3 ou 4, de préférence 2.

4. Mélange selon l'une quelconque des revendications 1 à 3, dans lequel entre les rapports MW/Z de deux composés il existe au moins une différence d'au moins 50 g/mole de double liaison, de préférence d'au moins 100 g/mole de double liaison, de façon particulièrement préférée d'au moins 250 g/mole de double liaison.

5. Mélange selon l'une quelconque des revendications 1 à 4, dans lequel un composé présente un rapport MW/Z de moins de 400 g/mole de double liaison, de préférence de moins de 300 g/mole de double liaison, de façon particulièrement préférée de moins de 200 g/mole de double liaison, en particulier de moins de 150 g/mole de doubles liaison.

6. Mélange selon l'une quelconque des revendications 1 à 5, dans lequel un composé présente un rapport MW/Z de plus de 400 g/mole de double liaison et de moins de 10 000 g/mole de double liaison, de préférence de plus de 600 g/mole de double liaison et de moins de 1 000 g/mole de double liaison.

7. Mélange selon l'une quelconque des revendications 1 à 6, dans lequel la valeur Z d'au moins un composé est comprise entre 2 et 6, de préférence est égale à 2, 3 ou 4.

8. Mélange selon l'une quelconque des revendications 1 à 7, dans lequel les composés ester $F_i$ sont ceux qui peuvent être obtenus par l'estérification de polyalcools $A_i$ par l'acide (méth)acrylique et chaque polyalcool $A_i$ comporte $Z_i$ fonctions hydroxy et de 2 à 50 atomes de carbone.

9. Mélange selon l'une quelconque des revendications 1 à 8, dans lequel un composé est représenté par l'une des formules suivantes :

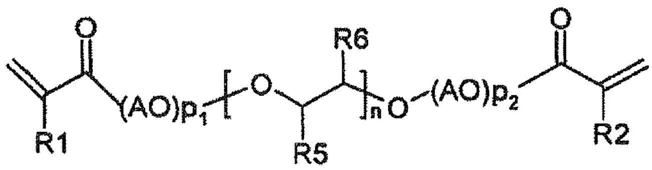

Ia,

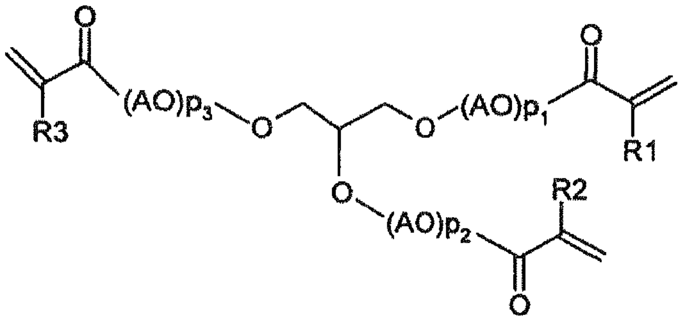

Ib,

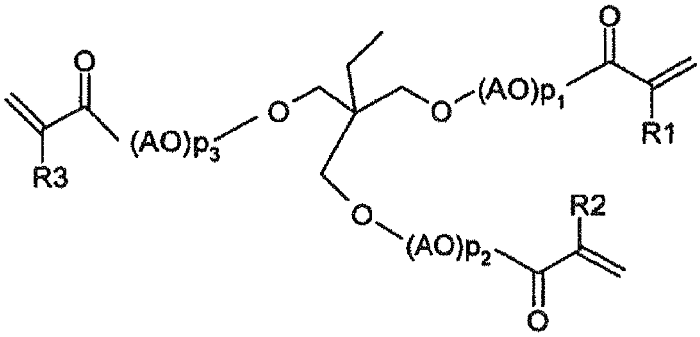

Ic,

ou

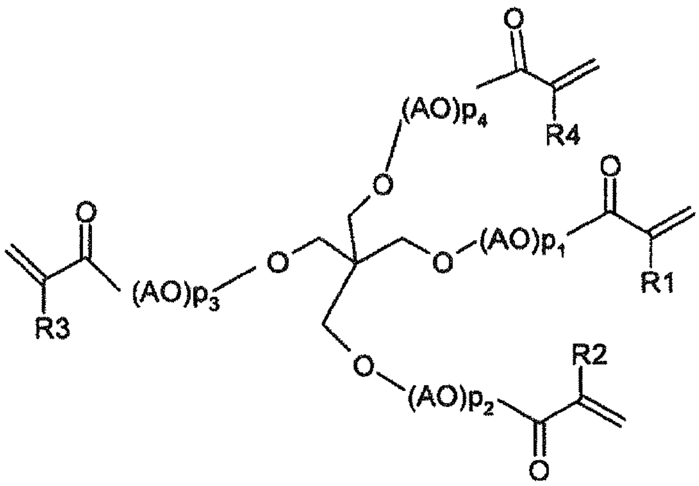

Id

où AO représente, indépendamment pour chaque AO, un groupe -O-CHR7-CHR8- ou -CHR7-CHR8-O-, R7 et R8 représentant, indépendamment l'un de l'autre, H, un groupe alkyle en $C_1$-$C_8$ linéaire ou ramifié,
R5 et R6 représentent, indépendamment l'un de l'autre, H, un groupe alkyle en $C_1$-$C_8$ linéaire ou ramifié,
n est 1, 2 ou 3,
p1 est 0, 1 ou 2,
p2 est 0, 1 ou 2,
p3 est 0, 1 ou 2,
p4 est 0, 1 ou 2,
R1, R2, R3, R4 représentent, indépendamment les uns des autres, H ou $CH_3$.

**10.** Mélange selon l'une quelconque des revendications 1 à 9, dans lequel un composé est représenté par l'une des formules suivantes :

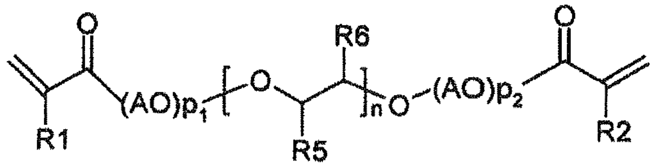

Ia,

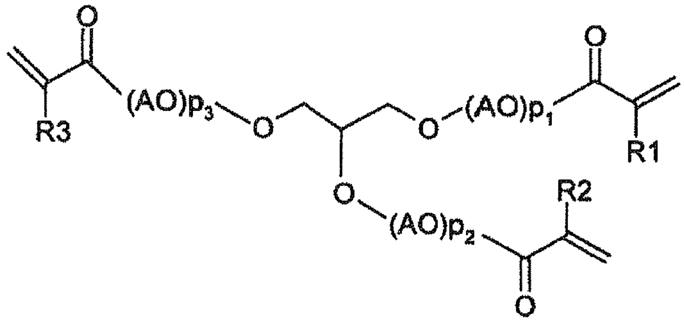

Ib,

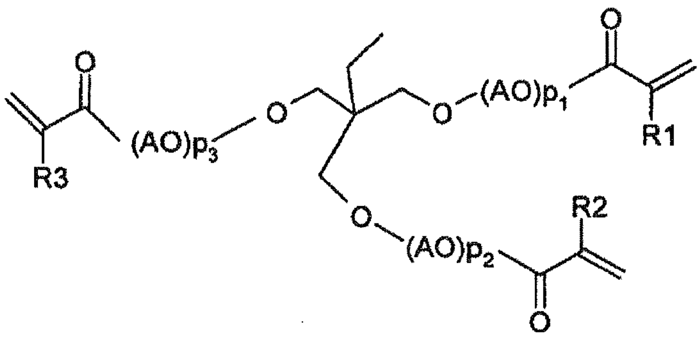

Ic,

ou

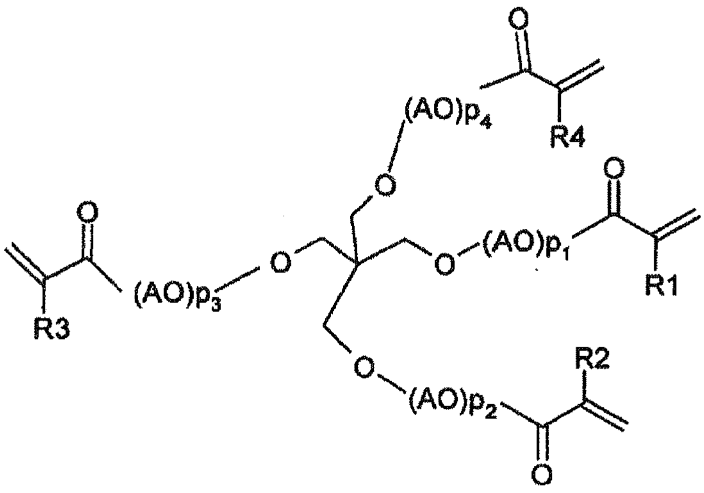

Id

où AO représente, indépendamment pour chaque AO, un groupe -O-CHR7-CHR8- ou -CHR7-CHR8-O-, R7 et R8 représentant, indépendamment l'un de l'autre, H, un groupe alkyle en $C_1$-$C_8$ linéaire ou ramifié,
R5 et R6 représentent, indépendamment l'un de l'autre, H, un groupe alkyle en $C_1$-$C_8$ linéaire ou ramifié,
n est 1, 2, 3, 4, 5, 6, 7, 8, 9, 10, 11, 12, 13, 14, 15, 16, 17, 18, 19 ou 20,
p1 est 7, 8, 9, 10, 11, 12, 13, 14, 15, 16, 17, 18, 19 ou 20,
p2 est 7, 8, 9, 10, 11, 12, 13, 14, 15, 16, 17, 18, 19 ou 20,
p3 est 7, 8, 9, 10, 11, 12, 13, 14, 15, 16, 17, 18, 19 ou 20,
p4 est 7, 8, 9, 10, 11, 12, 13, 14, 15, 16, 17, 18, 19 ou 20,
R1, R2, R3, R4 représentent, indépendamment les uns des autres, H ou $CH_3$.

**11.** Mélange selon l'une quelconque des revendications 9 ou 10, dans lequel AO représente, indépendamment pour chaque AO, EO ou PO,
EO représentant O-$CH_2$-$CH_2$-,
PO représentant chaque fois indépendamment O-$CH_2$-CH ( $CH_3$) - ou O-CH($CH_3$)-$CH_2$-
R5 et R6 représentent, indépendamment l'un de l'autre, H ou $CH_3$.

**12.** Procédé pour la préparation d'un mélange d'esters à base des esters $F_i$ selon l'une quelconque des revendications 1 à 11, à partir d'un mélange d'alcools à base des polyalcools $A_i$, comprenant les étapes

a) mise en réaction des polyalcools $A_i$ avec de l'acide (méth)acrylique en présence d'au moins un catalyseur d'estérification C et d'au moins un inhibiteur de polymérisation D ainsi qu'éventuellement d'un solvant E formant un azéotrope avec l'eau, avec formation d'un mélange d'esters à base des esters $F_i$,
b) éventuellement élimination d'au moins une partie de l'eau formée en a), à partir du mélange réactionnel, l'étape b) pouvant s'effectuer pendant ou après l'étape a),
f) éventuellement neutralisation du mélange réactionnel,
h) si l'on a utilisé un solvant E, éventuellement élimination de ce solvant par distillation et/ou
i) entraînement avec un gaz inerte dans les conditions réactionnelles.

**13.** Procédé pour la préparation d'un mélange d'esters à base des esters $F_i$ selon la revendication 12, **caractérisé en ce que**

- l'excès molaire de l'acide (méth)acrylique aux polyalcools $A_i$ est d'au moins $5*Z_i$ % en moles et
- l'acide (méth)acrylique éventuellement neutralisé, contenu dans le mélange réactionnel obtenu après la dernière étape, reste essentiellement dans le mélange réactionnel.

**14.** Procédé pour la préparation d'un mélange d'esters à base des esters $F_i$ selon l'une quelconque des revendications 12 ou 13, **caractérisé en ce que** l'acide (méth)acrylique est séparé à raison d'au maximum 75 % en poids, du mélange réactionnel contenant le mélange d'esters obtenu après la dernière étape.

**15.** Procédé pour la préparation d'un mélange d'esters à base des esters $F_i$ selon l'une quelconque des revendications 12 à 14, **caractérisé en ce que** le mélange réactionnel contenant le mélange d'esters obtenu après la dernière étape présente un indice d'acide d'au moins 25 mg de KOH/g selon DIN EN 3682.

**16.** Procédé pour la préparation d'un mélange d'esters à base des esters $F_i$ selon l'une quelconque des revendications 12 à 15, **caractérisé en ce que** le mélange réactionnel contenant le mélange d'esters obtenu après la dernière étape présente une teneur en acide (méth)acrylique d'au moins 0,5 % en poids.

**17.** Procédé pour la préparation d'un mélange d'esters à base des esters $F_i$ selon l'une quelconque des revendications 12 à 16, **caractérisé en ce que** dans la réaction a) le rapport molaire de l'acide (méth)acrylique au mélange d'alcools $A_i$ est d'au moins $5*Z_i$:1.

**18.** Procédé pour la préparation d'un hydrogel réticulé, comprenant les étapes

k) polymérisation d'un mélange d'esters à base des esters $F_i$ selon l'une quelconque des revendications 1 à 11 avec de l'acide (méth)acrylique, avec éventuellement des composés à insaturation monoéthylénique N supplémentaires, ainsi qu'éventuellement au moins un autre monomère hydrophile copolymérisable M, en présence d'au moins un amorceur de radicaux K et éventuellement au moins une base de greffage L,
l) éventuellement post-réticulation du mélange réactionnel obtenu à partir de k),
m) séchage du mélange réactionnel obtenu à partir de k) ou l) et
n) éventuellement broyage et/ou tamisage du mélange réactionnel obtenu à partir de k), 1) ou m).

**19.** Procédé pour la préparation d'un hydrogel réticulé, comprenant les étapes a) à i) selon l'une quelconque des revendications 12 à 17 et en outre

k) polymérisation du mélange réactionnel provenant de l'une des étapes a) à i), si effectuées, avec éventuellement des composés à insaturation monoéthylénique N supplémentaires, ainsi qu'éventuellement au moins un autre monomère hydrophile copolymérisable M, en présence d'au moins un amorceur de radicaux K et éventuellement d'au moins une base de greffage L,
l) éventuellement post-réticulation du mélange réactionnel obtenu à partir de k),
m) séchage du mélange réactionnel obtenu à partir de k) ou l) et
n) éventuellement broyage et/ou tamisage du mélange réactionnel obtenu à partir de k), 1) ou m).

**20.** Polymère, pouvant être obtenu conformément à un procédé selon l'une quelconque des revendications 18 ou 19.

**21.** Hydrogel polymère, contenant au moins un monomère hydrophile M sous forme incorporée par polymérisation, réticulé avec un mélange d'esters à base des esters $F_i$ selon l'une quelconque des revendications 1 à 11.

**22.** Hydrogel polymère, contenant au moins un monomère hydrophile M sous forme incorporée par polymérisation, réticulé avec un mélange réactionnel contenant un mélange d'esters à base des esters $F_i$, tel qu'il peut être obtenu selon un procédé des revendications 12 à 15.

**23.** Utilisation d'un polymère selon l'une quelconque des revendications 20 à 22, dans des articles d'hygiène, des matériaux d'emballage et dans des non-tissés.

**24.** Mélanges de substances, contenant

- 0,1 à 40 % en poids d'au moins un mélange d'esters à base des esters $F_i$ selon l'une quelconque des revendications 1 à 11, et d'acide (méth)acrylique,
- 0,5 - 99,9 % en poids d'au moins un monomère hydrophile M,
- 0 - 10 % en poids d'au moins un catalyseur d'estérification. C,
- 0 - 5 % en poids d'au moins un inhibiteur de polymérisation D et
- 0 - 10 % en poids d'un solvant E,

étant entendu que la somme est toujours égale à 100 % en poids.

**25.** Mélange de substances selon la revendication 24, contenant en outre

- des diluants G à jusqu'à 100 % en poids.

**26.** Hydrogel réticulé, pouvant être obtenu à partir d'un mélange de substances selon la revendication 24 ou 25 et en outre

l) éventuellement post-réticulation du mélange réactionnel obtenu,
m) séchage du mélange réactionnel obtenu directement ou obtenu à partir de 1) et
n) éventuellement broyage et/ou tamisage du mélange réactionnel obtenu directement ou obtenu à partir de 1) ou m).

**27.** Utilisation d'un mélange réactionnel pouvant être obtenu selon l'une quelconque des revendications 12 à 17 ou d'un mélange de substances selon la revendication 24 ou 25

- en tant qu'agent de réticulation radicalaire d'hydrogels absorbant l'eau,
- en tant que produit de départ pour la préparation de dispersions de polymères,
- en tant que produit de départ pour la préparation de polyacrylates,
- en tant que matière première pour peintures ou
- en tant qu'additif pour ciment.

**28.** Hydrogel réticulé selon l'une quelconque des revendications 20, 21, 22 ou 26, ayant une teneur résiduelle en agent de réticulation inférieure à 10 ppm, de préférence inférieure à 8 ppm, de façon particulièrement préférée, inférieure à 5 ppm.

**29.** Utilisation d'un mélange d'esters à base des esters $F_i$ selon l'une quelconque des revendications 1 à 11, pour la préparation de polymères formant des hydrogels absorbant des liquides aqueux.

**30.** Utilisation d'un mélange d'esters selon la revendication 29, chaque composant ester $F_i$ étant présent à raison de moins de 2 % en poids, de préférence 1 % en poids, par rapport à la quantité totale des monomères.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9015830 A
- WO 0232964 A
- DE 19646484
- WO 9321237 A
- WO 93212373 A
- US 4187383 A
- WO 200114438 A
- WO 200110920 A
- EP 279303 A
- DE 19941136 A
- DE 3843843 A
- DE 3843854 A
- DE 19937911 A
- DE 19929258 A
- EP 331845 A
- EP 554651 A
- DE 3843854 A1
- DE 10063175
- US 4286082 A
- DE 2706135 C
- US 4340706 A
- DE 3713601 C
- DE 2840010 C
- DE 4344548 A
- DE 020780 A4
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 0801483 A
- EP 0455985 A
- EP 0467073 A
- EP 0312952 A
- EP 0205874 A
- EP 0499774 A
- DE 2612846 A
- DE 4020780 A
- EP 0205674 A
- US 5145906 A
- EP 0530438 A
- EP 0670073 A
- US 4057521 A
- US 4062817 A
- US 4525527 A
- US 4295987 A
- US 5011892 A
- US 4076663 A
- US 4931497 A
- WO 0138402 A
- DE 19854575
- US 5041496 A
- EP 0343427 A
- DE 1301566 C
- WO 9957355 A1
- EP 1023883 A2
- WO 9526209 A
- DE 19604601 A1
- EP 0316518 A
- EP 0202127 A
- WO 0065084 A
- WO 0065348 A
- WO 0035502 A
- DE 19737434
- WO 988439 A
- WO 9524173 A
- WO 9111977 A
- EP 389023 A
- WO 9425077 A
- WO 9701317 A
- WO 9918905 A
- EP 834297 A
- US 5762644 A
- US 5895381 A
- WO 9857609 A
- WO 2000065083 A
- WO 2000069485 A
- WO 2000069484 A
- WO 2000069481 A
- US 6123693 A
- EP 1104666 A
- WO 2001024755 A
- WO 2001000115 A
- EP 105373 A
- WO 2001041692 A
- EP 1074233 A
- WO 9848753 A
- WO 9841179 A
- WO 9709022 A
- WO 9846182 A

- WO 9846181 A
- WO 2001043679 A
- WO 2001043680 A
- WO 2000061052 A
- EP 1108408 A
- WO 2001033962 A
- DE 200020662
- WO 2001001910 A
- WO 2001001908 A
- WO 2001001909 A
- WO 2001001906 A
- WO 2001001905 A
- WO 200124729 A
- EP 311344 A
- EP 850623 A
- WO 9526207 A
- EP 894502 A
- EP 850616 A
- WO 9822063 A
- WO 9749365 A
- EP 903134 A
- EP 887060 A
- EP 887059 A
- EP 887058 A
- EP 887057 A
- EP 887056 A
- EP 931530 A
- WO 9925284 A
- WO 9322998 A
- WO 9820916 A
- EP 306262 A
- WO 9945973 A
- WO 9828478 A
- EP 0615736 A1
- WO 200036216 A1
- US 3556932 A
- WO 9111162 A
- US 3224926 A
- US 3440135 A
- US 3932209 A
- US 4035147 A
- US 4822453 A
- US 4888093 A
- US 4898642 A
- US 5137537 A
- EP 811636 A1

**In der Beschreibung aufgeführte Nicht-Patentliteratur**

- *Analytiker Taschenbuch,* 1984, vol. 4, 433-442
- **Houben-Weyl.** Methoden der Organischen Chemie. Thieme Verlag, 1979, vol. 6/1a
- **Houben-Weyl.** Methoden der Organischen Chemie. Thieme Verlag, 1979, vol. 6/1a, 373-385
- Verdampfertechnik. **R. Billet.** Bibliographisches Institut Mannheim. HTB-Verlag, 1965, 53
- Liquid - Liquid Extraction - Apparatus. **Ullmann.** Encyclopedia of Industrial Chemistry. 1999
- **F.L. Buchholz ; A.T. Graham.** Modern Superabsorbent Polymer Technology. Wiley-VCH, 1998
- Superabsorbents. **Markus Frank.** Ullmann's Handbuch der technischen Chemie. 2003, 35
- **von Robert F. Gould.** Kontaktwinkel, Benetzbarkeit und Adhäsion. *American Chemical Society,* 1964